# EUROPEAN PATENT APPLICATION

(11) **EP 4 620 957 A1**
(43) Date of publication of application: **24.09.2025**
(21) Application number: 24165627.1
(22) Date of filing: 22.03.2024
(51) Int. Cl.: C07D 471/04, C07D 519/00, A61P 35/00, A61K 31/437

(54) **IMIDAZO[4,5-B]PYRIDIN-7-AMINE COMPOUNDS BINDING AURORA KINASE A AND USES THEREOF**

(71) Applicant: Eberhard Karls Universität Tübingen, 72074 Tübingen (DE)
(72) Inventor: Wagner, Benedikt, 72074 Tübingen (DE); Reiner, Juliander, 72074 Tübingen (DE); Moschopoulou, Athina Anastasia, 72074 Tübingen (DE); Zender, Lars, 72074 Tübingen (DE); Laufer, Stefan A., 72074 Tübingen (DE)
(74) Representative: Kuttenkeuler, David

(57) **Abstract**

The present invention relates to metabolically stable compounds that modulate the binding of AURKA to its interacting proteins, especially TPX2. The compounds of the present invention have high therapeutic efficiency due to its high metabolic stability and low toxicity. The present invention also pertains to the use of such compounds in the prevention and/or treatment of proliferative diseases, such as cancer, and kits comprising the same.

## Description

### FIELD OF THE INVENTION

The present invention relates to heterocyclic compounds that interact with Aurora Kinase A (AURKA) and thereby modulate the binding of AURKA to its interacting proteins, especially TPX2. The compounds of the present invention show high therapeutic efficiency due to its high metabolic stability and low toxicity. The present invention further pertains to pharmaceutical compositions, kits, methods for the synthesis of the compounds and various uses of such compounds, preferably in the prevention and/or treatment of proliferative diseases, such as cancer.

### DESCRIPTION

A major limitation in drug development is the limited therapeutic index of currently available therapies or molecularly targeted therapies. Therapeutic index measures the effectiveness of a drug in relation to its adverse effect and provides a quantifiable parameter for the relative safety of a drug. It is crucial to consider the therapeutic index of a drug, since for example, in cancer treatment, drug related toxicities often necessitate restricted drug dosing regimens, resulting in subtotal tumor remissions, which represent an important cause for tumor recurrences and the development of resistance.

Cancer is a major lethal disease for humans and is caused by physiologically uncontrolled proliferation of cancer cells. Despite tremendous efforts on cancer studies and treatments over the past decades, cancer remains one of the most common causes of death in the world. Cancer affects a multitude of normal physiological conditions of the human body, resulting in serious pathological reactions that often lead to the death of the patient.

Aurora kinase A (AURKA) is a serine/threonine-protein kinase that plays a key role during mitosis and meiosis. AURKA's enzymatic activity is highest during the G2 phase to M phase transition in the cell cycle, and its function is essential for a normal cell proliferation. AURKA is required for the recruitment of several important proteins necessary for the formation of the mitotic spindle, and AURKA is also necessary for the proper separation of the centrosomes after the mitotic spindle has been formed. Interestingly, AURKA has been shown to be dysregulated in several different cancer diseases, and AURKA is known to interact with multiple proteins, including MYC, P53, and TPX2. Due to its crucial role in cell proliferation disease, modulating the activity or binding of AURKA is an attractive approach in treating proliferative diseases, such as cancer.

TPX2, also known as targeting protein for Xklp2, is a microtubule assembly factor that plays a key role in microtubule nucleation and growth during the mitotic phase. TPX2 is also important in activating and recruiting AURKA during mitosis. In the presence of nuclear import factor importin a, TPX2 is unable to bind to AURKA, though it is still able to bind microtubules via its amino-terminal domain, which leads to inhibition of M phase microtubule nucleation. TPX2 recruits and activates AURKA by utilizing its short 43 amino acid long amino-terminal sequence to bind the catalytic domain of AURKA, locking the kinase into its active conformation. Importantly, the interaction between AURKA and TPX2 is essential for the formation of the mitotic spindle. TPX2 is known to be overexpressed in different types of cancer including, but not limited thereto, hepatocellular carcinoma (HCC), medullary thyroid cancer, bladder carcinoma, and metastatic breast cancer.

Most work currently in the state of the art on the context of AURKA and TPX2 focuses on inhibiting the binding of AURKA and TPX2 as a strategy against cancer cells.

For example, McIntyre, et al. (in "Characterization of three druggable hot-spots in the Aurora-A/TPX2 interaction using biochemical, biophysical, and fragment-based approaches." ACS chemical biology 12.11 (2017): 2906-2914.) proposed druggable hotspots that disrupt the interaction between AURKA and TPX2.

Cole, et al. (in "Computationally-guided optimization of small-molecule inhibitors of the Aurora A kinase-TPX2 protein-protein interaction." Chemical Communications 53.67 (2017): 9372-9375. Discloses) proposed another approach to inhibit AURKA and TPX2 interaction.

Yan, et al. (in "Aurora-A kinase inhibitor scaffolds and binding modes." Drug discovery today 16.5-6 (2011): 260-269.) also speculates that inhibitors ARUKA and TPX2 could be beneficial to the discovery and optimization of enzyme inhibitors as therapeutic agents.

Takahashi, et al. (in "The AURKA/TPX2 axis drives colon tumorigenesis cooperatively with MYC." Annals of oncology 26.5 (2015): 935-942.) proposed inhibiting the AURKA/TPX2 axis would be a novel synthetic lethal therapeutic approach for MYC-driven cancers. "

Other works in the state of the art have focused on decreasing the level of either the AURKA or the TPX protein. For example, Gomes-Filho, et al. (in "Aurora A kinase and its activator TPX2 are potential therapeutic targets in KRAS-induced pancreatic cancer." Cellular Oncology 43 (2020): 445-460.) proposed the use of an siRNA-based approach to reduce the AURKA and TPX2 expression.

However, this approach to inhibit the AURKA/TPX2 binding or to altogether decrease the expression of AURKA or TPX protein might not serve the purpose of anti-cancer therapy, especially taking into the account that the interaction of AURKA with TPX2 mostly plays a joint role in stabilizing the mitotic spindle apparatus. Accordingly, cancer cells tend to show overactivity of the AURKA/TPX2 axis, which must be curbed.

Other works, such as Bavetsias, et al. (in "Aurora isoform selectivity: design and synthesis of imidazo [4,5-b] pyridine derivatives as highly selective inhibitors of Aurora-A kinase in cells." Journal of Medicinal Chemistry 56.22 (2013): 9122-9135.) discloses a specific inhibitor comprising imidazo[4,5-b]pyridine derivatives of AURKA activity. However, since the kinase activity of AURKA is crucial in cell viability, directly inhibiting its kinase activity will not be applicable as a therapeutic approach due to its potential harmful side-effects.

WO2022/096679 discloses anilide-based compounds that modulate the conformation of Aurora kinase A (AURKA) as well as its interactome as a promising therapeutic target for the treatment of TP53 altered liver carcinomas and TP53; RB1 altered small cell lung carcinomas.

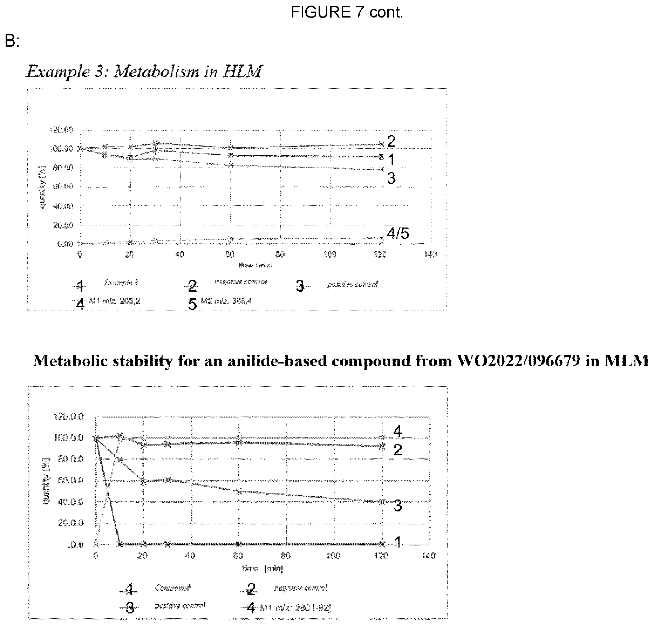

However, the anilide based compound displays poor therapeutic efFiciency, mainly due to its metabolic instability (see Figure 7B). This is mainly attributed to the functionally important, but hydrolysis labile anilide moiety.

Therefore, it is an object of the present invention to provide metabolically stable kinase activity sparing compounds with high therapeutic index, which increase the interaction of AURKA to its binding partner, preferably TPX2 by modulating AURKA conformation, thereby selectively kill the diseased cells of target.

### BRIEF DESCRIPTION OF THE INVENTION

Generally, and by way of brief description, the main aspects of the present invention can be described as follows:
In **a first aspect,** the invention pertains to a compound comprising a nine-membered heterocyclic ring according to formula (I), (II) or (III).
In **a second aspect,** the present invention relates to a pharmaceutical composition comprising a compound comprising a nine-membered heterocyclic ring according to formula (I), (II) or (III) optionally together with a pharmaceutically acceptable carrier and/or excipient.
In **a third aspect,** the invention pertains to a compound comprising a nine-membered heterocyclic ring according to formula (I), (II) or (III), or a pharmaceutical composition comprising the same, for use in medicine.
In **a fourth aspect,** the invention pertains a compound comprising a nine-membered heterocyclic ring according to formula (I), (II) or (III), or a pharmaceutical composition comprising the same, for use in the prevention and/or treatment of a proliferative disease, such as cancer.
In **a fifth aspect,** the invention pertains to a kit comprising a compound according to this invention, or a pharmaceutical composition comprising the same.
In **a sixth aspect,** the invention pertains to the use of a compound comprising a nine-membered heterocyclic ring according to formula (I), (II) or (III), a pharmaceutical composition or a kit comprising the same, in selectively modulating the interaction of a serine/threonine kinase, or a variant thereof, with at least one binding protein of the serine/threonine kinase, or a variant thereof.
In **a seventh aspect,** the invention pertains to the use of a compound comprising a nine-membered heterocyclic ring according to formula (I), (II) or (III), a pharmaceutical composition or a kit comprising the same, in modulating the AURKA interactome.
In **an eighth aspect,** the invention pertains to a method of producing a compound comprising a nine-membered heterocyclic ring according to formula (I), (II) or (III).

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the object is solved by providing a compound, or solvates, salts, N-oxides, racemic mixtures, diastereomers, enantiomers, tautomers, isotopically labeled forms, and combinations thereof, wherein the compound comprises a nine-membered heterocyclic ring according to the formula (I): 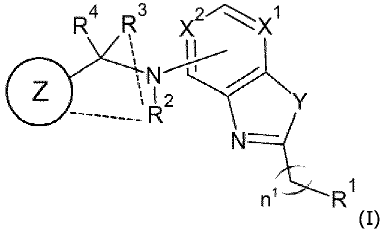
X¹ and X² are each independently selected from C or N atoms;
Y is selected from the group consisting of NH, N-alkyl, O, S;
n¹ is any natural number selected from 0, 1, 2, 3 and preferably is 2;
R¹ is selected from the group consisting of substituted or non-substituted homocyclic ring, substituted or unsubstituted heterocyclic ring, alkyl, and alkoxy;
R² is selected from H, alkyl and alkylene group that is also covalently bonded to any of Z, R³ or R⁴;
R³ is selected from H, alkyl;
R⁴ is selected from H, alkyl, and
Z constitutes one or more unsubstituted or substituted aromatic ring.

In one preferred embodiment of the compound with Formula (I) according to the present invention, said substituted or non-substituted aromatic ring Z further comprises groups selected from
a) 4-(methyl-1*H*-pyrazol-4-yl)naphthalen-1-yl, 5-(methyl-1*H*-pyrazol-4-yl)furan-2-yl, 5-(methyl-1*H*-pyrazol-4-yl)thiophene-2-yl, or
b)
   has formula ZI 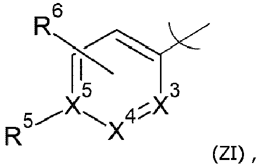 wherein
   X³, X⁴ and X⁵ are each independently selected from C or N atom;
   if X⁵ = N, R⁵ is dispensed with;
   if X⁵ = C, R⁵ is selected from H atom; halogen, particularly Br; Cl and F; C₁₋₃-alkyl, in particular isoprop-2-yl; C₁₋₃-alkoxy, in particular methoxy-1-yl; diC₁₋₃-alkyl amine group, in particular N,N-dimethylamin-1-yl; N,N-(Dimethy(*d*₃))amin-1-yl; diC₁₋₃-alkyl phosphine oxide, in particular dimethylphosphine oxide, halogen substituted sulfane, in particular pentafluoro-λ6-sulfane;
   substituted and/or non-substituted five-membered hetero and/or homocycles, in particular substituted and/or non-substituted pyrrole, particularly,1-methyl-1*H*-pyrrole-3-yl; hexahydro-1*H*-furo[3,4-*c*]pyrrole-1-yl,substituted and/or non-substituted oxazole, in particular 3,5-dimethylisoxazol-4-yl; substituted and/or non-substituted imidazole, in particular 1-methyl-1*H*-imidazol-4-yl; 4-methyl-1*H*-imidazol-1-yl; substituted and/or non-substituted pyrazole, in particular 1-cyclopropyl-1*H*-pyrazol-4-yl; 1,3-dimethy-1*H*-pyrazol-4-yl; 1,5-dimethyl-1*H*-pyrazole-4-yl; 1,3,5-trimethyl-1*H*-pyrazol-4-yl; 1-difluormethyl-1*H*-pyrazol-4-yl; 1-ethyl-1*H*-pyrazol-4-yl; 1-(2-(morpholin-4-yl)-ethyl)-1*H*-pyrazol-4-yl; 1*H*-pyrazol-4-yl; 1-isopropyl-1*H*-pyrazol-4-yl; 1-(methyl-(*d*₃))-1*H*-pyrazol-4-yl; 1-methyl-1*H-*pyrazol-3-yl; 1-methyl-1*H*-pyrazol-4-yl; 1-methyl-1*H*-pyrazol-5-yl; 1-(methylsulfonyl)-1*H*-pyrazol-4-yl; 1-(oxetan-3-yl)-1*H*-pyrazol-4-yl; 1-(tetrahydro-2*H*-pyran-4-yl)-1*H*-pyrazol-4-yl; 1-(trifluoromethyl)-1*H*-pyrazol-4-yl; 3,5-dimethyl-1*H*-pyrazol-4-yl; 3-methyl-1*H*-pyrazol-4-yl; 1-methyl-3-(trifluoromethyl)-1*H*-pyrazole-4-yl; 1-propyl-1*H-*pyrazole-4-yl; 2-(ethan-1-ol)-1*H*-pyrazol-4-yl; 1-(2-methoxyethyl)-1*H*-pyrazole-4-yl; 4-methyl-1*H*-pyrazol-1-yl; substituted and/or non-substituted Triazole, in particular 1-methyl-1,2,4-1*H*-triazol-3-yl; 1-methyl-1,2,3-1*H*-triazol-4-yl; substituted and/or non-substituted Thiazole, in particular 2-Methylthiazol-5-yl; substituted and/or non-substituted Pyrrolidine, in particular, 3,3-difluoropyrrolidin-1-yl; 3-methoxypyrrolidin-1-yl;
   substituted and/or non-substituted six-membered hetero and/or homocycles, particularly substituted and/or non-substituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or non-substituted pyrimidine, in particular, pyrimidin-5-yl; substituted and/or non-substituted morpholine, in particular, thiomorpholin-1,1-dioxid-4-yl; (2*R*,6*S*)-dimethylmorpholin-4-yl; (2*S*,6*S*)-dimethylmorpholin-4-yl; (3*R*,5*S*)-dimethylmorpholin-4-yl; (3*S*,5*S*)-dimethylmorpholin-4-yl; (*R*)-2-methylmorpholin-4-yl; (*R*)-3-methylmorpholin-4-yl; (*S*)-2-methylmorpholin-4-yl; (*S*)-3-methylmorpholin-4-yl; 2,2-dimethylmorpholin-4-yl; 3,3-dimethylmorpholin-4-yl; morpholin-4-yl; substituted and/or non-substituted piperidine, in particular 4,4-difluoropiperidin-1-yl;
   substituted and/or non-substituted four-membered hetero and/or homocycles, in particular substituted and/or non-substituted azetidine, particularly, 3,3-difluoroazetidin-1-yl; 3-dimethylaminoazetidin-1-yl; 3-fluoroazetidin-1-yl; azetidin-1-yl; and
   substituted and/or non-substituted hetero and/or homo bicycles, in particular (1*S*,4*S*)-2-oxa-5-azabicyclo[2.2.1]heptan-5-yl; (1*R*, 4*R*)-2-oxa-5-azabicyclo[2.2.1]heptan-5-yl;3-oxa-8-azabicyclo[3.2.1]octan-8-yl; 8-oxa-3-azabicyclo[3.2.1]octan-3-yl; 2-oxa-6-azaspiro[3.3]heptan-6-yl; 1*H*-pyrrolo[2,3-b]pyridin-4-yl; 2-methyl-2*H*-indazol-5-yl; pyrazolo[1,5-*a*]pyridin-3-yl; quinolin-4-yl;
   R⁶ is selected from H, halogen, in particular Br, Cl, F; unsubstituted or substituted C₁₋₃-alkoxy, in particular methoxy, ethoxy, OCF₃, OCHF₂; methylsulfonyl, dimethylphosphine oxide; and unsubstituted or substituted C₁₋₃-alkyl, in particular methyl or CF₃ or
c) 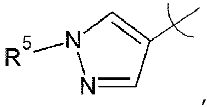 wherein R⁵ is selected from H atom and C₁₋₃-alkyl groups, in particular methyl group.

In another preferred embodiment of the compound with formula (I) according to the present invention, said R¹ further comprises groups selected from benzofuran-5-yl, benzofuran-6-yl, cyclopropyl, cyclobutyl, furan-2-yl, furan-3-yl, 5-methylfuran-2-yl, 5-phenylfuran-2-yl, phenoxy, phenyl, tetrahydrofuran-3-yl, thiophen-2-yl, thiophen-3-yl, isoprop-2-yl and methoxy.

In another preferred embodiment in the current invention, the compound according to the invention has formula (II), 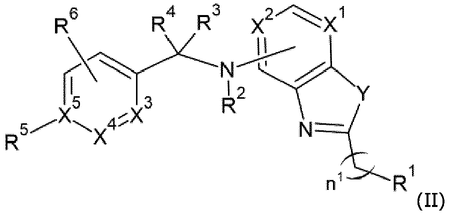 and solvates, salts, N-oxides, racemic mixtures, diastereomers, enantiomers, tautomers, isotopically labeled forms which are compounds in which one or more atoms are replaced by a corresponding atom having the same number of protons but differing in the number of neutrons, and combinations thereof;
wherein:
X¹, X², X³, X⁴ and X⁵ are each individually selected from C or N atom;
Y is selected from the group consisting of NH, N-alkyl, unsubstituted or substituted C₁₋₆-alkyl or cycloalkyl, O, S;
n¹ is any number selected from 0, 1, 2, 3;
R¹ is selected from the group consisting of benzofuran-5-yl, benzofuran-6-yl, cyclopropyl, cyclobutyl, furan-2-yl, furan-3-yl, 5-methylfuran-2-yl, 5-phenylfuran-2-yl, phenoxy, phenyl, tetrahydrofuran-3-yl, thiophen-2-yl, thiophen-3-yl, isoprop-2-yl and methoxy;
R² is selected from H, alkyl, in particular methyl;
R³ is selected from H, alkyl;
R⁴ is selected from H, alkyl;
if X⁵ = N, R⁵ is dispensed with;
and if X⁵ is C, R⁵ is selected from H, halogen, particularly Br; Cl and F; C₁₋₃-alkyl, in particular isoprop-2-yl;
C₁₋₃-alkoxy, in particular methoxy-1-yl; diC₁₋₃-alkylamine group, in particular N,N-dimethylamin-1-yl; N,N-(Dimethy(*d*₃))amin-1-yl; diC₁₋₃-alkyl phosphine oxide, in particular dimethylphosphine oxide, halogen substituted sulfane, in particular pentafluoro-λ6-sulfane;
substituted and/or unsubstituted five-membered hetero and/or homocycles, in particular substituted and/or unsubstituted pyrrole, particularly,1-methyl-1*H*-pyrrole-3-yl; hexahydro-1*H-*furo[3,4-*c*]pyrrole-1-yl,substituted and/or unsubstituted oxazole, in particular 3,5-dimethylisoxazol-4-yl; substituted and/or unsubstituted imidazole, in particular 1-methyl-1*H*-imidazol-4-yl; 4-methyl-1*H-*imidazol-1-yl; substituted and/or unsubstituted pyrazole, in particular 1-cyclopropyl-1*H*-pyrazol-4-yl; 1,3-dimethy-1*H*-pyrazol-4-yl; 1,5-dimethyl-1*H*-pyrazole-4-yl; 1,3,5-trimethyl-1*H*-pyrazol-4-yl; 1-difluormethyl-1*H*-pyrazol-4-yl; 1-ethyl-1*H*-pyrazol-4-yl; 1-(2-(morpholin-4-yl)-ethyl)-1*H*-pyrazol-4-yl; 1*H*-pyrazol-4-yl; 1-isopropyl-1*H*-pyrazol-4-yl; 1-(methyl-(*d*3))-1*H*-pyrazol-4-yl; 1-methyl-1*H-*pyrazol-3-yl; 1-methyl-1*H*-pyrazol-4-yl; 1-methyl-1*H*-pyrazol-5-yl; 1-(methylsulfonyl)-1*H*-pyrazol-4-yl; 1-(oxetan-3-yl)-1*H*-pyrazol-4-yl; 1-(tetrahydro-2*H*-pyran-4-yl)-1*H*-pyrazol-4-yl; 1-(trifluoromethyl)-1*H*-pyrazol-4-yl; 3,5-dimethyl-1*H*-pyrazol-4-yl;3-methyl-1*H*-pyrazol-4-yl; 1-methyl-3-(trifluoromethyl)-1*H*-pyrazole-4-yl; 1-propyl-1*H-*pyrazole-4-yl; 2-(ethan-1-ol)-1*H*-pyrazol-4-yl; 1-(2-methoxyethyl)-1*H*-pyrazole-4-yl; 4-methyl-1*H*-pyrazol-1-yl; substituted and/or unsubstituted Triazole, in particular 1-methyl-1,2,4-1*H*-triazol-3-yl; 1-methyl-1,2,3-1*H*-triazol-4-yl; substituted and/or unsubstituted Thiazole, in particular 2-Methylthiazol-5-yl; substituted and/or unsubstituted Pyrrolidine, in particular, 3,3-difluoropyrrolidin-1-yl; 3-methoxypyrrolidin-1-yl;
substituted and/or unsubstituted six-membered hetero and/or homocycles, particularly substituted and/or unsubstituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or unsubstituted pyrimidine, in particular, pyrimidin-5-yl; substituted and/or unsubstituted morpholine, in particular, thiomorpholin-1,1-dioxid-4-yl; (2R,6S)-dimethylmorpholin-4-yl; (25,65)-dimethylmorpholin-4-yl; (3*R*,5*S*)-dimethylmorpholin-4-yl; (3*S*,5*S*)-dimethylmorpholin-4-yl; (*R*)-2-methylmorpholin-4-yl; (*R*)-3-methylmorpholin-4-yl; (*S*)-2-methylmorpholin-4-yl; (*S*)-3-methylmorpholin-4-yl; 2,2-dimethylmorpholin-4-yl; 3,3-dimethylmorpholin-4-yl; morpholin-4-yl; substituted and/or unsubstituted piperidine, in particular 4,4-difluoropiperidin-1-yl;
substituted and/or unsubstituted four-membered hetero and/or homocycles, in particular substituted and/or unsubstituted azetidine, particularly, 3,3-difluoroazetidin-1-yl; 3-dimethylaminoazetidin-1-yl; 3-fluoroazetidin-1-yl; azetidin-1-yl; and
substituted and/or unsubstituted hetero and/or homo bicycles, in particular (1*S*,4*S*)-2-oxa-5-azabicyclo[2.2.1]heptan-5-yl; (1*R*, 4*R*)-2-oxa-5-azabicyclo[2.2.1]heptan-5-yl;3-oxa-8-azabicyclo[3.2.1]octan-8-yl; 8-oxa-3-azabicyclo[3.2.1]octan-3-yl; 2-oxa-6-azaspiro[3.3]heptan-6-yl; 1*H*-pyrrolo[2,3-*b*]pyridin-4-yl; 2-methyl-2*H*-indazol-5-yl; pyrazolo[1,5-*a*]pyridin-3-yl; quinolin-4-yl;
R⁶ is selected from H, halogen, in particular Br, Cl, F; unsubstituted or substituted C₁₋₃-alkoxy, in particular methoxy, ethoxy, OCF₃, OCHF₂; methylsulfonyl, dimethylphosphine oxide; and unsubstituted or substituted C₁₋₃-alkyl, in particular methyl or CF₃.
In a further preferred embodiment, the compound according to the invention have formula (I) or (II), wherein the n¹ is 2.

In a particularly preferred embodiment, the compound according to the invention has formula (III) 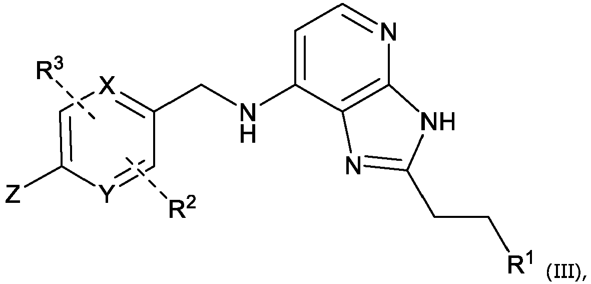
X and Y are each individually selected from C or N atom;
R¹ is selected from the group consisting of cycloalkane, in particular cyclopropyl, cyclobutyl or (hetero)aryl, in particular furan, particularly furan-2-yl, furan-3-yl, methylfuran, in particular 5-methylfuran-2-yl, thiophen, in particular thiophen-2-yl, thiophen-3-yl and phenyl;
R² and R³ are each individually selected from H, F, alkyl, in particular methyl, alkoxy, in particular methoxy and ethoxy and -O-halogen alkyl, in particular OCF₃, OCHF₂;
Wherein Z comprises unsubstituted or substituted aromatic or aliphatic heterocycle, in particular wherein Z is selected from the formula (ZIII), (ZIV), (ZV), and (ZVI),
wherein formula (ZIII) is 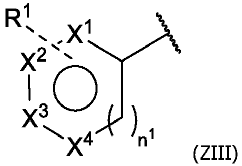
Wherein X¹, X², X³ and X⁴ are each independently selected from C or N,
n¹ is any number selected from 0, 1, 2, 3;
R¹ is selected from H, F, alkyl, in particular methyl
and wherein formula (ZIV) is, 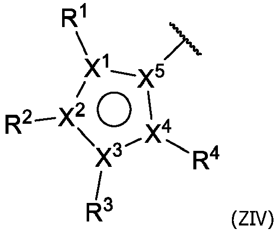
Wherein X¹, X², X³ and X⁴ are each independently selected from C, N, O or S and X⁵ is selected from C or N;
and wherein if X¹ is S or O, R¹ is dispensed with, and if X¹ is C or N, R¹ is selected from H, F, alkyl, in particular methyl, methyl-*d*₃, ethyl and cyclopropyl, cyclic ether, in particular oxetane, halogen alkyl, in particular CHF₂ and CF₃,
and wherein if X² is S or O, R² is dispensed with, and if X² is C or N, R² is selected from H, F, alkyl, in particular methyl, methyl-*d*₃, ethyl and cyclopropyl, cyclic ether, in particular oxetane, halogen alkyl, in particular CHF₂ and CF₃,
and wherein if X³ is S or O, R³ is dispensed with, and if X³ is C or N, R³ is selected from H, F, alkyl, in particular methyl, methyl-*d*₃, ethyl and cyclopropyl, cyclic ether, in particular oxetane, halogen alkyl, in particular CHF₂ and CF₃,
and wherein if X⁴ is S or O, R⁴ is dispensed with, and if X⁴ is C or N, R⁴ is selected from H, F, alkyl, in particular methyl, methyl-*d*₃, ethyl and cyclopropyl, cyclic ether, in particular oxetane, halogen alkyl, in particular CHF₂ and CF₃
and wherein formula (ZV) is, 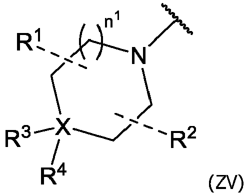
Wherein X is selected from C, O, S or SOz;
n¹ is any number selected from 0, 1, 2;
R¹ and R² are each independently selected from H, F, alkyl, in particular methyl;
and wherein if X is O, S or SO₂, R³ and R⁴ are dispensed with, and if X is C, R³ and R⁴ are selected from H, F;
and wherein formula (ZVI) is, 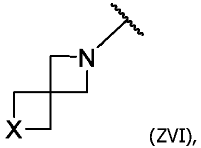
wherein X is selected from O, S or SOz;

Exemplary selection of 100 Example formulas of the compounds from a larger database according to the present invention are illustrated in Table 1.

**Table 1. Example Formula**

| Chemical name | Chemical structures |
|---|---|
| *Example 1* | 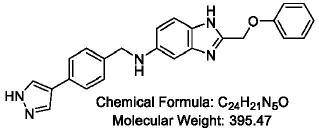 |
| *N-*{4-(1*H*-pyrazol-4-yl)benzyl)-2-(phenoxymethyl)-1*H-*benzo[*d*]imidazol-5-amine | |
| *Example 2:* | 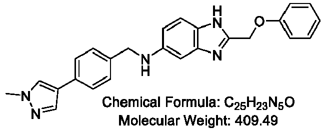 |
| *N-*(4-(1-methyl-1*H*-pyrazol-4-yl)benzyl)-2-(phenoxymethyl)-1*H*-benzo[*d*]imidazol-5-amine | |
| *Example 3* | 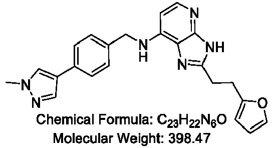 |
| 2-(2-(furan-2-yl)ethyl)-*N-*(4-(1-methyl-1*H*-pyrazol-4-yl)benzyl)-3*H*-imidazo[4,5-*b*]pyridin-7-amine | |
| *Example 4* | 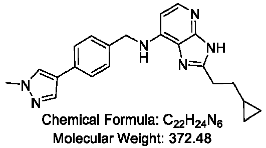 |
| 2-(2-cyclopropylethyl)-*N-*(4-(1-methyl-1*H*-pyrazol-4-yl)benzyl)-3*H*-imidazo[4,5-*b*]pyridin-7-amine | |
| *Example 5* | 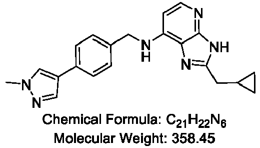 |
| 2-(cyclopropylmethyl)-*N-*(4-(1-methyl-1*H-*pyrazol-4-yl)benzyl)-3*H*-imidazo[4,5-*b*]pyridin-7-amine | |
| *Example 6* | 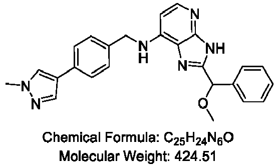 |
| 2-(methoxy(phenyl)methyl)-*N-*(4-(1-methyl-1*H-*pyrazol-4-yl)benzyl)-3*H*-imidazo[4,5-*b*]pyridin-7-amine | |
| *Example 7* | 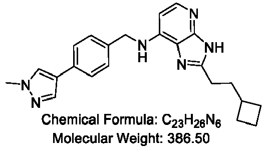 |
| 2-(2-cyclobutylethyl)-*N-*(4-(1-methyl-1*H*-pyrazol-4-yl)benzyl)-3*H*-imidazo[4,5-*b*]pyridin-7-amine | |
| *Example 8* | 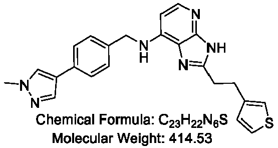 |
| *N-*(4-(1-methyl-1*H*-pyrazol-4-yl)benzyl)-2-(2-(thiophen-3-yl)ethyl)-3*H*-imidazo[4,5-*b*]pyridin-7-amine | |
| *Example 9* | 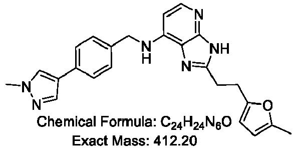 |
| *N-*(4-(1-methyl-1*H*-pyrazol-4-yl)benzyl)-2-(2-(5-methylfuran-2-yl)ethyl)-3*H*-imidazo[4,5-*b*]pyridine-amine | |
| *Example 10* | 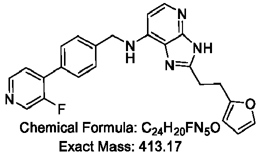 |
| *N-*(4-(3-fluoropyridin-4-yl)benzyl)-2-(2-(furan-2-yl)ethyl)-3*H*-imidazo[4,5-*b*]pyridin-7-amine | |
| *Example 11* | 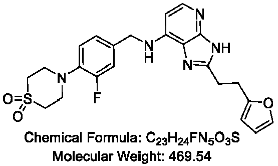 |
| 4-(2-fluoro-4-(((2-(2-(furan-2-yl)ethyl)-3*H-*imidazo[4,5-*b*]pyridin-7-yl)amino)methyl)phenyl) thiomorpholine 1,1-dioxide | |
| *Example 12* | 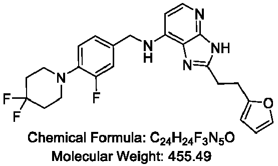 |
| *N-*(4-(4,4-difluoropiperidin-1-yl)-3-fluorobenzyl)-2-(2-(furan-2-yl)ethyl)-3*H*-imidazo[4,5-*b*]pyridin-7-amine | |
| *Example 13* | 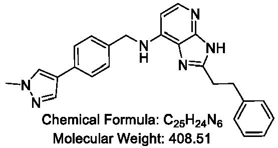 |
| *N*-(4-(1-methyl-1*H*-pyrazol-4-yl)benzyl)-2-phenethyl-3*H*-imidazo[4,5-*b*]pyridin-7-amine | |
| *Example 14* | 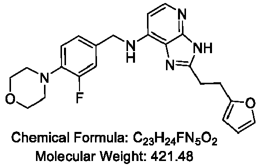 |
| *N*-(3-fluoro-4-morpholinobenzyl)-2-(2-(furan-2-yl)ethyl)-3*H*-imidazo[4,5-*b*]pyridin-7-amine | |
| *Example 15* | 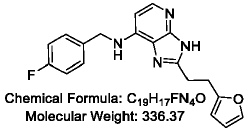 |
| *N*-(4-fluorobenzyl)-2-(2-(furan-2-yl)ethyl)-3*H-*imidazo[4,5-b]pyridin-7-amine | |
| *Example 16* | 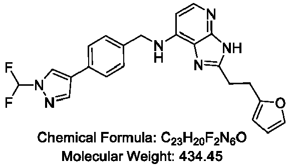 |
| *N-*(4-(1-(difluoromethyl)-1*H*-pyrazol-4-yl)benzyl)-2-(2-(furan-2-yl)ethyl)-3*H*-imidazo[4,5-*b*]pyridin-7-amine | |
| *Example 17* | 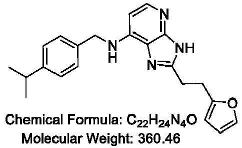 |
| 2-(2-(furan-2-yl)ethyl)-*N*-(4-isopropylbenzyl)-3*H-*imidazo[4,5-b]pyridin-7-amine | |
| *Example 18* | 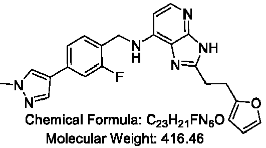 |
| *N*-(2-fluoro-4-(1-methyl-1*H*-pyrazol-4-yl)benzyl)-2-(2-(furan-2-yl)ethyl)-3*H*-imidazo[4,5-*b*]pyridin-7-amine | |
| *Example 19* | 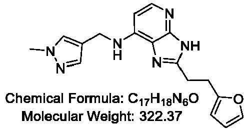 |
| 2-(2-(furan-2-yl)ethyl)-*N-*((1-methyl-1*H*-pyrazol-4-yl)methyl)-3*H-*imidazo[4,5-*b*]pyridin-7-amine | |
| *Example 20* | 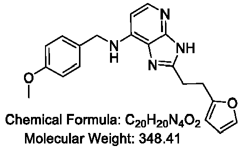 |
| 2-(2-(furan-2-yl)ethyl)-*N-*(4-methoxybenzyl)-3*H-*imidazo[4,5-b]pyridin-7-amine | |
| *Example 21* | 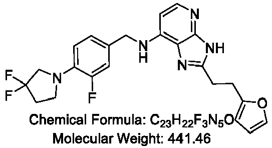 |
| *N-*(4-(3,3-difluoropyrrolidin-1-yl)-3-fluorobenzyl)-2-(2-(furan-2-yl)ethyl)-3*H*-imidazo[4,5-*b*]pyridin-7-amine | |
| *Example 23* | 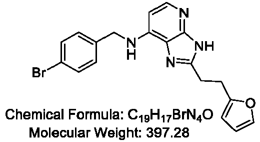 |
| *N*-(4-bromobenzyl)-2-(2-(furan-2-yl)ethyl)-3*H-*imidazo[4,5-*b*]pyridin-7-amine | |
| *Example 24* | 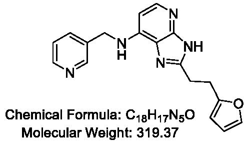 |
| 2-(2-(furan-2-yl)ethyl)-*N-*(pyridin-3-ylmethyl)-3*H-*imidazo[4,5-*b*]pyridin-7-amine | |
| *Example 25* | 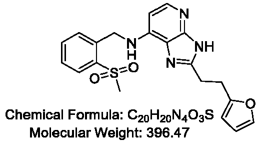 |
| 2-(2-(furan-2-yl)ethyl)-*N*-(2-(methylsulfonyl)benzyl)-3*H*-imidazo[4,5-*b*]pyridin-7-amine | |
| *Example 26* | 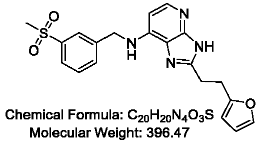 |
| 2-(2-(furan-2-yl)ethyl)-*N*-(3-(methylsulfonyl)benzyl)-3*H*-imidazo[4,5-*b*]pyridin-7-amine | |
| *Example 27* | 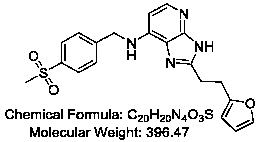 |
| 2-(2-(furan-2-yl)ethyl)-*N*-(4-(methylsulfonyl)benzyl)-3*H*-imidazo[4,5-*b*]pyridin-7-amine | |
| *Example 28* | 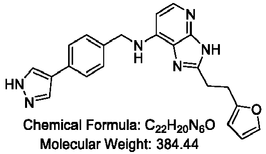 |
| *N-*(4-(1*H*-pyrazol-4-yl)benzyl)-2-(2-(furan-2-yl)ethyl)-3*H*-imidazo[4,5-*b*]pyridin-7-amine | |
| *Example 29* | 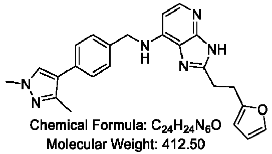 |
| *N*-(4-(1,3-dimethyl-1*H*-pyrazol-4-yl)benzyl)-2-(2-(furan-2-yl)ethyl)-3*H*-imidazo[4,5-*b*]pyridin-7-amine | |
| *Example 30* | 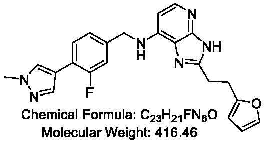 |
| *N*-(3-fluoro-4-(1-methyl-1*H*-pyrazol-4-yl)benzyl)-2-(2-(furan-2-yl)ethyl)-3*H*-imidazo[4,5-*b*]pyridin-7-amine | |
| *Example 31* | 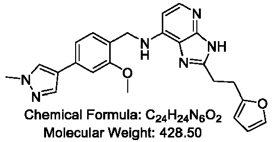 |
| 2-(2-(furan-2-yl)ethyl)-*N*-(2-methoxy-4-(1-methyl-1*H-*pyrazol-4-yl)benzyl)-3*H*-imidazo[4,5-*b*]pyridin-7-amine | |
| *Example 32* | 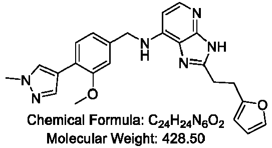 |
| 2-(2-(furan-2-yl)ethyl)-*N-*(3-methoxy-4-(1-methyl-1*H-*pyrazol-4-yl)benzyl)-3*H*-imidazo[4,5-*b*]pyridin-7-amine | |
| *Example 33* | 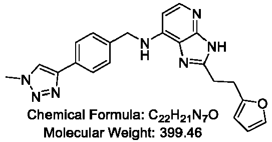 |
| 2-(2-(furan-2-yl)ethyl)-*N-*(4-(1-methyl-1*H*-1,2,3-triazol-4-yl)benzyl)-3*H*-imidazo[4,5-*b*]pyridin-7-amine | |
| *Example 34* | 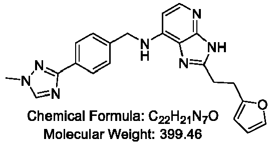 |
| 2-(2-(furan-2-yl)ethyl)-*N*-(4-(1-methyl-1*H*-1,2,4-triazol-3-yl)benzyl)-3*H*-imidazo[4,5-*b*]pyridin-7-amine | |
| *Example 35* | 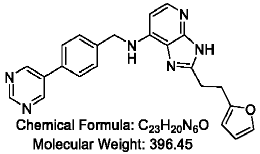 |
| 2-(2-(furan-2-yl)ethyl)-*N*-(4-(pyrimidin-5-yl)benzyl)-3*H*-imidazo[4,5-*b*]pyridin-7-amine | |
| *Example 36* | 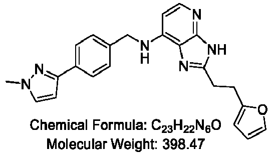 |
| 2-(2-(furan-2-yl)ethyl)-*N-*(4-(1-methyl-1*H*-pyrazol-3-yl)benzyl)-3*H*-imidazo[4,5-*b*]pyridin-7-amine | |
| *Example 37* | 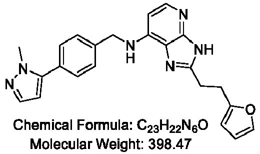 |
| 2-(2-(furan-2-yl)ethyl)-*N-*(4-(1-methyl-1*H*-pyrazol-5-yl)benzyl)-3*H*-imidazo[4,5-*b*]pyridin-7-amine | |
| *Example 38* | 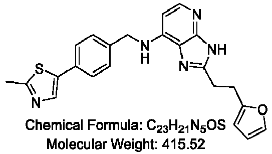 |
| 2-(2-(furan-2-yl)ethyl)-*N*-(4-(2-methylthiazol-5-yl)benzyl)-3*H*-imidazo[4,5-*b*]pyridin-7-amine | |
| *Example 39* | 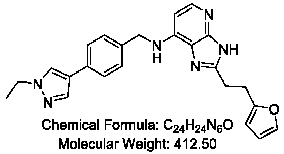 |
| *N-*(4-(1-ethyl-1*H*-pyrazol-4-yl)benzyl)-2-(2-(furan-2-yl)ethyl)-3*H*-imidazo[4,5-*b*]pyridin-7-amine | |
| *Example 40* | 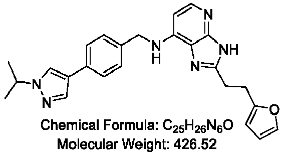 |
| 2-(2-(furan-2-yl)ethyl)-*N-*(4-(1-isopropyl-1*H-*pyrazol-4-yl)benzyl)-3*H*-imidazo[4,5-*b*]pyridin-7-amine | |
| *Example 41* | 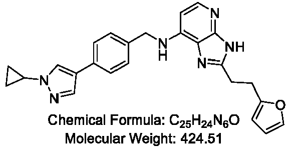 |
| *N*-(4-(1-cyclopropyl-1*H*-pyrazol-4-yl)benzyl)-2-(2-(furan-2-yl)ethyl)-3*H*-imidazo[4,5-*b*]pyridin-7-amine | |
| *Example 42* | 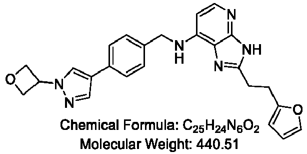 |
| 2-(2-(furan-2-yl)ethyl)-*N-*(4-(1-(oxetan-3-yl)-1*H-*pyrazol-4-yl)benzyl)-3*H*-imidazo[4,5-*b*]pyridin-7-amine | |
| *Example 43* | 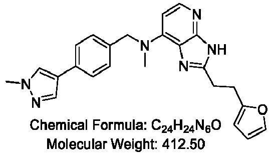 |
| 2-(2-(furan-2-yl)ethyl)-*N*-methyl-*N*-(4-(1-methyl-1*H-*pyrazol-4-yl)benzyl)-3*H*-imidazo[4,5-*b*]pyridin-7-amine | |
| *Example 44* | 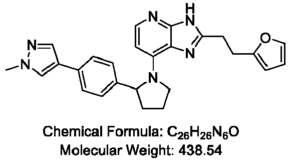 |
| 2-(2-(furan-2-yl)ethyl)-7-(2-(4-(1-methyl-1*H*-pyrazol-4-yl)phenyl)pyrrolidin-1-yl)-3*H*-imidazo[4,5-*b*]pyridine | |
| *Example 45* | 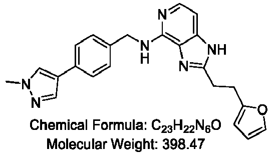 |
| 2-(2-(furan-2-yl)ethyl)-*N-*(4-(1-methyl-1*H*-pyrazol-4-yl)benzyl)-1*H*-imidazo[4,5-*c*]pyridin-4-amine | |
| *Example 46* | 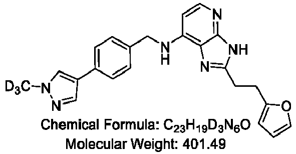 |
| 2-(2-(furan-2-yl)ethyl)-*N-*(4-(1-(methyl-*d*3)-1*H-*pyrazol-4-yl)benzyl)-3*H*-imidazo[4,5-*b*]pyridin-7-amine | |
| *Example 47* | 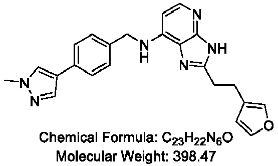 |
| 2-(2-(furan-3-yl)ethyl)-*N-*(4-(1-methyl-1*H*-pyrazol-4-yl)benzyl)-3*H*-imidazo[4,5-*b*]pyridin-7-amine | |
| *Example 48* | 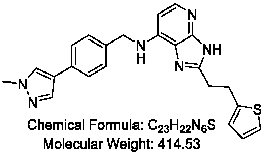 |
| *N-*(4-(1-methyl-1*H*-pyrazol-4-yl)benzyl)-2-(2-(thiophen-2-yl)ethyl)-3*H*-imidazo[4,5-*b*]pyridin-7-amine | |
| *Example 49* | 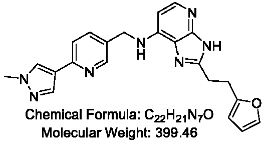 |
| 2-(2-(furan-2-yl)ethyl)-*N-*((6-(1-methyl-1*H*-pyrazol-4-yl)pyridin-3-yl)methyl)-3*H*-imidazo[4,5-*b*]pyridin-7-amine | |
| *Example 50* | 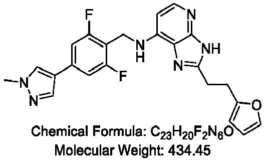 |
| *N*-(2,6-difluoro-4-(1-methyl-1*H*-pyrazol-4-yl)benzyl)-2-(2-(furan-2-yl)ethyl)-3*H*-imidazo[4,5-*b*]pyridin-7-amine | |
| *Example 51* | 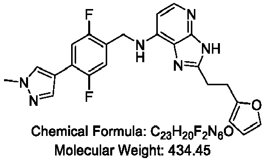 |
| *N*-(2,5-difluoro-4-(1-methyl-1*H*-pyrazol-4-yl)benzyl)-2-(2-(furan-2-yl)ethyl)-3*H*-imidazo[4,5-*b*]pyridin-7-amine | |
| *Example 52* | 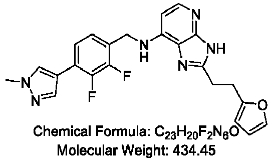 |
| *N*-(2,3-difluoro-4-(1-methyl-1*H*-pyrazol-4-yl)benzyl)-2-(2-(furan-2-yl)ethyl)-3*H*-imidazo[4,5-*b*]pyridin-7-amine | |
| *Example 53* | 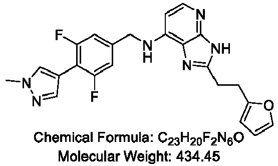 |
| *N*-(3,5-difluoro-4-(1-methyl-1*H*-pyrazol-4-yl)benzyl)-2-(2-(furan-2-yl)ethyl)-3*H*-imidazo[4,5-*b*]pyridin-7-amine | |
| *Example 54* | 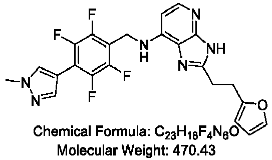 |
| 2-(2-(furan-2-yl)ethyl)-*N-*(2,3,5,6-tetrafluoro-4-(1-methyl-1*H*-pyrazol-4-yl)benzyl)-3*H*-imidazo[4,5-b]pyridin-7-amine | |
| *Example 55* | 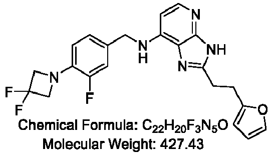 |
| *N-*(4-(3,3-difluoroazetidin-1-yl)-3-fluorobenzyl)-2-(2-(furan-2-yl)ethyl)-3*H*-imidazo[4,5-*b*]pyridin-7-amine | |
| *Example 56* | 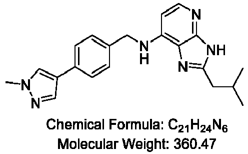 |
| 2-isobutyl-*N-*(4-(1-methyl-1*H*-pyrazol-4-yl)benzyl)-3*H-*imidazo[4,5-*b*]pyridin-7-amine | |
| *Example 57* | 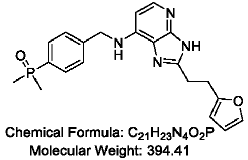 |
| (4-(((2-(2-(furan-2-yl)ethyl)-3*H*-imidazo[4,5-*b*]pyridin-7-yl)amino)methyl)phenyl)dimethyl phosphine oxide | |
| *Example 58* | 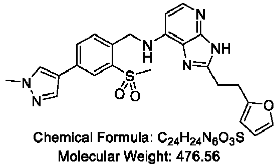 |
| 2-(2-(furan-2-yl)ethyl)-*N-*(4-(1-methyl-1*H*-pyrazol-4-yl)-2-(methylsulfonyl)benzyl)-3*H*-imidazo[4,5-*b*]pyridin-7-amine | |
| *Example 59* | 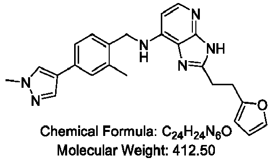 |
| 2-(2-(furan-2-yl)ethyl)-*N*-(2-methyl-4-(1-methyl-1*H-*pyrazol-4-yl)benzyl)-3*H*-imidazo[4,5-*b*]pyridin-7-amine | |
| *Example 60* | 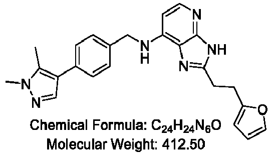 |
| *N-*(4-(1,5-dimethyl-1*H*-pyrazol-4-yl)benzyl)-2-(2-(furan-2-yl)ethyl)-3*H*-imidazo[4,5-*b*]pyridin-7-amine | |
| *Example 61* | 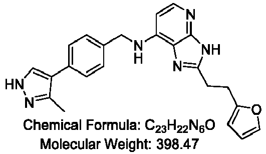 |
| 2-(2-(furan-2-yl)ethyl)-*N*-(4-(3-methyl-1*H*-pyrazol-4-yl)benzyl)-3*H*-imidazo[4,5-*b*]pyridin-7-amine | |
| *Example 62* | 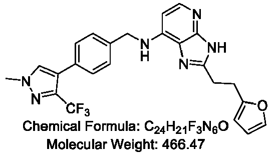 |
| 2-(2-(furan-2-yl)ethyl)-*N-*(4-(1-methyl-3-(trifluoromethyl)-1*H*-pyrazol-4-yl)benzyl)-3*H-*imidazo[4,5-b]pyridin-7-amine | |
| *Example 63* | 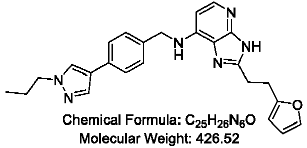 |
| *N-*(4-(1-propyl-1*H*-pyrazol-4-yl)benzyl)-2-(2-(furan-2-yl)ethyl)-3*H*-imidazo[4,5-*b*]pyridin-7-amine | |
| *Example 64* | 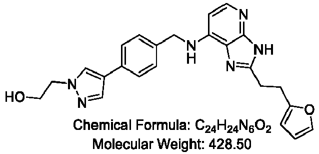 |
| 2-(4-(4-(((2-(2-(furan-2-yl)ethyl)-3*H*-imidazo[4,5-*b*]pyridin-7-yl)amino)methyl)phenyl)-1*H*-pyrazol-1-yl)ethan-1-ol | |
| *Example 65* | 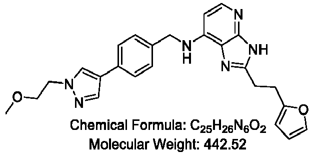 |
| 2-(2-(furan-2-yl)ethyl)-*N-*(4-(1-(2-methoxyethyl)-1*H-*pyrazol-4-yl)benzyl)-3*H*-imidazo[4,5-*b*]pyridin-7-amine | |
| *Example 66* | 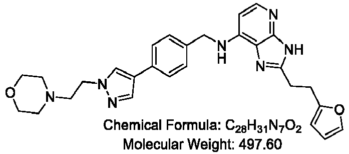 |
| 2-(2-(furan-2-yl)ethyl)-*N*-(4-(1-(2-morpholinoethyl)-1*H*-pyrazol-4-yl)benzyl)-3*H*-imidazo[4,5-*b*]pyridin-7-amine | |
| *Example 67* | 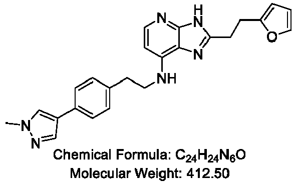 |
| 2-(2-(furan-2-yl)ethyl)-*N*-(4-(1-methyl-1*H*-pyrazol-4-yl)phenethyl)-3*H*-imidazo[4,5-*b*]pyridin-7-amine | |
| *Example 68* | 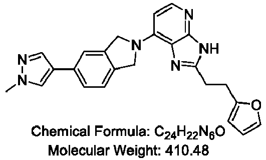 |
| 2-(2-(furan-2-yl)ethyl)-7-(5-(1-methyl-1*H*-pyrazol-4-yl)isoindolin-2-yl)-3*H*-imidazo[4,5-*b*]pyridine | |
| *Example 69* | 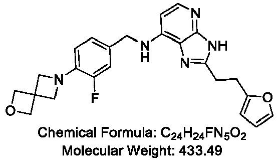 |
| *N-*(3-fluoro-4-(2-oxa-6-azaspiro[3.3]heptan-6-yl)benzyl)-2-(2-(furan-2-yl)ethyl)-3*H*-imidazo[4,5-*b*]pyridin-7-amine | |
| *Example 70* | 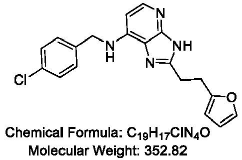 |
| *N-*(4-chlorobenzyl)-2-(2-(furan-2-yl)ethyl)-3*H-*imidazo[4,5-b]pyridin-7-amine | |
| *Example 71* | 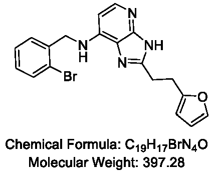 |
| *N*-(2-bromobenzyl)-2-(2-(furan-2-yl)ethyl)-3*H-*imidazo[4,5-*b*]pyridin-7-amine | |
| *Example 72* | 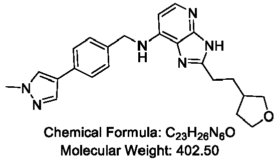 |
| *N*-(4-(1-methyl-1*H*-pyrazol-4-yl)benzyl)-2-(2-(tetrahydrofuran-3-yl)ethyl)-3*H*-imidazo[4,5-*b*]pyridin-7-amine | |
| *Example 73* | 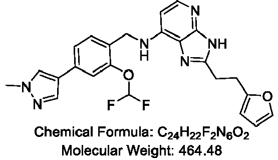 |
| *N-*(2-(difluoromethoxy)-4-(1-methyl-1*H*-pyrazol-4-yl)benzyl)-2-(2-(furan-2-yl)ethyl)-3*H*-imidazo[4,5-*b*]pyridin-7-amine | |
| *Example 74* | 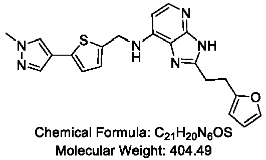 |
| 2-(2-(furan-2-yl)ethyl)-*N-*((5-(1-methyl-1*H*-pyrazol-4-yl)thiophen-2-yl)methyl)-3*H*-imidazo[4,5-*b*]pyridin-7-amine | |
| *Example 75* | 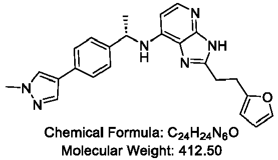 |
| (*S*)-2-(2-(furan-2-yl)ethyl)-*N*-(1-(4-(1-methyl-1*H-*pyrazol-4-yl)phenyl)ethyl)-3*H*-imidazo[4,5-*b*]pyridin-7-amine | |
| *Example 76* | 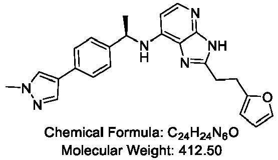 |
| (*R*)-2-(2-(furan-2-yl)ethyl)-*N-*(1-(4-(1-methyl-1*H-*pyrazol-4-yl)phenyl)ethyl)-3*H*-imidazo[4,5-*b*]pyridin-7-amine | |
| *Example 77* | 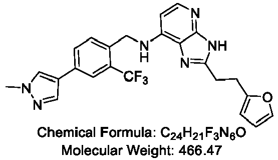 |
| 2-(2-(furan-2-yl)ethyl)-*N-*(4-(1-methyl-1*H*-pyrazol-4-yl)-2-(trifluoromethyl)benzyl)-3*H*-imidazo[4,5-*b*]pyridin-7-amine | |
| *Example 78* | 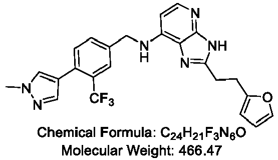 |
| 2-(2-(furan-2-yl)ethyl)-*N*-(4-(1-methyl-1*H*-pyrazol-4-yl)-3-(trifluoromethyl)benzyl)-3*H*-imidazo[4,5-*b*]pyridin-7-amine | |
| *Example 79* | 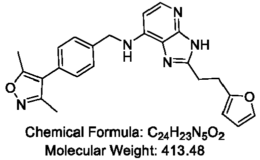 |
| *N*-(4-(3,5-dimethylisoxazol-4-yl)benzyl)-2-(2-(furan-2-yl)ethyl)-3*H*-imidazo[4,5-*b*]pyridin-7-amine | |
| *Example 80* | 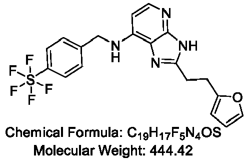 |
| 2-(2-(furan-2-yl)ethyl)-*N-*(4-(pentafluoro-λ6-sulfaneyl)benzyl)-3*H*-imidazo[4,5-*b*]pyridin-7-amine | |
| *Example 81* | 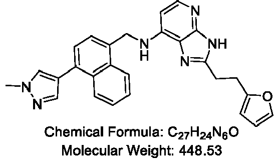 |
| 2-(2-(furan-2-yl)ethyl)-*N-*((4-(1-methyl-1*H*-pyrazol-4-yl)naphthalen-1-yl)methyl)-3*H*-imidazo[4,5-*b*]pyridin-7-amine | |
| *Example 82* | 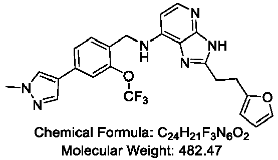 |
| 2-(2-(furan-2-yl)ethyl)-*N*-(4-(1-methyl-1*H*-pyrazol-4-yl)-2-(trifluoromethoxy)benzyl)-3*H*-imidazo[4,5-*b*]pyridin-7-amine | |
| *Example 83* | 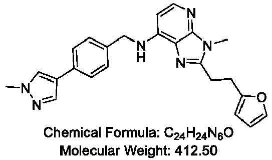 |
| 2-(2-(furan-2-yl)ethyl)-3-methyl-*N*-(4-(1-methyl-1*H-*pyrazol-4-yl)benzyl)-3*H*-imidazo[4,5-*b*]pyridin-7-amine | |
| *Example 84* | 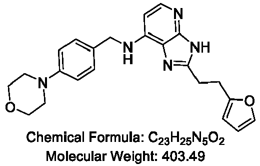 |
| 2-(2-(furan-2-yl)ethyl)-*N*-(4-morpholinobenzyl)-3*H-*imidazo[4,5-*b*]pyridin-7-amine | |
| *Example 85* | 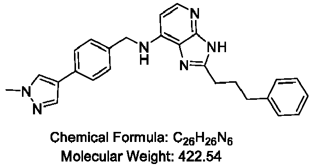 |
| *N*-(4-(1-methyl-1*H*-pyrazol-4-yl)benzyl)-2-(3-phenylpropyl)-3*H*-imidazo[4,5-*b*]pyridin-7-amine | |
| *Example 86* | 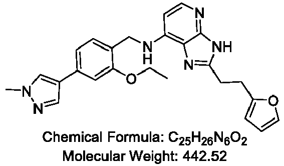 |
| *N*-(2-ethoxy-4-(1-methyl-1*H*-pyrazol-4-yl)benzyl)-2-(2-(furan-2-yl)ethyl)-3*H*-imidazo[4,5-*b*]pyridin-7-amine | |
| *Example 87* | 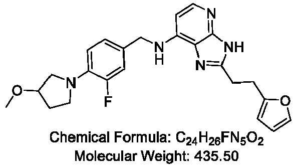 |
| *N-*(3-fluoro-4-(3-methoxypyrrolidin-1-yl)benzyl)-2-(2-(furan-2-yl)ethyl)-3*H*-imidazo[4,5-*b*]pyridin-7-amine | |
| *Example 88* | 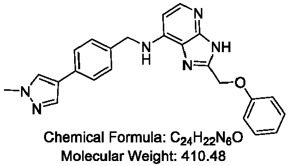 |
| *N*-(4-(1-methyl-1*H*-pyrazol-4-yl)benzyl)-2-(phenoxymethyl)-3*H*-imidazo[4,5-*b*]pyridin-7-amine | |
| *Example 89* | 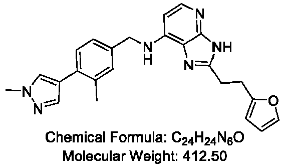 |
| 2-(2-(furan-2-yl)ethyl)-*N-*(3-methyl-4-(1-methyl-1*H-*pyrazol-4-yl)benzyl)-3*H*-imidazo[4,5-*b*]pyridin-7-amine | |
| *Example 90* | 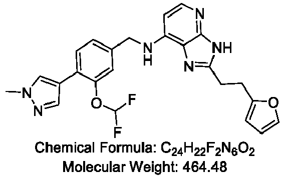 |
| *N*-(3-(difluoromethoxy)-4-(1-methyl-1*H*-pyrazol-4-yl)benzyl)-2-(2-(furan-2-yl)ethyl)-3*H*-imidazo[4,5-*b*]pyridin-7-amine | |
| *Example 91* | 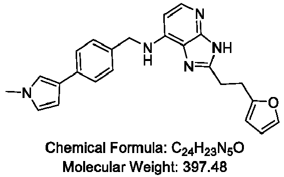 |
| 2-(2-(furan-2-yl)ethyl)-*N-*(4-(1-methyl-1*H*-pyrrol-3-yl)benzyl)-3*H*-imidazo[4,5-*b*]pyridin-7-amine | |
| *Example 92* | 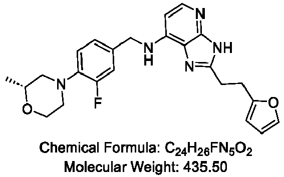 |
| (*R*)-*N-*(3-fluoro-4-(2-methylmorpholino)benzyl)-2-(2-(furan-2-yl)ethyl)-3*H*-imidazo[4,5-*b*]pyridin-7-amine | |
| *Example 93* | 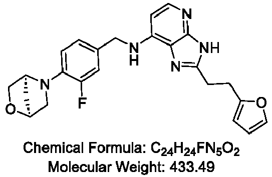 |
| *N-*(4-((1*S*,4*S*)-2-oxa-5-azabicyclo[2.2.1]heptan-5-yl)-3-fluorobenzyl)-2-(2-(furan-2-yl)ethyl)-3*H-*imidazo[4,5-*b*]pyridin-7-amine | |
| *Example 94* | 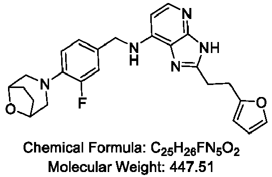 |
| *N*-(4-(8-oxa-3-azabicyclo[3.2.1]octan-3-yl)-3-fluorobenzyl)-2-(2-(furan-2-yl)ethyl)-3*H*-imidazo[4,5-*b*]pyridin-7-amine | |
| *Example 95* | 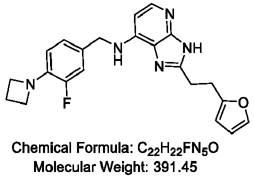 |
| *N*-(4-(azetidin-1-yl)-3-fluorobenryl)-2-(2-(furan-2-yl)ethyl)-3*H*-imidazo[4,5-*b*]pyridin-7-amine | |
| *Example 96* | 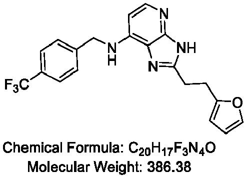 |
| 2-(2-(furan-2-yl)ethyl)-*N*-(4-(trifluoromethyl)benzyl)-3*H*-imidazo[4,5-*b*]pyridin-7-amine | |
| *Example 97* | 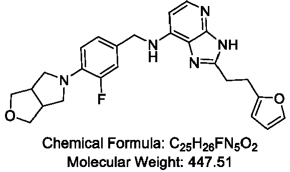 |
| *N*-(3-fluoro-4-(tetrahydro-1*H-*furo[3,4-*c*]pyrrol-5(3*H*)-yl)benzyl)-2-(2-(furan-2-yl)ethyl)-3*H*-imidazo[4,5-*b*]pyridin-7-amine | |
| *Example 98* | 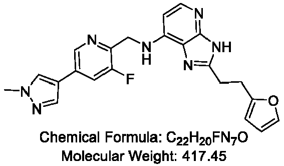 |
| *N-*((3-fluoro-5-(1-methyl-1*H*-pyrazol-4-yl)pyridin-2-yl)methyl)-2-(2-(furan-2-yl)ethyl)-3*H*-imidazo[4,5-*b*]pyridin-7-amine | |
| *Example 99* | 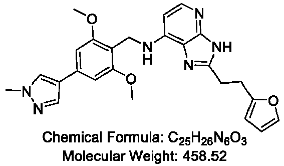 |
| *N*-(2,6-dimethoxy-4-(1-methyl-1*H*-pyrazol-4-yl)benzyl)-2-(2-(furan-2-yl)ethyl)-3*H*-imidazo[4,5-*b*]pyridin-7-amine | |
| *Example 100* | 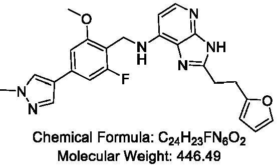 |
| *N*-(2-fluoro-6-methoxy-4-(1-methyl-1*H*-pyrazol-4-yl)benzyl)-2-(2-(furan-2-yl)ethyl)-3*H*-imidazo[4,5-*b*]pyridin-7-amine | |

In one embodiment of the present invention, the compounds according to the invention are the solvates, salts, N-oxides, racemic mixtures, diastereomers, enantiomers, tautomers, isotopically labeled forms which are compounds in which one or more atoms are replaced by a corresponding atom having the same number of protons but differing in the number of neutrons, and combinations thereof of the compounds according to formula (I), (II) or (III).

The nine-membered heterocyclic ring in the compound according to formula (I), (II) or (III) in this invention comprise, as is also evident from the formula itself, at least five carbon atoms and one nitrogen atom. The inventor has been motivated by the finding that another AURKA ligand disclosed in WO2022/096679 comprising an anilide moiety displays poor metabolic stability (Figure 7B), which is detrimental for clinical application. Specifically, the functionally important anilide moiety is prone to hydrolysis. However, all conventional attempts to stabilize this hydrolysis labile anilide motif by either chemical or galenic modifications failed for the flurophenyl series.

Therefore, the present invention is based on the surprising finding that a cyclization of the anilide to a nine-member heterocyclic ring, especially an imidazopyridine stabilizes the AURKA ligand against hydrolysis while maintaining its function. In a preferred embodiment, the compound according to the invention comprises an imidazopyridine ring, wherein N and Y are both nitrogen atoms.

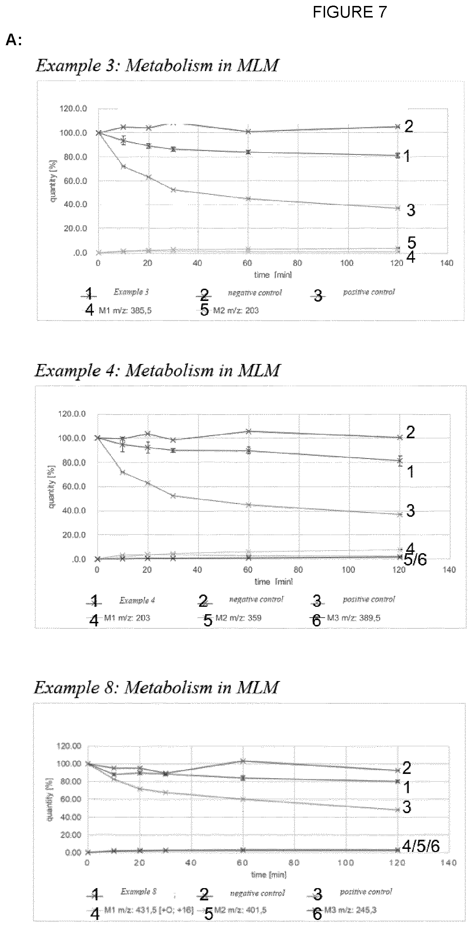

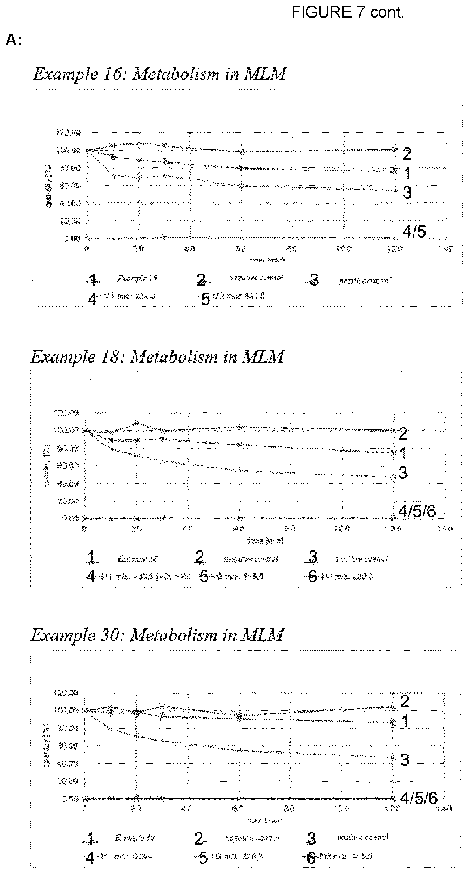

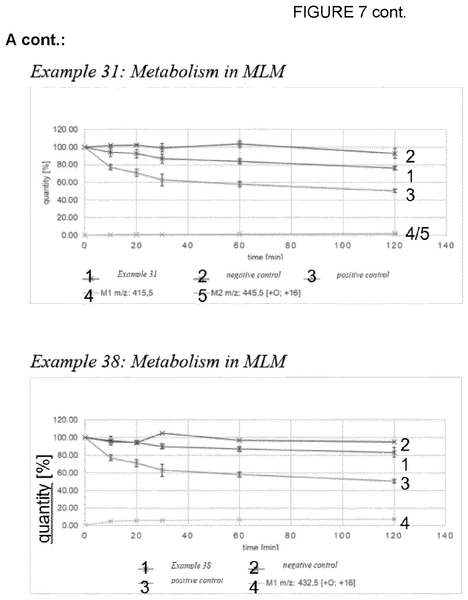

So, an important embodiment of the compound in the present invention is an improved metabolic stability (Figure 7A), characterized by an in vivo or in vitro clearance half-life of at least 2 hours or longer, preferably 5 hours or longer, even more preferably 10 hours or longer, and/or is insensitive to biotransformation by drugmetabolizing enzyme, in particular human hepatic microsomal enzyme, such as CYP.

The clearance time should be understood in the context of the invention as the time it takes for the concentration of the compound to drop below a certain level after administering said compound either in vivo, such as though ingestion of the compound, or in vitro, such as by adding the compound to the culture medium. Accordingly, the clearance half-life refers to the time it takes for the concentration compound to drop to 50% of the concentration at the time of administration, either *in-vivo* or *in-vitro.*

In another important embodiment of the compound according to the invention, a compound comprising a nine-membered heterocyclic ring according to formula (I), (II) or (III) solves the above problems by specifically modulating the conformation of AURKA, thereby modifying the protein-protein interaction of AURKA with binding proteins, such as MYC and/or TPX2. In the context of the current invention, this specific modulating may refer to change in secondary structure or change in protein dynamics, to achieve the functional effect of increase the interaction between AURKA and its binding partners, preferably with TPX2.

Yet another particularly preferred embodiment relates to a compound according to this invention, wherein the compound is capable of binding, preferably of specifically binding, to a serine/threonine kinase. According to this invention, the compound is capable of binding, preferably of specifically binding, to any serine/threonine kinase. A particularly preferred serine/threonine kinase, in the context of this invention, is Aurora Kinase A (AURKA), or a variant thereof.

A further preferred embodiment relates to a compound according to this invention, wherein the compound modulates, stabilizes and/or destabilizes, preferably specifically modulates, stabilizes and/or destabilizes, the conformation of the serine/threonine kinase, preferably of AURKA, or the variant thereof.

Additionally preferred is a compound, wherein the compound modulates, preferably specifically modulates, more preferably specifically stabilizes an interaction of the serine/threonine kinase, preferably of AURKA, or the variant thereof, with at least one binding protein.

The expression "modulates" as used herein means that upon administration of a compound according to this invention, the conformation of AURKA is altered, preferably because the compound is inducing a conformational shift in AURKA. Thereby, the compound is modulating, *i.e.,* altering, the binding of at least one binding protein, such as MYC and/or TPX2, to AURKA. Accordingly, the term "modulates" further refers to inducing a shift in the protein-protein interaction (PPI) of AURKA with at least one binding protein, such as a change of the binding of MYC and/or TPX2 to AURKA.

The expression "stabilizes" as used herein means that upon administration of a compound according to this invention, a certain conformational state of the serine/threonine kinase, preferably of AURKA, is more likely kept in that particular state than altered into a different conformational state, *i.e.,* the likelihood of a conformational shift of the serine/threonine kinase, preferably of AURKA, is reduced or prevented. The term "stabilizes" can also refer to locking a serine/threonine kinase, preferably AURKA, into a particular conformation. The expression "stabilizes" as used herein further refers to inducing an enhanced binding of certain binding proteins to the serine/threonine kinase, preferably to AURKA. For example, the compounds of this invention may stabilize the binding of TPX2 to AURKA.

The expression "destabilizes" as used herein means that upon administration of a compound according to this invention, a certain conformational state of the serine/threonine kinase, preferably of AURKA, is more likely altered into a different conformational state than kept or locked in that particular conformational state, *i.e.,* the likelihood of a conformational shift of the serine/threonine kinase, preferably of AURKA, is enhanced or enforced. The term "destabilizes" can also refer to inducing a conformational shift in the serine/threonine kinase, preferably of AURKA.

Simultaneously, the use of a compound comprising a nine-membered heterocyclic ring according to formula (I), (II) or (III) as above is characterized by a largely reduced or no inhibition of the kinase activity of AURKA, making the novel compounds comprising a nine-membered heterocyclic ring according to formula (I), (II) or (III) of this invention useful for treating various cancer diseases, such as HCC and SCLC.

Additionally, the compound comprising a nine-membered heterocyclic ring is characterized by the high specificity to AURKA, in that it does not bind promiscuously to other kinases, modulate the conformation of other kinases, or inhibit their kinase activities. This is evidenced by the the fact that there was no hit for kinase inhibition from a panel against 345 wt-kinases at Compound-Concentrations of 300 nM and 1000 nM.

The *in-vivo* efficacy of the compound according to the present invention has been demonstrated by the inventor using different mouse models. In a mouse lung cancer model, in particular a mouse small cell lung cancer (SCLC) model, the compound according to the present invention is shown to reduce the tumor volume in a small cell lung cancer mouse model by at least 75%, and improve subject survival duration by at least 50%. In a mouse liver cancer model, the compound according to the present invention is shown to improve the subject survival duration by at least 40% compared with a control, and by at least 17% compared with another drug Alisertib.

As used herein, the term "therapeutically effective amount" shall refer to the amount of a compound or a combination of different compounds that will elicit the biological or medical response of a tissue, system, animal or human subject that is being sought, for instance, by a researcher or clinician, in accordance with the herein disclosed invention. Furthermore, the term "therapeutically effective amount" means any amount which, as compared to a corresponding subject who has not received such an amount of a compound or a combination of different compounds according to this invention, results in improved treatment, healing, prevention, or amelioration of a disease, disorder, or side effect, or a decrease in the rate of advancement of a disease or disorder.

The invention extends isotope labelled forms of the compounds comprising a nine-membered heterocycle according to formula (I), and/or a compound having formula (II), and/or a compound having formula (III), or any other compound of this invention. As used herein, the term "isotope" refer to atoms of the same element that have the same number of protons, but a different number of neutrons. The term "isotope labeling" therefore accordingly referred substituting an atom with another of the same element, *i.e.* with the same proton number, but a different number of neutrons. For example, deuterium is an isotope of hydrogen comprising one proton, and one neutron. A "deuterium labeled" compound therefore refer to a compound with the same chemical structure, but with one or more hydrogen atom being substituted by deuterium. As used herein, the terms "isotope labeled" and "isotope substituted" are interchangeable.

In the invention, the term "Example X ", wherein X is any natural number, shall refer to the example compound with the corresponding number X as listed in Table 1. For example, Example 1, if according to the context should refer to a compound would refer to the compound with the IUPAC naming of *N*-(4-(1*H*-pyrazol-4-yl)benzyl)-2-(phenoxymethyl)-1*H*-benzo[*d*]imidazol-5-amine, as defined in Table 1.

In another important embodiment, the use of a compound comprising a nine-membered heterocyclic ring according to formula (I), (II) or (III) as above is characterized by a superior therapeutic index. In the context of the current invention, a therapeutic index is a measurement of the margin of safety that exists between the dose of a drug that produces the desired effect and the dose that produces unwanted and possibly dangerous side effects. Formally, it can be defined as the ratio between the median lethal dose (LD50) and the median effective dose (ED50) in animals or defined as the ratio between the median growth inhibition concentration of healthy cells and the effective concentration in killing of disease cells, specifically cancer cells. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

In one related embodiment, the compound comprising a nine-membered heterocyclic ring according to formula (I), (II) or (III) is non-toxic to healthy cells, with median growth inhibition concentration of at least 10 uM or higher, preferably 20 uM or higher, or even more preferably 50 uM or higher in healthy cells. In the present invention, the median growth inhibition concentration is the concentration required to kill half of the test subjects, such as healthy cells, after a certain test duration.

In one related embodiment, the compound comprising a nine-membered heterocyclic ring according to formula (I), (II) or (III) is effective in inhibiting the growth of diseased cells, preferably cancer cells, with median effective concentration of at most 1000 nM or lower, preferably 100 nM or lower, or even more preferably 10 nM or lower. In the present invention, the median effective concentration is the concentration required to eliminate half of the diseased cells, such as cancer cells, after a certain test duration.

Therefore, in an important embodiment, the compound comprising a nine-membered heterocyclic ring according to formula (I), (II) or (III) has a therapeutic index of at least 10 or higher, preferably 20 or higher, more preferably 50 or higher, more preferably 100 or higher or even more preferably 500 or higher.

Additionally, the invention shall further include any pharmaceutically acceptable salt of the compounds mentioned above. The pharmaceutically acceptable salts are either acid or base addition salts of a compound as above, with pharmaceutically acceptable acids or bases. According to this invention, any suitable pharmaceutically acceptable organic and inorganic acid may be used, such as, for example, sulfuric acid, sulfamic acid, hydrochloric acid, hydrobromic acid, phosphoric acid, dicarboxylic acids, tricarboxylic acids and hydroxycarboxylic acids having 2 to 10 carbon atoms, such as oxalic acid, malonic acid, maleic acid, fumaric acid, lactic acid, citric acid, and benzoic acid, CrC4-alkylsulfonic acids, such as methanesulfonic acid, cycloaliphatic sulfonic acids, such as S-(+)-10-camphor sulfonic acid, or aromatic sulfonic acids, such as benzenesulfonic acid and toluenesulfonic acid. Further, any suitable pharmaceutically acceptable organic and inorganic base may be used according to this invention, such as, for example, ammonium hydroxide, alkali metal hydroxides, such as sodium hydroxide or potassium hydroxide, alkaline earth metal hydroxides, such as calcium or magnesium hydroxide, or organic nitrogen bases, such as dimethylamine, trimethylamine, ethanolamine, diethanolamine, triethanolamine, choline, procaine, arginine, or ethylenediamine.

According to the present invention, solvates of the above compounds are also within the scope of this invention. One example of solvates are hydrates, but any other solvate shall also be included.

It is to be understood that in any embodiment of the invention, where the compound comprising a nine-membered heterocycle according to formula (I), (II) or (III), and/or a compound having formula (II), and/or a compound having formula (III), comprises at least one chiral center, e.g. an asymmetric carbon atom, the invention extends to all the optical isomers thereof, as well as to racemates and to isomer mixtures thereof, wherein any isomer or any number of isomers may be present in any amount. Examples of optical isomers are enantiomers and diastereomers, which shall also be included in the present invention. Further, the invention also extends to all tautomeric, crystal and/or polymorphic forms of the compounds comprising a nine-membered heterocycle according to formula (I), and/or a compound having formula (II), and/or a compound having formula (III), or any other compound of this invention.

According to the present invention, prodrugs of the compounds above shall also be included. The term "prodrug" or "pro-drug" shall relate to a compound or an agent that can be transformed into the actual active compound. Such transformation usually occurs in the human or animal body through a physicochemical transformation process, such as an enzymatic or chemical cleavage. Candidate enzymes that could be utilized to activate the prodrugs according to the present invention include lipases, proteases or glycosidases. Importantly, prodrugs can be designed so that the transformation process occurs in particular tissues or regions of the human or animal body. Without being limited thereto, an example of a prodrug of a compound according to the present invention is any compound as above in form of an ester, or a compound comprising a carboxylic acid substituent wherein the free hydrogen is replaced by an alkyl, such as alkoxycarbonyloxymethyl having from 3 to 6 carbon atoms, 1-methyl-1-(alkoxycarbonyloxy)-ethyl having from 5 to 8 carbon atoms, (Ci-C6)alkanoyloxy-methyl, piperidino-, pyrrolidino- or morpholino(C2-C3)alkyl, or arylactyl.

According to the present invention, it might be beneficial to use a prodrug of a compound of this invention, because said prodrug might be more soluble in a liquid, such as more water soluble, than the ultimate active compound. Also, a different route of administration available for a pro-drug might be beneficial over the required route of administration of the final drug. For example, a pro-drug may be administrable by intravenous administration. Moreover, the penetration of certain organs might differ between final drug and prodrug. For example, a prodrug might be able to cross the blood-brain-barrier, whereas the active end-drug might not. Also, bioavailability of a prodrug might be advantageous over bioavailability of the final drug.

According to this invention, the term "heteroatom" shall refer to an atom selected from oxygen, nitrogen, and sulfur. According to this invention, any group containing heteroatoms may contain either 1, or 2, or 3 heteroatoms selected from oxygen, nitrogen, and sulfur. The 1, or 2, or 3 heteroatoms may be either the same or different.

The term "nine-membered ring" as used herein means that the ring has nine ring atoms. Accordingly, any term used herein in the form of "x-membered ring", with x being any integer number, shall refer a ring with x number of ring atoms. E.g., for purine x is 9.

The term "ring" used herein shall mean either to a simple cycle of atoms and bonds in a molecule or to a connected set of atoms and bonds in which every atom and bond is a member of a cycle. Said ring may encompass both aromatic and non-aromatic ring system. In particular, they can include both heterocyclic and homocyclic groups.

A heterocyclic group includes at least one heteroatom, i.e an atom selected from oxygen, nitrogen and sulfur, and may be saturated or partially unsaturated. A homocyclic group comprise atoms of one type, and is typically cyclic hydrocarbon group.

The term "aromatic" as used herein shall be used to describe a compound, a chemical group or moiety having aromaticity, which shall normally mean a cyclic (ring-shaped) structure with pi-bonds in resonance, or in other words, contains delocalized electron.

Such aromatic rings can be homocyclic, meaning they may contain ring-forming atoms of the same type. Preferably, such homocyclic ring contains 3 to 14 (e.g., 5, 6, 7, 8, 9, or 10, such as 5, 6, or 10) carbon atoms which can be arranged in one ring (e.g., phenyl) or two or more condensed rings (e.g., naphthyl). Exemplary homocyclic aromatic ring groups include cyclopropenylium, cyclopentadienyl, phenyl, indenyl, naphthyl, azulenyl, fluorenyl, anthryl, and phenanthryl.

Such aromatic rings can also be heterocyclic, which means an aromatic ring as defined above, in which one or more carbon atoms in the aryl group are replaced by heteroatoms (such as O, S, or N). In particular, a heteroaromatic or heteroaryl group comprises at least one nitrogen atom as a ring member. In addition, the heteroaromatic or heteroaryl group can, besides the at least one nitrogen atom, further include an oxygen and/or a sulfur atom as ring member.

The term "non-aromatic ring" represents cyclic non-aromatic rings with preferably 3 to 14 carbon atoms, such as 3 to 12 or 3 to 10 carbon atoms. They could typically be "cycloalkyl" group which represents cyclic version of alkyl groups. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" is also meant to include bicyclic and tricyclic versions thereof.

A "substituted ring" or "substitute cycle" means that one or more (such as 1 to the maximum number of hydrogen atoms bound to a ring-forming atom, e.g., 1, 2, 3, 4, 5, 6, 7, 8, 9, or up to 10, such as between 1 to 5, 1 to 4, or 1 to 3, or 1 or 2) hydrogen atoms bonded to any ring-forming atoms are replaced with a substituent other than hydrogen (when more than one hydrogen atom is replaced the substituents may be the same or difFerent).

In direct opposite sense to it, the term "non-substituted ring", "non-substituted cycle", "unsubstituted ring" or "unsubstituted cycle" means that none of the hydrogen atoms bonded to any ring-forming atoms is replaced.

The term "alkyl" shall refer to any straight-chain or branched carbon group, such as methyl, ethyl, isopropyl, n-propyl, 2-butyl, isobutyl, n-butyl, pentyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1-ethyl-1-methylpropyl, or 1-ethyl-2-methylpropyl. The term "alkyl" shall also include any group which comprises an alkyl group, as named above. Alkyl shall preferably refer to a carbon group having - unless otherwise stated - 1 to 8, in particular 1 to 6, and more preferably 1 to 4 carbon atoms. In some embodiments, methyl is preferred.

The term "halogen" shall refer to an atom selected from fluorine (F), chlorein (Cl), bromine (Br), iodine (I) and astatine (At)

The terms "of the [present] invention", "in accordance with the invention", "according to the invention" and the like, as used herein are intended to refer to all aspects and embodiments of the invention described and/or claimed herein.

**A second aspect** of this invention relates to a pharmaceutical composition comprising a compound according to this invention, and a pharmaceutically acceptable carrier, stabilizer and/or excipient. Pharmaceutical excipients that may be added include, without being limited thereto, preservatives, antioxidants, and antimicrobial agents; wetting, emulsifying, and suspending agents; fragrance and colouring additives; compositions for improving compressibility; compositions for creating a delayed, sustained, or controlled release of the active ingredient; and various salts to change the osmotic pressure of the pharmaceutical preparation, or to act as a buffer. Such excipients are well known to the skilled artisan.

The compounds of this invention are customarily administered in the form of pharmaceutical compositions, which comprise at least one compound comprising a nine-membered heterocycle according to formula (I), and/or a compound having formula (II), and/or a compound having formula (III), optionally together with an inert carrier (e.g. a pharmaceutically acceptable excipient) and, where appropriate, other compounds and/or drugs.

The skilled artisan is aware of suitable forms of pharmaceutical compositions, for example solid forms, such as powders, granules, tablets, in particular film tablets, lozenges, sachets, cachets, sugar-coated tablets, capsules, such as hard gelatin capsules and soft gelatin capsules, or suppositories, semisolid medicinal forms, such as ointments, creams, hydrogels, pastes or plasters, or liquid medicinal forms, such as solutions, emulsions, in particular oil-in- water emulsions, suspensions, for example lotions, injection preparations and infusion preparations. In addition, it is also possible to use liposomes or microspheres.

In a particularly preferred embodiment, a pharmaceutical composition comprising at least one compound comprising a nine-membered heterocycle according to formula (I), and/or a compound having formula (II), and/or a compound having formula (III), a pharmaceutically acceptable salt, solvate or optical isomer thereof, is provided in form of a gel, an ointment, a salve, a cream, a tablet, a pill, a capsule, a troche, a dragée, a powder, an aerosol spray, a nasal spray, a suppository, and/or a solution.

When producing a pharmaceutical composition according to this invention, at least one compound comprising a nine-membered heterocycle according to formula (I), and/or a compound having formula (II), and/or a compound having formula (III), is optionally mixed or diluted with one or more carriers and/or excipients. Carriers and/or excipients, according to this invention, can be solid, semi-solid or liquid materials, which may serve as a vehicle, a carrier or a medium for the active compound.

Further, a pharmaceutical composition according to this invention might further comprise at least one acceptable auxiliary substance, such as a wetting agent, an emulsifying agent, a suspending agent, a preservative, an antioxidant, an anti-irritant, a chelating agent, a coating agent, an emulsion stabilizer, a gel former, a resin, a solvent, a solubilizer, a neutralizing agent, a pigment, a silicone derivative, a binder, a filler, a drying agent, a thickener, a wax, a plasticizer, or the like.

**A further aspect** of this invention pertains to a compound according to this invention, or a pharmaceutical composition comprising a compound according to this invention, for use in medicine.

**Yet another aspect** of this invention relates to a compound according to this invention, or a pharmaceutical composition comprising a compound according to this invention, for use in the prevention and/or treatment of a proliferative disease, such as cancer, preferably wherein the cancer is a solid cancer, such as a cancer stemming from any solid organ tissue, or a liquid cancer.

The term "treatment" shall refer to a partial or total inhibition and/or reduction of symptoms of a particular disease, such as a proliferative disease, like cancer, in a subject, as well as a partial or total destruction of, for example, diseased cells, or cancer cells. The term "prevention" shall refer to preventing or delaying the onset of a clinically evident disease, such as a proliferative disease, like cancer, in a subject. In the context of the present invention, prevention and/or treatment shall include both preventive and/or actual treatment of the disease symptoms of a disease, such as a proliferative disease, like cancer, which can be alleviated and/or even completely removed using said treatment.

A "proliferative disease" in the context of the present invention shall preferably refer to a disease such as a cancer or a tumor disease. Cancer diseases or tumor diseases that can be treated by a compound or pharmaceutical composition of the present invention include, but are not limited to, cancer or tumor diseases selected from the group of liver cancer, hepatocellular carcinoma (HCC), lung cancer, small cell lung cancer (SCLC), bladder cancer, ovarian cancer, uterine cancer, endometrial cancer, breast cancer, gastric cancer, urinary bladder cancer, renal cancer, pancreatic cancer, stomach cancer, cervical cancer, cephalic cancer, thyroid cancer, esophageal cancer, medulloblastoma, head and neck cancer, lymphoma, leukemia, acute myeloid leukemia, acute lymphocytic leukemia, chronic lymphocytic leukemia, chronic myeloid cancer, prostate cancer, salivary gland cancer, bone cancer, brain cancer, glioma, colon cancer, rectal cancer, colorectal cancer, kidney cancer, skin cancer, melanoma, glioblastoma, squamous cell carcinoma, pleomorphic adenoma, endometrioma, nasopharynx cancer, small intestine cancer, testicular cancer, Hodgkin lymphoma, non-Hodgkin lymphoma, cancer of the anus, cancer of the anal canal, cancer of the anorectum, cancer of the oropharynx, vaginal carcinoma, vulval carcinoma, enteric cancer, endocrine gland cancer, adrenal cancer, urethral cancer, lymphocytoma, cystic cancer, nephritic cancer, hydrouretic cancer, renal cell carcinoma, renal pelvic carcinoma, hypophyseal adenomatosis, adenocarcinoma, and/or a MYC-dependent tumor. Preferred cancers are liver cancer, more preferably hepatocellular carcinoma (HCC), even more preferably TP53 deficient or mutant HCC, and lung cancer, in particular small cell lung cancer (SCLC), preferably TP53/RB1 mutant and/or deficient SCLC.

Yet another preferred embodiment relates to the compound for use in the treatment and/or prevention of a proliferative disease in a subject, wherein the proliferative disease is a cancer which is refractory or resistant to treatment with at least one prior therapy.

For the prevention and/or treatment of a disease according to this invention, the appropriate dosage of a compound according to this invention will depend on a multitude of different variables, such as the specific type of disease, in particular the specific type of the cancer disease to be treated, the severity and course of the disease, in particular the severity and course of the specific cancer disease, previous therapy, the patient's clinical history, age, size/weight, sex, and response to the compound according to this invention.

A particularly preferred embodiment relates to a compound or a pharmaceutical composition comprising a compound as above for use according to this invention, wherein the compound is modulating, preferably specifically modulating, or more preferably specifically stabilizing, an interaction of a serine/threonine kinase, preferably AURKA, or a variant thereof, with at least one binding protein in a cell, such as in a human cancer cell, preferably wherein the serine/threonine kinase is AURKA, or a variant thereof, and the at least one binding protein is TPX2, thereby preventing and/or treating the proliferative disease, such as cancer, in the subject.

Further preferred is that the modulating, preferably specifically stabilizing, of an interaction of a serine/threonine kinase, preferably AURKA, or a variant thereof, with at least one binding protein in a cell, induces the death of at least one tumor cell. Also preferred is a compound according to this invention, or a pharmaceutical composition comprising a compound according to this invention, wherein the compound enters a cell by diffusion, and then modulates, preferably specifically stabilize, the interaction of a serine/threonine kinase, preferably AURKA, or a variant thereof, with the at least one binding protein in the cell. Preferably, said modulating, preferably specifically stabilizing, of the interaction of the serine/threonine kinase, preferably AURKA, or the variant thereof, then induces the death of at least one tumor cell.

In an additionally preferred embodiment, the subject to be treated is a mammal, such as a mouse, rat, guinea pig, rabbit, cat, dog, monkey, or a human, preferably a human, for example a human patient, more preferably a human patient suffering from a proliferative disease, such as cancer. As will be further described herein, the subject or patient subjected to the treatment or uses of the invention in preferred embodiments is suffering from a proliferative disease, such as cancer. In a further preferred embodiment, the subject is suffering from a recurrent cancer disease.

Yet another embodiment relates to a subject, wherein the subject is at risk of having a proliferative disease, such as cancer. As used herein, the term "being at risk" refers to having a risk that is higher, preferably significantly higher, of developing a proliferative disease, such as cancer, compared to the majority of a matched group defined by basic medical factors such as age, gender, weight, and so on, which is well-known to the skilled person. The subject may be at risk due to a genetic predisposition, or due to exposure to carcinogenic agents, such as ionizing radiation or chemical mutagens. In case of lung cancer, a subject may also be at risk due to cigarette smoking.

**Yet another aspect** of this invention relates to a method for treating and/or preventing a proliferative disease, such as cancer, in a subject, comprising administering to the subject a therapeutically effective amount of a compound, and/or of a pharmaceutical composition according to this invention. The cancer to be treated and/or prevented is preferably a cancer as defined herein above.

Cancer diseases that can be treated by the compound, the combination, and/or the pharmaceutical composition of the present invention also include recurrent cancer diseases. The term "recurrent cancer diseases", as disclosed herein, comprises a recurrent cancer selected from the group, but not limited to, liver cancer, hepatocellular carcinoma (HCC), lung cancer, small cell lung cancer (SCLC), bladder cancer, ovarian cancer, uterine cancer, endometrial cancer, breast cancer, gastric cancer, urinary bladder cancer, renal cancer, pancreatic cancer, stomach cancer, cervical cancer, cephalic cancer, thyroid cancer, esophageal cancer, medulloblastoma, head and neck cancer, lymphoma, leukemia, acute myeloid leukemia, acute lymphocytic leukemia, chronic lymphocytic leukemia, chronic myeloid cancer, prostate cancer, salivary gland cancer, bone cancer, brain cancer, glioma, colon cancer, rectal cancer, colorectal cancer, kidney cancer, skin cancer, melanoma, glioblastoma, squamous cell carcinoma, pleomorphic adenoma, endometrioma, nasopharynx cancer, small intestine cancer, testicular cancer, Hodgkin lymphoma, non-Hodgkin lymphoma, cancer of the anus, cancer of the anal canal, cancer of the anorectum, cancer of the oropharynx, vaginal carcinoma, vulval carcinoma, enteric cancer, endocrine gland cancer, adrenal cancer, urethral cancer, lymphocytoma, cystic cancer, nephritic cancer, hydrouretic cancer, renal cell carcinoma, renal pelvic carcinoma, hypophyseal adenomatosis, and/or adenocarcinoma. Preferred recurrent cancers to be treated by the compound of the present invention are recurrent liver cancer, more preferably recurrent hepatocellular carcinoma (HCC), and recurrent lung cancer, in particular recurrent small cell lung cancer (SCLC).

**Yet another aspect** of this invention pertains to the use of a compound or a pharmaceutical composition according to this invention, in the manufacture of a medicament against a proliferative disease, such as cancer.

**A further aspect** of this invention relates to a kit comprising:
a compound, a combination of compounds, or a pharmaceutical composition according to this invention, and written instructions to apply the compound, the combination of compounds, or the pharmaceutical composition, optionally, a container holding the compound, the combination of compounds, or the pharmaceutical composition, and the written instructions.

**Another aspect** of this invention pertains to a method for producing a compound according to the present invention. Said method may be for example chemical synthesis.

In a preferred embodiment of the method for producing a compound according to the present invention, said compound is of the formula (A), and 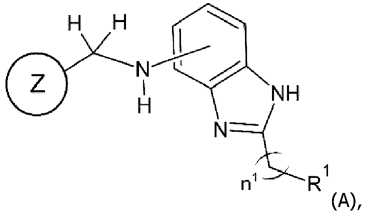 wherein Z has the formula (ZI) 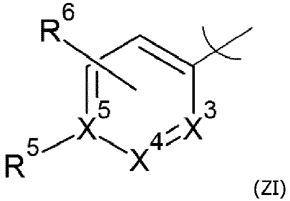 Wherein
X³, X⁴ and X⁵ are C; and wherein R⁵ is selected from
H, halogen, particularly Br; Cl and F; C1-3-alkyl, in particular isoprop-2-yl; C1-3-alkoxy, in particular methoxy-1-yl; diC1-3-alkylamine group, in particular N,N-dimethylamin-1-yl; N,N-(Dimethy(*d*₃))amin-1-yl; diC₁₋₃-alkyl phosphine oxide, in particular dimethylphosphine oxide, halogen substituted sulfane, in particular pentafluoro-A6-sulfane;
substituted and/or unsubstituted five-membered hetero and/or homocycles, in particular substituted and/or unsubstituted pyrrole, particularly,1-methyl-1*H*-pyrrole-3-yl; hexahydro-1*H*-furo[3,4-*c*]pyrrole-1-yl,substituted and/or unsubstituted Oxazole, in particular 3,5-dimethylisoxazol-4-yl; substituted and/or unsubstituted imidazole, in particular 1-methyl-1*H*-imidazol-4-yl; 4-methyl-1*H-*imidazol-1-yl; substituted and/or unsubstituted pyrazole, in particular 1-cyclopropyl-1*H*-pyrazol-4-yl; 1,3-dimethy-1*H*-pyrazol-4-yl; 1,5-dimethyl-1*H*-pyrazole-4-yl; 1,3,5-trimethyl-1*H*-pyrazol-4-yl; 1-difluormethyl-1*H*-pyrazol-4-yl; 1-ethyl-1*H*-pyrazol-4-yl; 1-(2-(morpholin-4-yl)-ethyl)-1*H*-pyrazol-4-yl; 1*H*-pyrazol-4-yl; 1-isopropyl-1*H*-pyrazol-4-yl; 1-(methyl-(*d*3))-1*H*-pyrazol-4-yl; 1-methyl-1*H-*pyrazol-3-yl; 1-methyl-1*H*-pyrazol-4-yl; 1-methyl-1*H*-pyrazol-5-yl; 1-(methylsulfonyl)-1*H*-pyrazol-4-yl; 1-(oxetan-3-yl)-1*H*-pyrazol-4-yl; 1-(tetrahydro-2*H-*pyran-4-yl)-1*H*-pyrazol-4-yl; 1-(trifluoromethyl)-1*H*-pyrazol-4-yl; 3,5-dimethyl-1*H*-pyrazol-4-yl;3-methyl-1*H*-pyrazol-4-yl; 1-methyl-3-(trifluoromethyl)-1*H*-pyrazole-4-yl; 1-propyl-1*H-*pyrazole-4-yl; 2-(ethan-1-ol)-1*H*-pyrazol-4-yl; 1-(2-methoxyethyl)-1*H*-pyrazole-4-yl; 4-methyl-1*H*-pyrazol-1-yl; substituted and/or unsubstituted Triazole, in particular 1-methyl-1,2,4-1*H*-triazol-3-yl; 1-methyl-1,2,3-1*H*-triazol-4-yl; substituted and/or unsubstituted Thiazole, in particular 2-Methylthiazol-5-yl; substituted and/or unsubstituted Pyrrolidine, in particular, 3,3-difluoropyrrolidin-1-yl; 3-methoxypyrrolidin-1-yl;
substituted and/or unsubstituted six-membered hetero and/or homocycles, particularly substituted and/or unsubstituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or unsubstituted pyrimidine, in particular, pyrimidin-5-yl; substituted and/or unsubstituted morpholine, in particular, thiomorpholin-1,1-dioxid-4-yl; (2*R*,6*S*)-dimethylmorpholin-4-yl; (2*S*,6*S*)-dimethylmorpholin-4-yl; (3*R*,5*S*)-dimethylmorpholin-4-yl; (3*S*,5*S*)-dimethylmorpholin-4-yl; (*R*)-2-methylmorpholin-4-yl; (*R*)-3-methylmorpholin-4-yl; (*S*)-2-methylmorpholin-4-yl; (*S*)-3-methylmorpholin-4-yl; 2,2-dimethylmorpholin-4-yl; 3,3-dimethylmorpholin-4-yl; morpholin-4-yl; substituted and/or unsubstituted piperidine, in particular 4,4-difluoropiperidin-1-yl;
substituted and/or unsubstituted four-membered hetero and/or homocycles, in particular substituted and/or unsubstituted azetidine, particularly, 3,3-difluoroazetidin-1-yl; 3-dimethylaminoazetidin-1-yl; 3-fluoroazetidin-1-yl; azetidin-1-yl; and
substituted and/or unsubstituted hetero and/or homo bicycles, in particular (1*S*,4*S*)-2-oxa-5-azabicyclo[2.2.1]heptan-5-yl; (1*R*, 4*R*)-2-oxa-5-azabicyclo[2.2.1]heptan-5-yl;3-oxa-8-azabicyclo[3.2.1]octan-8-yl; 8-oxa-3-azabicyclo[3.2.1]octan-3-yl; 2-oxa-6-azaspiro[3.3]heptan-6-yl; 1*H*-pyrrolo[2,3-*b*]pyridin-4-yl; 2-methyl-2*H*-indazol-5-yl; pyrazolo[1,5-*a*]pyridin-3-yl; quinolin-4-yl;
and wherein R⁶ is selected from H, halogen, in particular Br, Cl, F; unsubstituted or substituted C₁₋₃-alkoxy, in particular methoxy, ethoxy, OCF₃, OCHF₂; methylsulfonyl, dimethylphosphine oxide; and unsubstituted or substituted C₁₋₃-alkyl, in particular methyl or CF₃.,
and wherein X³, X⁴ and X⁵ are C, and said method comprises the steps of reacting an aldehyde and an aromatic amine through reductive amination.

In yet another preferred embodiment, said method for producing said compound according to formula (A) comprises a step of deprotection of a tosyl group.

In yet another preferred embodiment, said method for producing said compound according to formula (A) comprises the steps depicted in the following reaction scheme M1:

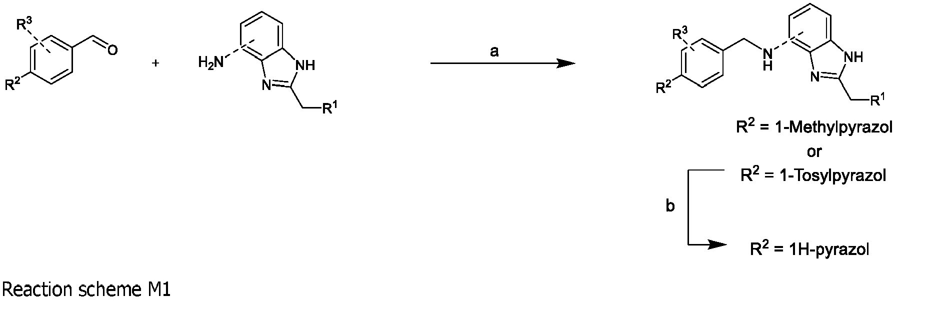

Wherein the reagents and conditions are NaBH₃CN, Acetic Acid (as catalyst), MeOH, 25°C in the reductive amination step a, and Cs₂CO₃, MeOH, at 50°C in the deprotection step.

In a preferred embodiment of the method for producing a compound according to the present invention, said compound is of the formula (B), 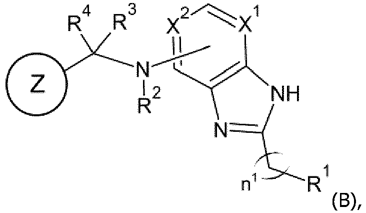 wherein Z is as defined according to the present invention,
said method comprising the steps of
bi) reacting a substituted aromatic nitro-substituted amid-substituted chloroderivative and a substituted aliphatic amine in a nucleophilic aromatic substitution forming an amide-substituted and nitro-substituted aromatic intermediate, and
bii) forming a imidazopyridine by cyclisation reaction of said amide-substituted and nitro-substituted aromatic intermediate from the previous step.

In an exemplary embodiment, said method for producing said compound according to formula (B) comprises the steps depicted in the following reaction scheme M2: 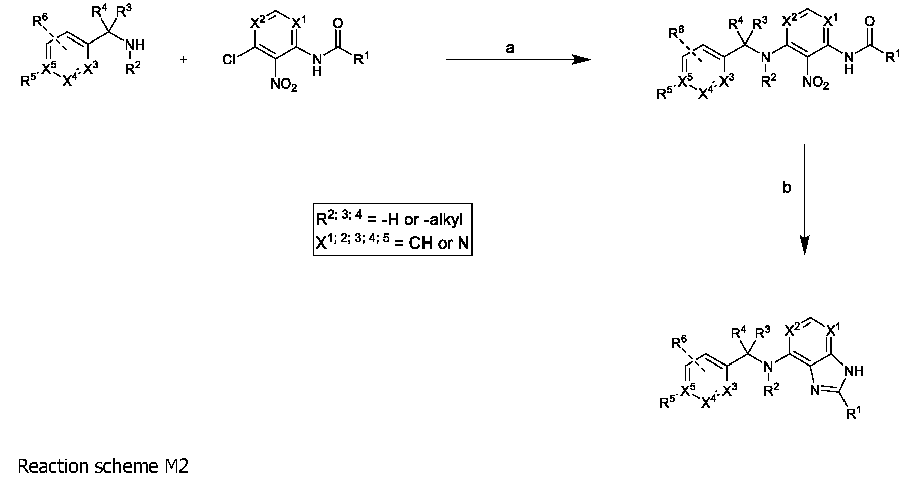 Wherein the reagents and conditions are DIPEA, DMSO at 25°C in reaction step a, and Fe, Acetic Acid, H2O, at 85°C in reaction step b.

In a preferred embodiment of the method for producing a compound according to the present invention, said compound is of the formula (C), 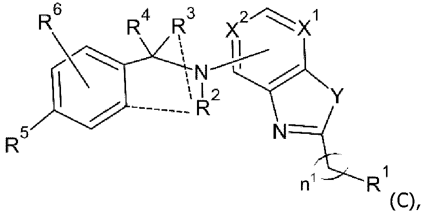 wherein isotopically labeled forms, and combinations thereof; wherein:
X¹ and X² are each independently selected from C or N;
Y is selected from the group consisting of NH, N-alkyl, O, S;
n¹ is any natural number selected from 0, 1, 2, 3;
R¹ is selected from the group consisting of substituted or non-substituted homocyclic ring, substituted or non-substituted heterocyclic ring, alkyl, and alkoxy;
R² is selected from H, alkyl and alkylene group that is also covalently bonded to the aromatic ring, R³ or R⁴ as shown by the dashed line;
R³ is selected from H, alkyl;
R⁴ is selected from H, alkyl,
R⁵ a substituted and/or unsubstituted hetero and/or homocycle selected from
   (i) substituted and/or unsubstituted five-membered hetero and/or homocycles, in particular substituted and/or unsubstituted pyrrole, particularly, 1-methyl-1*H*-pyrrole-3-yl;substituted and/or unsubstituted Oxazole, in particular 3,5-dimethylisoxazol-4-yl; substituted and/or unsubstituted imidazole, in particular 1-methyl-1*H*-imidazol-4-yl; substituted and/or unsubstituted pyrazole, in particular 1-cyclopropyl-1*H-*pyrazol-4-yl; 1,3-dimethy-1*H*-pyrazol-4-yl; 1,5-dimethyl-1*H*-pyrazole-4-yl; 1,3,5-trimethyl-1*H*-pyrazol-4-yl; 1-difluormethyl-1*H*-pyrazol-4-yl; 1-ethyl-1*H*-pyrazol-4-yl; 1-(2-(morpholin-4-yl)-ethyl)-1*H*-pyrazol-4-yl; 1*H*-pyrazol-4-yl; 1-isopropyl-1*H*-pyrazol-4-yl; 1-(methyl-(*d*₃))-1*H*-pyrazol-4-yl; 1-methyl-1*H*-yrazol-3-yl; 1-methyl-1*H*-pyrazol-4-yl; 1-methyl-1*H*-pyrazol-5-yl; 1-(methylsulfonyl)-1*H*-pyrazol-4-yl; 1-(oxetan-3-yl)-1*H*-pyrazol-4-yl; 1-(tetrahydro-2*H*-pyran-4-yl)-1*H*-pyrazol-4-yl; 1-(trifluoromethyl)-1*H*-pyrazol-4-yl; 3,5-dimethyl-1*H*-pyrazol-4-yl; 3-methyl-1*H*-pyrazol-4-yl; 1-methyl-3-(trifluoromethyl)-1*H*-pyrazole-4-yl; 1-propyl-1*H*-pyrazole-4-yl; 2-(ethan-1-ol)-1*H*-pyrazol-4-yl; 1-(2-methoxyethyl)-1*H*-pyrazole-4-yl; substituted and/or unsubstituted Triazole, in particular 1-methyl-1,2,4-1*H*-triazol-3-yl; 1-methyl-1,2,3-1*H*-triazol-4-yl; substituted and/or unsubstituted Thiazole, in particular 2-Methylthiazol-5-yl;
   (ii) substituted and/or unsubstituted six-membered hetero and/or homocycles, particularly substituted and/or unsubstituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or unsubstituted pyrimidine, in particular, pyrimidin-5-yl;
   (iii) substituted and/or unsubstituted hetero and/or homo bicycles, in particular 1*H*-pyrrolo[2,3-*b*]pyridin-4-yl; 2-methyl-2*H*-indazol-5-yl; pyrazolo[1,5*-a*]pyridin-3-yl; quinolin-4-yl; and wherein R⁶ is selected from H, halogen, in particular Br, Cl, F; unsubstituted or substituted C₁₋₃-alkoxy, in particular methoxy, ethoxy, OCF₃, OCHF₂; methylsulfonyl, dimethylphosphine oxide; and unsubstituted or substituted C₁₋₃-alkyl, in particular methyl or CF₃,
said method comprising the step of reacting a substituted aryl halide and a boronic acid pinacol ester substituted aromatic in a Suzuki reaction.

In an exemplary embodiment, said method for producing said compound according to formula (B) comprises the steps depicted in the following reaction scheme M3: 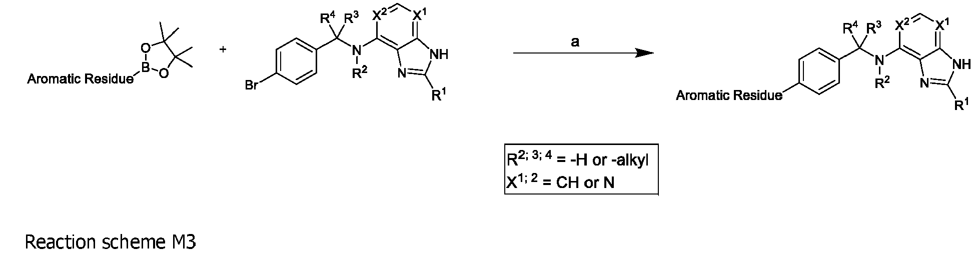 Wherein the reagents and conditions are XPhos Pd G₄, K₂CO₃ , Dioxan, H₂O, 80 - 90°C at 25°C in the reaction step a.

As used herein, the term "AURKA Ligand" also refer to the compounds according to the invention. The terms "MLN8237" or "AURKA inhibitor" refer to compounds as disclosed in WO2022/096679.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, *e.g.* "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

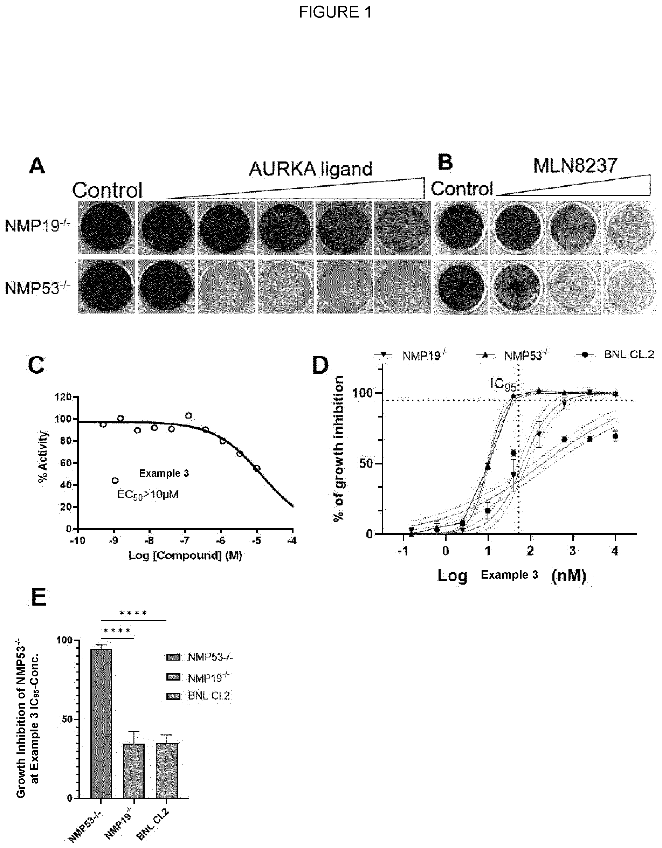

**Figure 1****:** shows (A) Crystal violet colony formation assay of NMP19-/- or NMP53 murine hepatocellular carcinoma cells. In brief, cells were seeded in 6-well plates on day 1. On day 2, cells were treated with either EXAMPLE 3 (AURKA Ligand) or with DMSO (control). After 5 days, wells were washed and cells were fixed with 4% PFA and then stained with Crystal Violet solution. Plates were scanned and pictures were saved. (B) Crystal violet colony formation assay of NMP19-/- or NMP53 murine hepatocellular carcinoma cells. In brief, cells were seeded in 6-well plates on day 1. On day 2, cells were treated with MLN8237 (AURKA inhibitor) or with DMSO (control). After 5 days, wells were washed and cells were fixed with 4% PFA and then stained with Crystal Violet solution. Plates were scanned and pictures were saved. (C) AURKA kinase assay with the AURKA ligand EXAMPLE 3. The AURKA kinase assay was performed by Reaction Biology Corp. and it is based on radiometric ATP quantification. Compounds were received as 10 mM stock. Compounds were tested in 10-dose IC50 mode with a 3-fold serial dilution starting at 10 µM. Control compound, Staurosporine, was tested in 10-dose IC50 mode with 4-fold serial dilution starting at 20 µM. Reactions were carried out at 10 µM ATP. (D) Growth curves of NMP19^{-/-}, NMP53^{-/-} and BNL CL.2 (non-transformed murine embryonic liver cell line). Cells were seeded in 96-well plates on day 1. On next day, they were treated with corresponding EXAMPLE 3 concentrations and left to grow for 48h. Afterwards, an XTT cell viability assay was performed, based on the quantification of metabolic activity of alive cells. Values were fitted in a response curve after normalization to DMSO treated cells and logarithmic transformation. (E) Bar graph corresponds to the growth inhibition in percentage of the IC95 concentration of EXAMPLE 3 against NMP53 cells. At the IC₉₅ of NMP53^{-/-} (HCC cells), no significant growth inhibition is seen on non-sensitive HCC NMP19^{-/-} and on non-transformed BNL C!.2 cells.

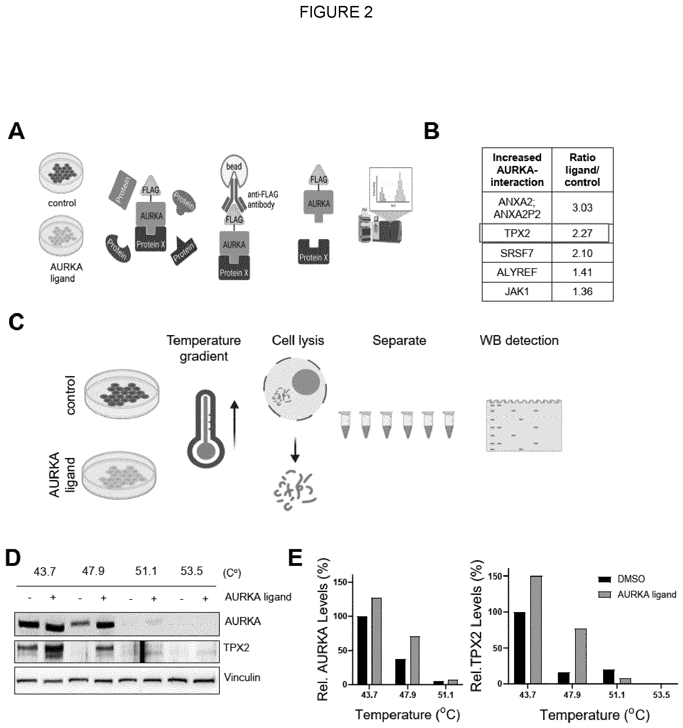

**Figure 2****:** shows (A) Schematic representation of Co-Immunoprecipitation assay. NMP53-/-cells were genetically engineered to express FLAG-AURKA induced by Doxycycline. Cells were either treated with DMSO (control) or AURKA Ligand (AURKA INHIBITOR). Antibodies against FLAG were used to precipitate AURKA together with proteins bound to AURKA. AURKA-binding partners that were enriched in the eluate, were sent for LC-MS analysis. (B) Binding partners with increased binding to FLAG-AURKA under AURKA INHIBITOR treatment were identified. From those, TPX2 was the only protein partner of AURKA that was already described. (C) Schematic representation of a Cellular Thermal Shift Assay (CETSA) used to confirm indirectly confirm stabilization of AURKA/TPX2 complex induced by AURKA ligand treatment. Cells were either treated with EXAMPLE 3 or DMSO (control). A heat gradient was applied to cells followed by lysis to isolate the soluble protein fraction. An SDS-Page was then run to identify the levels of non-melted proteins of interest under the treatment of AURKA ligand/DMSO (AURKA Ligand/Control). (D) SDS-Page results of NMP53^{-/-} cells treated with EXAMPLE 3 and subsequently heated to the temperatures stated. Both AURKA and TPX2 are stabilized at 43.7- 47.9 °C. (E) Quantification of the bands shown on D. using the densitometer of Image Lab software.

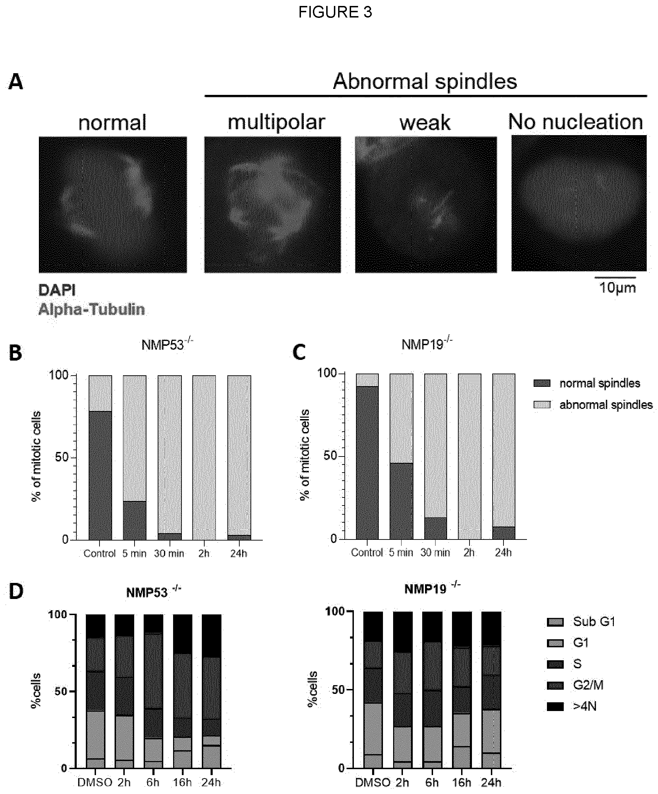

**Figure 3****:** shows (A) Representative pictures of alpha Tubulin staining (red) with DAPI (DNA, blue). Mitotic cells shown, are categorized to normal (bipolar) and abnormal (multipolar, weak, without nucleation). In brief, cells were grown in 4-chamber coverslips under treatment of EXAMPLE 3 or DMSO (control). After certain time points, cells were fixed, permeabilized and stained with antibody against alpha Tubulin. Afterwards a secondary antibody was added, against the primary together with DAPI. Finally, slides were mounted and analyzed in a fluorescent microscope. Bar graphs represent quantification of abnormal/normal spindles levels of (B) NMP53^{-/-} and (C) NMP19^{-/-} mitotic cells at time points after treatment with 15 nM of EXAMPLE 3. Pictures taken in an immunofluorescence microscope (as seen on A.) were used for the quantification. (D) Cell cycle analysis of NMP53 ^{-/-} and NMP19 ^{-/-} cells. Cells were isolated after treatment with EXAMPLE 3, and then stained with Propidium Iodine. Subsequently, they were analyzed in a Flow Cytometry device for quantification of the amount of PI in each cell. Cells were then quantified according to PI amount, which corresponds to certain cell cycle states.

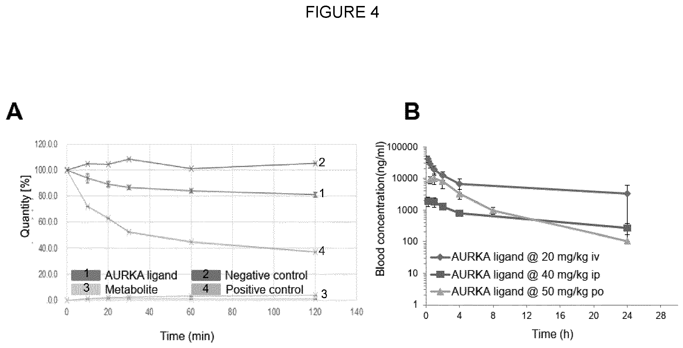

**Figure 4****:** shows (A) Metabolic stability of EXAMPLE 3 tested with murine liver microsomes (N=3). (B) Pharmacokinetic profile of EXAMPLE 3 after administration of the corresponding doses into wild type mice (N=3). Concentration of EXAMPLE 3 was measured and plotted over time.

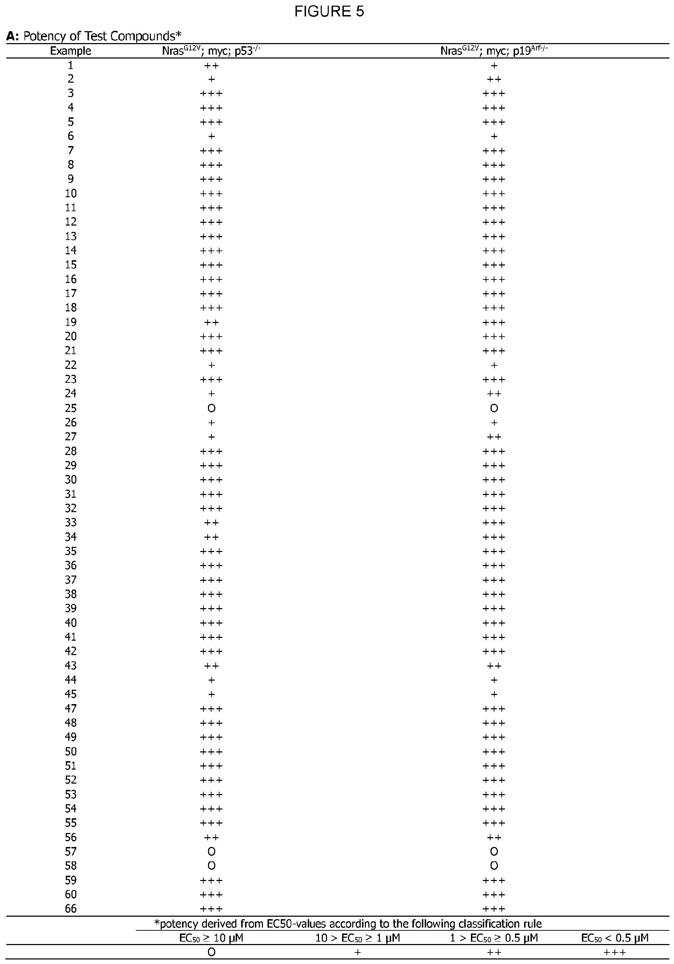

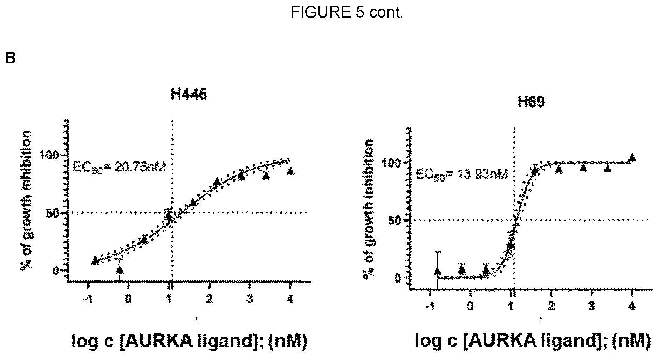

**Figure 5****:** shows (A) EC50 values cell proliferation data using XTT cell proliferation assay of selected compounds for different HCC-tumor-cell-lines; (B) shows an exemplary data set of an XTT-cell proliferation assay for the determination of EC50 values for cell proliferation of a selected compound for different SCLC-tumor-cell-lines.

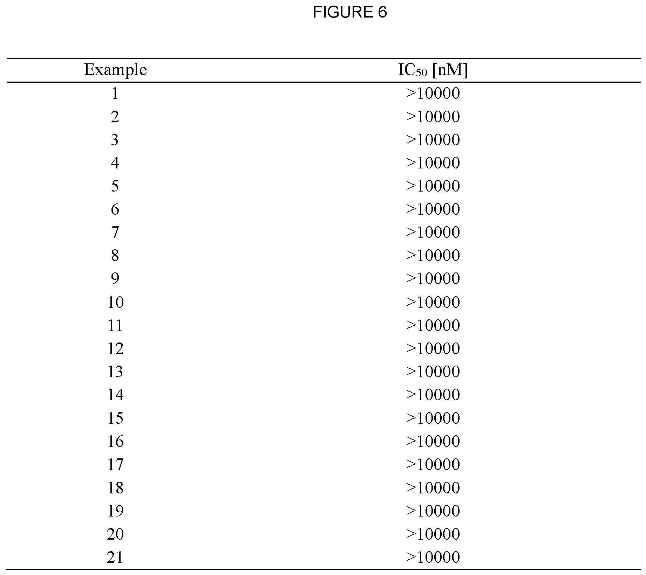

**Figure 6****:** shows AURKA-inhibition data of selected compounds. The AURKA-inhibition assay was performed by Reaction Biology Corp. and it is based on radiometric ATP quantification. Compounds were received as 10 mM stock. Compounds were tested in 10-dose IC50 mode with a 3-fold serial dilution starting at 10 µM. Control compound, Staurosporine, was tested in 10-dose IC50 mode with 4-fold serial dilution starting at 20 µM. Reactions were carried out at 10 µM ATP.
**Figure 7****:** shows (A) Metabolic stability of selected compounds in Mouse Liver Microsomes (MLM). Metabolic stability assays were performed in the presence of an NADPH-regenerating system consisting of 5 mM glucose-6-phosphate, 5 U/mL glucose-6 -phosphate dehydrogenase, and 1 mM NADP⁺. Liver microsomes (20 mg/mL), NADPH-regenerating system, and 4 mM MgCl₂·6 H₂O in 0.1 M TRIS-HCl-buffer (pH 7.4) were preincubated for 5 min at 37 °C and 750 rpm on a shaker. The reaction was started by adding the preheated compound at 10 mM resulting in a final concentration of 0.1 mM. The reaction was quenched at selected time points (0, 10, 20, 30, 60, and 120 min) by pipetting 100 µL of internal standard (ketoprofen) in acetonitrile. The samples were vortexed for 30 s and centrifuged. The supernatant was used directly for LC-MS analysis. All compound incubations were conducted at least in triplicates. Additionally, a negative control containing BSA (20 mg/mL) instead of liver microsomes and a positive control using Verapamil instead of compound were performed. Shown is the remaining unmetabolized percentage of compound in relation to the original loading quantity (equal to 100%). A value of 80% therefore means that 80% of the compound quantity could still be detected unmetabolized after timepoint x. If the m/z ratios of possible metabolites could be detected, their percentages were indicated as M1 for metabolite 1; M2 for metabolite 2 etc.; (B) shows metabolic stability of selected compounds in Human Liver Microsomes (HLM) and a comparative experiment with anilide-based compounds of WO 2022/096679.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:
**Example 1:** Due to the growth inhibition values it is clear that the compounds of the invention inhibit p53-altered carcinoma with high selectivity over other liver cell lines. Besides this the selectivity of the AURKA-Ligands of the invention compared with the AURKA-Inhibitor Alisertib (MLN8237) in p53 altered HCC is also remarkably higher without inhibition the enzymatic function of AURKA (Figure 1).
Based on the western blot data it is clearly visible that there is a ligand-dependent stabilization of AURKA and TPX2 (Figure 2).
Based on the data from Figure 3, it is clear that the ligands of the invention have a strong effect on the spindle assembly even at low concentrations and modify it sustainably over 24 h in p53-deficient cells in favor of abnormal spindles. The return to physiological states is achieved more quickly in p53-functional cells (Figure 3).
The data from Figure 4 show both the high metabolic stability of the molecules of this invention in murine liver microsomes and the very good pharmacokinetic properties of the novel molecules of this invention, which have plasma concentrations after i.v., i.p. and peroral application that correspond to a multiple of the EC50 concentration over a period of 24 h.

Selected compounds 1- 66 inhibit cell proliferation of different tumor cell lines with various efficacy (Figure 5). Growth inhibition curve with increasing concentration can also be shown.

**Example 2:** The compounds according to the invention do not inhibit the kinase activity of Aurora Kinase A, as seen by a IC₅₀ value of at least more than 10000 nM (Figure 6).

**Example 3:** The compounds according to the invention are metabolically stable in MLM and HLM (Figure 7).

**Example 4:** Synthesis of example compounds according to the invention with reductive amination and protection 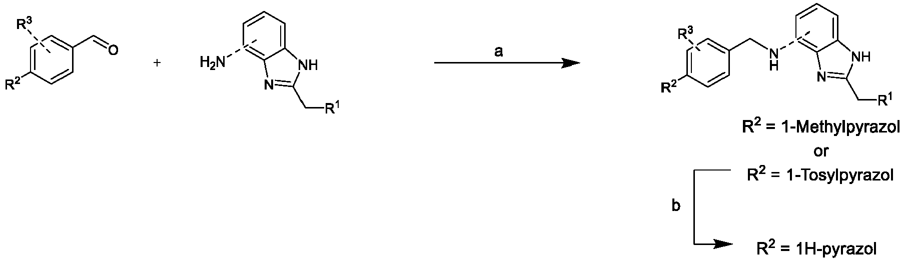 ^{a}Reagents and conditions: (a) NaBH₃CN, Acetic Acid (kat.), MeOH, 25°C; (b) Cs₂CO₃, MeOH, 50°C.

### Reductive amination (a):

To a mixture of the substituted benzaldehyde (100 - 150 mg) and the aromatic amine-component (1.03 eq.) in 5 ml methanol were added 5-10 drops of acetic acid. The mixture was stirred at room temperature for 1 h and afterwards cooled to -10°C. To the cooled solution Sodium Cyanoborohydride (1.50 eq.) was added and the mixture was stirred again at room temperature for 1.5 h until complete consumption of the starting materials. The mixture was poured into ice water, the precipitate was collected by filtration. After purification via column-chromatography (DCM : MeOH (1-10% MeOH)) the desired product was obtained as a white solid.

### Deprotection of Tosyl-Pyrazoles (b):

To a solution of the tosyl-protected-pyrazole-Derivatives in 12 ml methanol was added caesiumcarbonate (3.00 eq.). The mixture was heated at 50°C for 30 min until complete consumption of the starting material. The mixture was diluted with water and the product was extracted with ethyl acetate, washed with brine. The organic phase was dried over Na₂SO₄, filtered, and evaporated to dryness. The product was obtained as a white solid and used without further purification.

### Shown as an example for Synthesis in Synthetic scheme A

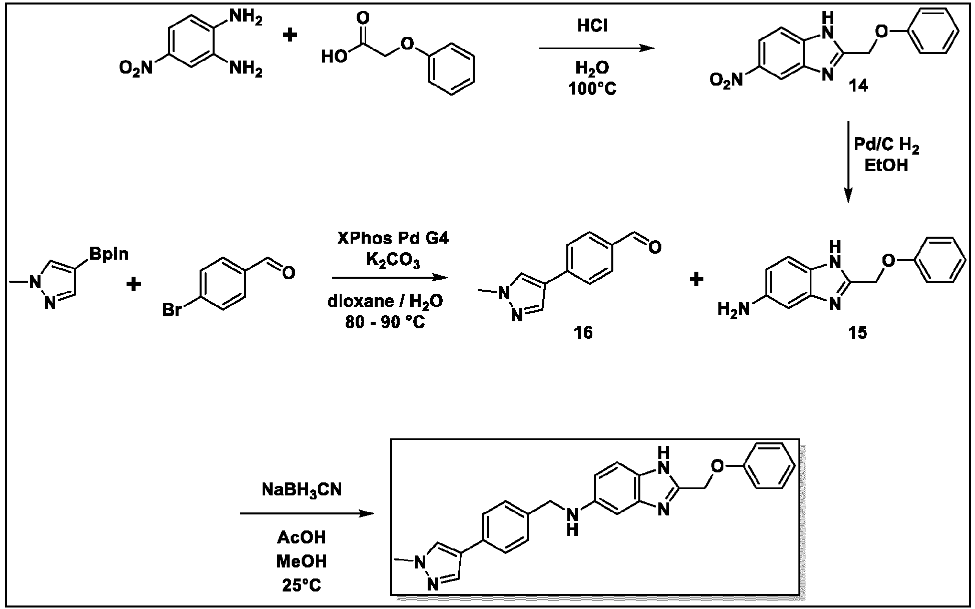

### 5-nitro-2-(phenoxymethyl)-1H-benzo[d]imidazole [14]

The mixture of 4-nitrophenylendiamine (1.00 eq.) and phenoxyacetic acid (1.00 eq.) in aqueous HCl (6N) (0.5 ml/mmol) was stirred under nitrogen-atmosphere at 100°C overnight until complete consumption of the starting materials was observed. The mixture was cooled to room temperature and the product was extracted with ethyl acetate. The organic phase was dried over Na₂SO₄, filtered, and evaporated to dryness. The product was obtained as an off-white solid and used without further purification.

¹H NMR (400 MHz, DMSO) δ 13.37 (s, 1H), 8.47 (d, *J* = 1.8 Hz, 1H), 8.13 (dd, *J* = 8.9, 2.2 Hz, 1H), 7.74 (d, *J* = 8.9 Hz, 1H), 7.33 (t, *J* = 7.9 Hz, 2H), 7.10 (d, *J* = 8.1 Hz, 2H), 6.99 (t, *J* = 7.3 Hz, 1H), 5.42 (s, 2H). TLC-MS (ESI) *m*/*z*: 269.9 [M+H]⁺.

### 2-(phenoxymethyl)-1H-benzo[d]imidazol-5-amine [15]

Pd/C (0.10 eq; 10%) was added to a solution of *14* in ethanol (10.00 ml/mmol). The reaction flask was then first flooded with nitrogen, followed by bubbling the solution with hydrogen for five minutes. The mixture was stirred under a hydrogen atmosphere for 16 h at room temperature until the reaction control showed complete conversion of the starting material. The hydrogen was then removed, Pd/C separated over diatomaceous earth and rinsed several times with methanol. The organic phase was then removed under reduced pressure and the resulting solid was used without further purification.

MS (ESI) *m*/*z*: 240.0 [M+H]⁺; 261.9 [M+Na]⁺.

### tert-butyl (4-(1-methyl-1H-pyrazol-4-yl)benzyl)carbamate [16]

The mixture of 4-brombenzaldehyde (1.00 eq.), 1-methyl-4-(4,4,5,5-tetramethyl-1,3,2-dioxaborolan-2-yl)-1*H* pyrazole (1.10 eq.), K₂CO₃ (3.00 eq.) and XPhos Pd G4 (0.005 eq.) in *1,4*-Dioxane / water (4+1; 2.85 ml/mmol of Dioxane) was stirred under nitrogen-atmosphere at 80 - 90°C overnight until complete consumption of the starting materials was observed. The mixture was cooled to room temperature, diluted dropwise with MeOH / H₂O (40/60) under constant stirring and cooled in an ice bath until complete precipitation of the product was observed. The precipitate was collected by filtration, washed with MeOH / H₂O (40/60) and dried. The product was obtained as a white solid and used without further purification.

¹H NMR (400 MHz, DMSO) δ 9.95 (s, 1H), 8.32 (s, 1H), 8.02 (s, 1H), 7.88 (d, *J* = 8.3 Hz, 2H), 7.79 (d, *J* = 8.2 Hz, 2H), 3.89 (s, 3H). TLC-MS (ESI) *m*/*z*: n.d.. HPLC *t_{R}* = 4.38 min.

### Example 2:

### N-(4-(1-methyl-1H-pyrazol-4-yl)benzyl)-2-(phenoxymethyl)-1H-benzo[d]imidazol-5-amine

To a mixture of *16*(1.00 eq.) and *15*(1.03 eq.) in 5 ml methanol were added 5-10 drops of acetic acid. The mixture was stirred at room temperature for 1 h and afterwards cooled to -10°C. To the cooled solution Sodium Cyanoborohydride (1.50 eq.) was added and the mixture was stirred again at room temperature for 1.5 h until complete consumption of the starting materials. The mixture was poured into ice water, the precipitate was collected by filtration. After purification via column-chromatography (DCM : MeOH (1-10% MeOH)) the desired product was obtained as a white solid.

¹H NMR (400 MHz, DMSO) δ 12.09 (s, 1H), 8.06 (s, 1H), 7.81 (s, 1H), 7.49 (d, *J* = 8.2 Hz, 2H), 7.35 (d, *J* = 8.2 Hz, 2H), 7.29 (ddd, *J* = 9.9, 4.8, 2.3 Hz, 3H), 7.05 (dd, *J* = 8.7, 0.9 Hz, 2H), 6.97 - 6.91 (m, 1H), 6.64 (dd, *J* = 8.7, 1.5 Hz, 1H), 6.51 (s, 1H), 6.10 (s, 1H), 5.16 (s, 2H), 4.27 (s, 2H), 3.84 (s, 3H). TLC-MS (ESI) *m*/*z*: 410.1 [M+H]⁺; 432.1 [M+Na]⁺. HPLC *t_{R}* = 5.56 min. FT-IR(ATR) [cm⁻¹]: 3050, 3029, 2933, 1631, 1595, 1586, 1490, 1442, 1363, 1292, 1232, 1209, 1172, 1031, 986, 954, 801, 751, 689.

The following examples were prepared using the procedures above:

### Example 1

### N-(4-(1H-pyrazol-4-yl)benzyl)-2-(phenoxymethyl)-1H-benzo[d]imidazol-5-amine

¹H NMR (400 MHz, DMSO) δ 12.89 (s, 1H), 12.06 (s, 1H), 8.12 (s, 1H), 7.88 (s, 1H), 7.54 (d, *J* = 8.2 Hz, 2H), 7.35 (d, *J* = 8.1 Hz, 2H), 7.32 - 7.25 (m, 3H), 7.05 (dd, *J* = 8.7, 0.8 Hz, 2H), 6.94 (t, *J* = 7.3 Hz, 1H), 6.63 (d, *J* = 8.2 Hz, 1H), 6.48 (s, 1H), 6.13 (s, 1H), 5.16 (s, 2H), 4.27 (d, *J* = 4.3 Hz, 2H). TLC-MS (ESI) *m*/*z*: 396.5 [M+H]⁺; 418.5 [M+Na]⁺. HPLC *t_{R}* = 4.50 min. FT-IR(ATR) [cm⁻¹]: 3401, 3139, 3028, 2938, 2843, 1630, 1597, 1587, 1490, 1420, 1371, 1228, 1172, 1032, 950, 806, 751, 689.

**Example 5:** Synthesis of example compounds according to the invention with nucleophilic aromatic substation and Imidazopyridine-formation. 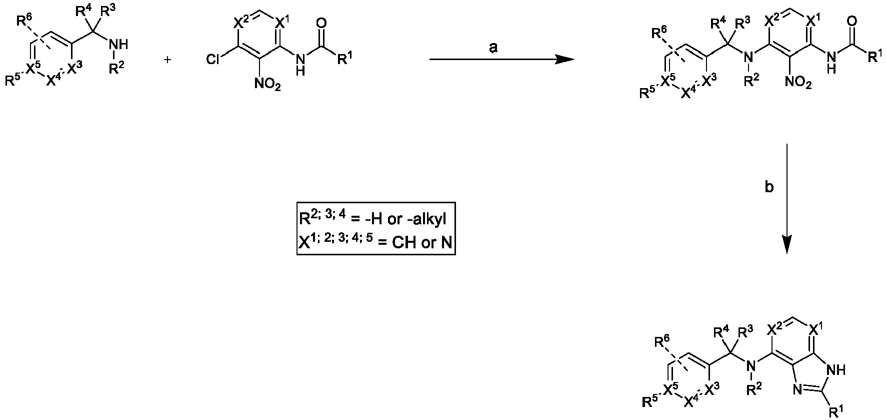 ^{a}Reagents and conditions: (a) DIPEA, DMSO, 25°C; (b) Fe, Acetic Acid, H₂O, 85°C.

### S_{N}Ar (a):

To a mixture of the substituted aromatic nitro-substituted chloroderivative (150 mg) and the substituted aliphatic amine (1.20 eq.) in 5 ml dimethyl sulfoxide was added DIPEA (2.00 eq.). The mixture was stirred at room temperature overnight until complete consumption of the starting materials. The mixture was diluted with methanol and water, the methanol was evaporated under reduced pressure and the precipitate was collected by filtration. The product was obtained as a yellow solid and used without further purification.

### Cyclisation (b):

To a solution of the substituted product from reaction (a) (100 - 200 mg) in a mixture of acetic acid (5 ml) and water (2 ml) was added powdered iron (3.00 eq.). The mixture was stirred at 85°C for 2 h until complete consumption of the starting material. The mixture was cooled to room temperature, diluted with water and NaOH-solution and filtered. The product was extracted from the clear mixture with ethyl acetate and washed with brine. The organic phase was dried over Na₂SO₄, filtered, and evaporated to dryness. After purification via column-chromatography (from 100% DCM to DCM/MeOH 9:1) the desired product was obtained as a white solid.

### Shown as an example for Synthesis in Synthetic scheme B

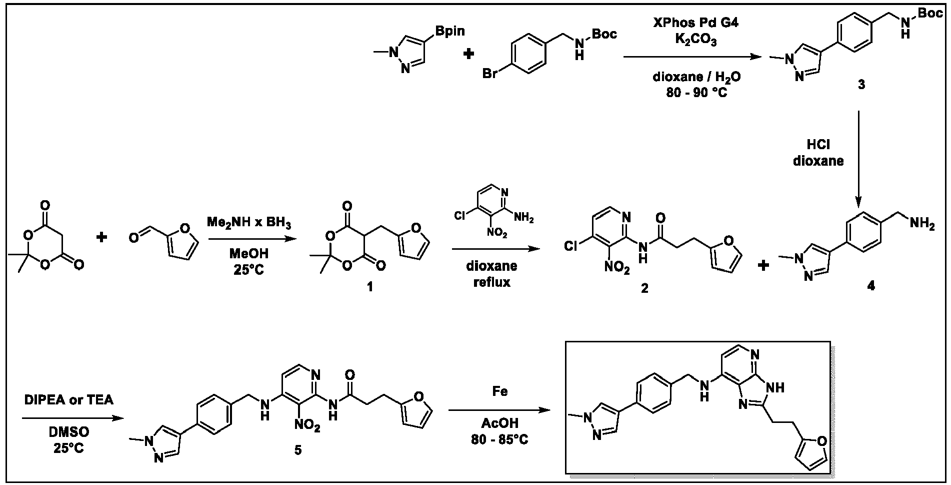

### 5-(furan-2-ylmethyl)-2,2-dimethyl-1,3-dioxane-4,6-dione [1]

To a mixture of Dimethyl borane (1.03 eq.) in methanol (1.5 ml/mmol) was added Meldrum's acid (1.00 eq.). After complete dissolution of the solids, furfural (1.20 eq.) was added dropwise over 30 minutes at 0°C - 5°C. The mixture was stirred for 30 - 60 minutes at room temperature until complete consumption of the starting materials was observed. The mixture was diluted with ice water (5 ml/mmol), acidified with HCl and the precipitate was collected by filtration. The raw product was dissolved in Ethyl acetate and washed with diluted HCl (pH = 2) to remove Dimethylamine-HCl. The organic phase was dried over Na₂SO₄, filtered, and evaporated to dryness. The product was obtained as an off-white solid and used without further purification.

¹H NMR (400 MHz, DMSO) δ 7.48 (d, *J* = 1.1 Hz, 1H), 6.34 (dd, *J* = 3.1, 1.9 Hz, 1H), 6.09 (d, *J* = 2.7 Hz, 1H), 4.82 (t, *J* = 4.8 Hz, 1H), 3.33 (d, *J* = 4.7 Hz, 2H), 1.82 (s, 3H), 1.65 (s, 3H). TLC-MS (ESI) *m*/*z*: n.d.. HPLC *t_{R}* = 3.87 min. FT-IR(ATR) [cm⁻¹]: 3110, 3006, 2947, 2879, 1781, 1739, 1595, 1512, 1443, 1393, 1380, 1356, 1319, 1290, 1256, 1256, 1237, 1196, 1165, 1139, 1104, 1069, 1032, 1019, 998, 957, 943, 906, 881, 820, 804, 740, 676.

### N-(4-chloro-3-nitropyridin-2-yl)-3-(furan-2-yl)propanamide [2]

1 (1.20 eq.) and 4-Chloro-3-nitropyridin-2-amine (1.00 eq.) were dissolved in *1,4*-dioxane (1.2 ml/mmol). The mixture was stirred at reflux conditions for 2 h until complete consumption of the starting materials was observed. The mixture was diluted with water and a small amount of methanol, the organic solvents were evaporated under reduced pressure and the precipitate was collected by filtration. The product was obtained as an off-white solid and used without further purification.

¹H NMR (400 MHz, DMSO) δ 11.23 (s, 1H), 8.60 (d, *J* = 5.3 Hz, 1H), 7.71 (d, *J* = 5.3 Hz, 1H), 7.51 (d, *J* = 0.5 Hz, 1H), 6.37 - 6.33 (m, 1H), 6.10 (d, *J* = 2.7 Hz, 1H), 2.87 (t, *J* = 7.3 Hz, 2H), 2.72 (t, *J* = 7.4 Hz, 2H). TLC-MS (ESI) *m*/*z:* 318.3 [M+Na]⁺. HPLC *t_{R}* = 6.40 min. FT-IR(ATR) [cm⁻¹]: 3277, 1675, 1579, 1553, 1534, 1503, 1403, 1358, 1214, 1173, 1144, 1070, 1013, 942, 922, 854, 837, 746, 732.

### tert-butyl (4-(1-methyl-1H-pyrazol-4-yl)benzyl)carbamate [3]

The mixture of tert-butyl (4-bromobenzyl)carbamate (1.00 eq.), 1-methyl-4-(4,4,5,5-tetramethyl-1,3,2-dioxaborolan-2-yl)-1*H*-pyrazole (1.10 eq.), K₂CO₃ (3.00 eq.) and XPhos Pd G4 (0.005 eq.) in *1,4*-Dioxane / water (4+1; 2.85 ml/mmol of Dioxane) was stirred under nitrogen-atmosphere at 80 - 90°C overnight until complete consumption of the starting materials was observed. After 500 ml of dioxane were removed under reduced pressure, the mixture was cooled to room temperature, diluted dropwise with MeOH / H₂O (40/60) under constant stirring and cooled in an ice bath until complete precipitation of the product was observed. The precipitate was collected by filtration, washed with MeOH / H₂O (40/60) and dried. The product was obtained as a white solid and used without further purification.

¹H NMR (400 MHz, DMSO) δ 8.08 (s, 1H), 7.82 (s, 1H), 7.49 (d, *J* = 8.0 Hz, 2H), 7.35 (t, *J* = 5.8 Hz, 1H), 7.21 (d, *J* = 8.0 Hz, 2H), 4.10 (d, *J* = 6.0 Hz, 2H), 3.85 (s, 3H), 1.40 (s, 9H). TLC-MS (ESI) *m*/*z*: 310.2 [M+Na]⁺. HPLC *t_{R}* = 6.72 min. FT-IR(ATR) [cm⁻¹]: 3228, 3024, 2974, 2925, 2640, 1718, 1629, 1582, 1550, 1522, 1485, 1436, 1420, 1405, 1377, 1332, 1278, 1259, 1249, 1144, 1131, 1069, 1010, 891, 791, 730.

### (4-(1-methyl-1H-pyrazol-4-yl)phenyl)methanamine [4]

To a solution of *3* (1.00 eq.) in *1,4*-dioxane (0.75 ml/mmol) was added 4N HCl in *1,4*-Dioxane (5.00 eq.). The mixture was stirred at room temperature for 2 h until complete consumption of the starting material. Afterwards aceton (2 ml/mmol) was added and the precipitate was collected by filtration. The product was obtained as a white solid (HCl-salt) and used without further purification.

### Generation of free base:

To a stirred solution of *4* as dihydrochloride in water (1.6 ml/mmol) was slowly added a solution of NaOH (2.10 eq.) in water. After complete addition, stirring was continued for another 30 min, the resulting solid was collected by filtration, and the filter cake was washed several times with water. The product was then dried and obtained as a light gray to white solid and used without further purification.

¹H NMR (400 MHz, DMSO) δ 8.07 (s, 1H), 7.81 (d, *J* = 0.7 Hz, 1H), 7.50 - 7.45 (m, 2H), 7.29 (d, *J* = 8.3 Hz, 2H), 3.85 (s, 3H), 3.69 (s, 2H), 2.10 (s, 2H). TLC-MS (ESI) *m*/*z*: n.d.. HPLC *t_{R}* = 1.34 min. FT-IR(ATR) [cm⁻¹]: 3318, 3109, 2909, 2849, 1612, 1564, 1473, 1444, 1422, 1404, 1371, 1194, 1185, 1079, 1067, 989, 930, 861, 851, 838, 804, 792.

### 3-(furan-2-yl)-N-(4-((4-(1-methyl-1H-pyrazol-4-yl)benzyl)amino)-3-nitropyridin-2-yl)propanamide [5]

To a mixture of 2 (1.00 eq.) and *4* (1.05 eq.) in dimethyl sulfoxide (6.00 ml/mmol) was added DIPEA (2.00 eq.). The mixture was stirred at room temperature overnight until complete consumption of the starting materials. The mixture was diluted with methanol followed by addition of aqueous methanol (60% v/v) to induce precipitation of the product. At a total volume of 1250 mL, no more precipitation was observed, and the slurry was filtered. The product was dried at room temperature under vacuum to obtain the product as a yellow solid that was used without further purification.

¹H NMR (400 MHz, DMSO) δ 10.69 (s, 1H), 8.09 (d, *J* = 6.3 Hz, 1H), 7.96 (t, *J* = 7.2 Hz, 2H), 7.82 (d, *J* = 5.1 Hz, 1H), 7.52 (d, *J* = 8.2 Hz, 3H), 7.31 (d, *J* = 8.1 Hz, 2H), 6.61 (d, *J* = 6.1 Hz, 1H), 6.34 (dd, *J* = 2.9, 2.0 Hz, 1H), 6.10 (d, *J* = 2.8 Hz, 1H), 4.51 (d, *J* = 5.9 Hz, 2H), 3.84 (s, 3H), 2.84 (t, *J* = 7.5 Hz, 2H), 2.64 (t, *J* = 7.5 Hz, 2H). TLC-MS (ESI) *m*/*z:* 447.2 [M+H]⁺; 469.2 [M+Na]⁺. HPLC *t_{R}* = 6.57 min. FT-IR(ATR) [cm⁻¹]: 3388, 3107, 2937, 1706, 1609, 1565, 1529, 1506, 1496, 1437, 1399, 1380, 1339, 1279, 1261, 1229, 1177, 1143, 1074, 984, 954, 864, 799, 728.

### Example 3

### 2-(2-(furan-2-yl)ethyl)-N-(4-(1-methyl-1H-pyrazol-4-yl)benzyl)-3H-imidazo[4,5-b]pyridin-7-amine

To a solution of *5* (1.00 eq.) in a mixture of acetic acid and water (2.5 : 1; 7 ml/mmol acetic acid) was added powdered iron (3.00 eq.). The mixture was stirred at 85°C for 2 h until complete consumption of the starting material. After complete conversion of the reaction, the batch was cooled down to room temperature, diluted with water and NaOH-solution and filtered. The product was extracted from the clear mixture with ethyl acetate and washed with brine. The organic phase was dried over Na₂SO₄, filtered, and evaporated to dryness. After purification via column-chromatography (from 100% DCM to DCM/MeOH 9:1) the desired product was obtained as a white solid.

¹H NMR (400 MHz, DMSO) δ 12.40 (s, 1H), 8.06 (s, 1H), 7.80 (s, 1H), 7.72 (d, *J* = 5.6 Hz, 1H), 7.52 (d, *J* = 1.0 Hz, 1H), 7.48 (d, *J* = 8.0 Hz, 2H), 7.34 (d, *J* = 8.1 Hz, 2H), 7.15 (t, *J* = 6.0 Hz, 1H), 6.35 - 6.31 (m, 1H), 6.19 (d, *J* = 5.5 Hz, 1H), 6.12 (d, *J* = 2.9 Hz, 1H), 4.53 (d, *J* = 5.9 Hz, 2H), 3.84 (s, 3H), 3.11 (dd, J= 13.9, 5.7 Hz, 4H). TLC-MS (ESI) *m*/*z*: 399.2 [M+H]⁺; 421.2 [M+Na]⁺. HPLC *t_{R}* = 4.49 min. FT-IR(ATR) [cm⁻¹]: 3227, 3103, 3026, 2935, 2642, 1627, 1585, 1545, 1522, 1506, 1437, 1374, 1363, 1335, 1292, 1216, 1198, 1146, 1130, 1072, 982, 954, 891, 793, 733.

### The following examples were prepared using the procedures above:

### Example 4

### 2-(2-cyclopropylethyl)-N-(4-(1-methyl-1H-pyrazol-4-yl)benzyl)-3H-imidazo[4,5-b]pyridin-7-amine

¹H NMR (400 MHz, DMSO) δ 12.30 (s, 1H), 8.05 (s, 1H), 7.79 (s, 1H), 7.71 (d, *J* = 5.5 Hz, 1H), 7.47 (d, *J* = 8.1 Hz, 2H), 7.34 (d, *J* = 8.1 Hz, 2H), 7.11 (t, *J* = 6.0 Hz, 1H), 6.17 (d, *J* = 5.6 Hz, 1H), 4.52 (d, *J* = 6.1 Hz, 2H), 3.84 (s, 3H), 2.83 (t, *J* = 7.6 Hz, 2H), 1.66 (dd, *J* = 15.0, 7.2 Hz, 2H), 0.79 - 0.71 (m, 1H), 0.42 - 0.37 (m, 2H), 0.05 (q, *J* = 5.0 Hz, 2H). TLC-MS (ESI) *m*/*z*: 373.3 [M+H]⁺; 395.2 [M+Na]⁺. HPLC *t_{R}* = 4.73 min. FT-IR(ATR) [cm⁻¹]: 3389, 2992, 2916, 2850, 1599, 1560, 1522, 1438, 1402, 1334, 1275, 1221, 1140, 1010, 989, 952, 800, 728, 659.

### Example 5

### 2-(cyclopropylmethyl)-N-(4-(1-methyl-1H-pyrazol-4-yl)benzyl)-3H-imidazo[4,5-b]pyridin-7-amine

¹H NMR (400 MHz, DMSO) δ 12.32 (s, 1H), 8.06 (s, 1H), 7.80 (s, 1H), 7.73 (s, 1H), 7.48 (d, *J* = 7.5 Hz, 2H), 7.35 (d, *J* = 7.7 Hz, 2H), 7.13 (s, 1H), 6.19 (s, 1H), 4.52 (d, *J* = 4.3 Hz, 2H), 3.84 (s, 3H), 2.67 (d, *J* = 6.5 Hz, 2H), 1.20 - 1.11 (m, 1H), 0.50 (d, *J* = 6.7 Hz, 2H), 0.27 (d, *J* = 3.2 Hz, 2H). TLC-MS (ESI) *m*/*z*: 359.1 [M+H]⁺; 381.0 [M+Na]⁺. HPLC *t_{R}* = 4.17 min. FT-IR(ATR) [cm⁻¹]: 3009, 2942, 2865, 2801, 2751, 2703, 1608, 1566, 1522, 1437, 1391, 1333, 1284, 1000, 954, 828, 795, 659.

### Example 6

### 2-(methoxy(phenyl)methyl)-N-(4-(1-methyl-1H-pyrazol-4-yl)benzyl)-3H-imidazo[4,5-b]pyridin-7-amine

¹H NMR (400 MHz, DMSO) δ 12.75 (s, 1H), 8.05 (s, 1H), 7.78 (d, *J* = 9.8 Hz, 2H), 7.48 (s,4H), 7.33 (dd, *J* = 17.9, 9.5 Hz, 6H), 6.20 (s, 1H), 5.51 (s, 1H), 4.50 (s, 2H), 3.84 (s, 3H), 3.36 (s, 3H). TLC-MS (ESI) *m*/*z*: 425.2 [M+H]⁺; 447.2 [M+Na]⁺. HPLC *t_{R}* = 5.13 min. FT-IR(ATR) [cm⁻¹]: 2935, 2879, 2847, 1618, 1597, 1566, 1522, 1437, 1388, 1335, 1287, 1196, 1148, 1099, 1073, 996, 954, 888, 856, 827, 798, 754, 718, 701, 662.

### Example 7

### 2-(2-cyclobutylethyl)-N-(4-(1-methyl-1H-pyrazol-4-yl)benzyl)-3H-imidazo[4,5-b]pyridin-7-amine

¹H NMR (400 MHz, DMSO) δ 12.28 (s, 1H), 8.06 (s, 1H), 7.80 (s, 1H), 7.72 (s, 1H), 7.48 (d, *J* = 7.2 Hz, 2H), 7.34 (d, *J* = 7.4 Hz, 2H), 7.11 (s, 1H), 6.18 (s, 1H), 4.51 (d, *J* = 3.4 Hz, 2H), 3.84 (s, 3H), 2.65 (t, *J* = 7.0 Hz, 2H), 2.33 - 2.19 (m, 1H), 2.00 (d, *J* = 4.2 Hz, 2H), 1.87 - 1.73 (m, 4H), 1.60 (dd, *J* = 16.8, 8.0 Hz, 2H). TLC-MS (ESI) *m*/*z*: 387.0 [M+H]⁺; 408.9 [M+Na]⁺. HPLC *t_{R}* = 5.88 min. FT-IR(ATR) [cm⁻¹]: 3018, 2923, 2850, 2747, 2707, 2658, 1617, 1565, 1522, 1437, 1400, 1363, 1331, 1278, 1217, 1182, 1145, 985, 954, 886, 824, 794, 722, 660.

### Example 8

### N-(4-(1-methyl-1H-pyrazol-4-yl)benzyl)-2-(2-(thiophen-3-yl)ethyl)-3H-imidazo[4,5-b]pyridin-7-amine

¹H NMR (400 MHz, DMSO) δ 12.38 (s, 1H), 8.06 (s, 1H), 7.80 (s, 1H), 7.72 (s, 1H), 7.46 (dd, *J* = 13.2, 4.8 Hz, 3H), 7.34 (d, *J* = 7.2 Hz, 2H), 7.18 (d, *J* = 24.8 Hz, 2H), 7.00 (d, *J* = 4.1 Hz, 1H), 6.19 (s, 1H), 4.53 (d, *J* = 1.0 Hz, 2H), 3.84 (s, 3H), 3.09 (d, *J* = 5.4 Hz, 4H). TLC-MS (ESI) *m*/*z*: 414.8 [M+H]⁺; 436.7 [M+Na]⁺. HPLC *t_{R}* = 5.11 min. FT-IR(ATR) [cm⁻¹]: 2932, 2854, 2812, 2781, 2731, 2693, 2650, 2623, 2585, 2548, 1605, 1560, 1508, 1437, 1388, 1331, 1280, 1217, 1191, 1141, 984, 954, 892, 858, 825, 797, 775, 745, 661.

### Example 9

### N-(4-(1-methyl-1H-pyrazol-4-yl)benzyl)-2-(2-(5-methylfuran-2-yl)ethyl)-3H-imidazo[4,5-b]pyridin-7-amine

¹H NMR (400 MHz, DMSO) δ 12.38 (s, 1H), 8.06 (s, 1H), 7.80 (s, 1H), 7.72 (d, *J* = 4.8 Hz, 1H), 7.48 (d, *J* = 7.8 Hz, 2H), 7.34 (d, *J* = 8.0 Hz, 2H), 7.15 (s, 1H), 6.19 (d, *J* = 5.0 Hz, 1H), 5.96 (d, *J* = 2.9 Hz, 1H), 5.91 (d, *J* = 1.9 Hz, 1H), 4.53 (d, *J* = 4.7 Hz, 2H), 3.84 (s, 3H), 3.06 (m, 4H), 2.20 (s, 3H). TLC-MS (ESI) *m*/*z*: 412.9 [M+H]⁺; 434.9 [M+Na]⁺. HPLC *t_{R}* = 5.38 min. FT-IR(ATR) [cm⁻¹]: 3232, 2921, 2842, 2803, 2717, 2658, 2571, 2555, 2529, 2464, 2374, 1629, 1584, 1565, 1522, 1438, 1370, 1334, 1292, 1214, 1190, 1144, 984, 954, 890, 824, 790, 750, 704, 659.

### Example 10

### N-(4-(3-fluoropyridin-4-yl)benzyl)-2-(2-(furan-2-yl)ethyl)-3H-imidazo[4,5-b]pyridin-7-amine

¹H NMR (400 MHz, DMSO) δ 12.28 (s, 1H), 8.63 (d, *J* = 2.5 Hz, 1H), 8.48 (d, *J* = 4.9 Hz, 1H), 7.74 (d, *J* = 4.4 Hz, 1H), 7.61 (dd, *J* = 9.9, 4.6 Hz, 3H), 7.53 (d, *J* = 8.2 Hz, 3H), 7.28 (s, 1H), 6.33 (d, *J* = 1.9 Hz, 1H), 6.20 (d, *J* = 5.1 Hz, 1H), 6.12 (d, *J* = 2.8 Hz, 1H), 4.63 (d, *J* = 5.5 Hz, 2H), 3.17 - 3.04 (m, 4H). TLC-MS (ESI) *m*/*z*: 413.9 [M+H]⁺; 435.8 [M+Na]⁺. HPLC *t_{R}* = 5.59 min. FT-IR(ATR) [cm⁻¹]: 2789, 2753, 2724, 2680, 2666, 2596, 2576, 2552, 2534, 2508, 2491, 1604, 1522, 1481, 1438, 1400, 1363, 1340, 1286, 1262, 1232, 1210, 1147, 1000, 889, 822, 791, 730, 701, 661.

### Example 13

### N-(4-(1-methyl-1H-pyrazol-4-yl)benzyl)-2-phenethyl-3H-imidazo[4,5-b]pyridin-7-amine

¹H NMR (400 MHz, DMSO) δ 12.40 (s, 1H), 8.06 (s, 1H), 7.80 (s, 1H), 7.72 (d, *J* = 3.3 Hz, 1H), 7.48 (d, *J* = 7.9 Hz, 2H), 7.34 (d, *J* = 8.0 Hz, 2H), 7.30 - 7.24 (m, 4H), 7.18 (ddd, *J* = 8.4, 4.0, 1.8 Hz, 2H), 6.19 (d, *J* = 4.2 Hz, 1H), 4.52 (d, *J* = 5.1 Hz, 2H), 3.84 (s, 3H), 3.14 - 3.02 (m, 4H). TLC-MS (ESI) *m*/*z*: 409.5 [M+H]⁺; 431.5 [M+Na]⁺. HPLC *t_{R}* = 5.37 min. FT-IR(ATR) [cm⁻¹]: 2934, 2714, 2638, 2590, 2549, 2524, 2498, 1629, 1585, 1441, 1363, 1332, 1292, 1140, 985, 953, 892, 827, 792, 750, 719, 695, 661.

### Example 15

### N-(4-fluorobenzyl)-2-(2-(furan-2-yl)ethyl)-3H-imidazo[4,5-b]pyridin-7-amine

¹H NMR (400 MHz, DMSO) δ 12.42 (s, 1H), 7.72 (d, *J* = 4.8 Hz, 1H), 7.52 (s, 1H), 7.44 - 7.36 (m, 2H), 7.20 (s, 1H), 7.12 (t, *J* = 8.6 Hz, 2H), 6.33 (s, 1H), 6.17 (d, *J* = 5.0 Hz, 1H), 6.11 (d, *J* = 2.8 Hz, 1H), 4.53 (d, *J* = 5.5 Hz, 2H), 3.10 (dd, *J* = 13.2, 5.6 Hz, 4H). TLC-MS (ESI) *m*/*z*: 337.4 [M+H]⁺; 359.3 [M+Na]⁺. HPLC *t_{R}* = 5.06 min. FT-IR(ATR) [cm⁻¹]: 3411, 2858, 2736, 2691, 1610, 1506, 1442, 1395, 1333, 1276, 1216, 1144, 983, 886, 826, 796, 755.

### Example 16

### N-(4-(1-(difluoromethyl)-1H-pyrazol-4-yl)benzyl)-2-(2-(furan-2-yl)ethyl)-3H-imidazo[4,5-b]pyridin-7-amine

¹H NMR (400 MHz, DMSO) δ 12.42 (s, 1H), 8.66 (s, 1H), 8.23 (s, 1H), 7.89 (d, *J* = 59.3 Hz, 1H), 7.72 (d, *J* = 5.5 Hz, 1H), 7.62 (d, *J* = 8.0 Hz, 2H), 7.52 (d, *J* = 1.0 Hz, 1H), 7.40 (d, *J* = 8.0 Hz, 2H), 7.20 (t, *J* = 6.0 Hz, 1H), 6.33 (dd, *J* = 3.0, 1.9 Hz, 1H), 6.18 (d, *J* = 5.5 Hz, 1H), 6.12 (d, *J* = 2.9 Hz, 1H), 4.56 (d, *J* = 5.6 Hz, 2H), 3.17 - 3.06 (m, 4H). TLC-MS (ESI) *m*/*z*: 435.3 [M+H]⁺; 457.3 [M+Na]⁺. HPLC *t_{R}* = 5.09 min. FT-IR(ATR) [cm⁻¹]: 3339, 3007, 2930, 2850, 2780, 2708, 2659, 1608, 1507, 1411, 1388, 1332, 1283, 1187, 1141, 1107, 1061, 1029, 993, 954, 886, 831, 809, 791, 725, 663.

### Example 17

### 2-(2-(furan-2-yl)ethyl)-N-(4-isopropylbenzyl)-3H-imidazo[4,5-b]pyridin-7-amine

¹H NMR (400 MHz, DMSO) δ 12.40 (s, 1H), 7.73 (s, 1H), 7.52 (d, *J* = 0.9 Hz, 1H), 7.28 (d, *J* = 7.8 Hz, 2H), 7.24 - 7.01 (m, 3H), 6.33 (dd, *J* = 2.9, 1.9 Hz, 1H), 6.19 (d, *J* = 3.8 Hz, 1H), 6.11 (d, *J* = 2.9 Hz, 1H), 4.50 (d, *J* = 4.3 Hz, 2H), 3.18 - 3.04 (m, 4H), 2.83 (dp, *J* = 13.4, 6.7 Hz, 1H), 1.16 (d, *J* = 6.9 Hz, 6H). TLC-MS (ESI) *m*/*z*: 361.2 [M+H]⁺; 383.1 [M+Na]⁺. HPLC *t_{R}* = 6.28 min. FT-IR(ATR) [cm⁻¹]: 3235, 2959, 2926, 2865, 2715, 2655, 1630, 1584, 1550, 1507, 1438, 1339, 1292, 1146, 1012, 891, 791, 730, 696.

### Example 18

### N-(2-fluoro-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.44 (s, 1H), 8.14 (s, 1H), 7.87 (s, 1H), 7.77 (s, 1H), 7.52 (s, 1H), 7.41 (d, *J* = 11.7 Hz, 1H), 7.32 (s, 2H), 7.08 (s, 1H), 6.33 (s, 1H), 6.22 (s, 1H), 6.11 (d, *J* = 2.4 Hz, 1H), 4.59 (d, *J* = 4.1 Hz, 2H), 3.84 (s, 3H), 3.11 (dd, *J* = 11.7, 4.9 Hz, 4H). TLC-MS (ESI) *m*/*z*: 417.2 [M+H]⁺; 439.2 [M+Na]⁺. HPLC *t_{R}* = 5.06 min. FT-IR(ATR) [cm⁻¹]: 3227, 3029, 2973, 2939, 2713, 2641, 2562, 1629, 1586, 1551, 1440, 1368, 1336, 1280, 1259, 1230, 1167, 1147, 1102, 986, 893, 870, 818, 789, 724, 697, 668.

### Example 19

### 2-(2-(furan-2-yl)ethyl)-N-((1-methyl-1H-pyrazol-4-yl)methyl)-3H-imidazo[4,5-b]pyridin-7-amine

¹H NMR (400 MHz, DMSO) δ 12.38 (s, 1H), 7.77 (s, 1H), 7.58 (s, 1H), 7.51 (s, 1H), 7.37 (s, 1H), 6.74 (s, 1H), 6.31 (d, *J* = 9.1 Hz, 2H), 6.11 (s, 1H), 4.36 (s, 2H), 3.76 (s, 3H), 3.09 (d, *J* = 8.1 Hz, 4H). TLC-MS (ESI) *m*/*z*: 323.3 [M+H]⁺; 345.2 [M+Na]⁺. HPLC *t_{R}* = 2.77 min. FT-IR(ATR) [cm⁻¹]: 3243, 2934, 2841, 2716, 2646, 2602, 2573, 1632, 1583, 1551, 1441, 1405, 1292, 1251, 1230, 1161, 1141, 1101, 1070, 1014, 985, 892, 790, 734, 698.

### Example 20

### 2-(2-(furan-2-yl)ethyl)-N-(4-methoxybenzyl)-3H-imidazo[4,5-b]pyridin-7-amine

¹H NMR (400 MHz, DMSO) δ 12.39 (s, 1H), 7.72 (d, *J* = 3.2 Hz, 1H), 7.52 (s, 1H), 7.29 (d, *J* = 8.2 Hz, 2H), 7.08 (s, 1H), 6.86 (d, *J* = 8.0 Hz, 2H), 6.33 (s, 1H), 6.18 (d, *J* = 3.9 Hz, 1H), 6.11 (d, *J* = 2.5 Hz, 1H), 4.46 (d, *J* = 4.1 Hz, 2H), 3.70 (s, 3H), 3.10 (dd, *J* = 13.0, 5.0 Hz, 4H). TLC-MS (ESI) *m*/*z*: 349.3 [M+H]⁺; 371.3 [M+Na]⁺. HPLC *t_{R}* = 4.88 min. FT-IR(ATR) [cm1]: 3430, 2956, 2935, 2837, 2776, 2741, 2685, 2656, 2600, 2561, 2487, 1610, 1512, 1457, 1437, 1390, 1333, 1304, 1275, 1248, 1175, 1140, 1030, 990, 890, 821, 799, 753.

### Example 24

### 2-(2-(furan-2-yl)ethyl)-N-(pyridin-3-ylmethyl)-3H-imidazo[4,5-b]pyridin-7-amine

¹H NMR (400 MHz, DMSO) δ 12.41 (s, 1H), 8.60 (d, *J* = 1.3 Hz, 1H), 8.47 - 8.38 (m, 1H), 7.79 - 7.71 (m, 2H), 7.51 (s, 1H), 7.32 (dd, *J* = 7.7, 4.8 Hz, 1H), 7.21 (t, *J* = 5.6 Hz, 1H), 6.36 - 6.29 (m, 1H), 6.23 (d, *J* = 5.6 Hz, 1H), 6.11 (d, *J* = 2.9 Hz, 1H), 4.60 (d, *J* = 6.1 Hz, 2H), 3.17 - 3.05 (m, 4H). TLC-MS (ESI) *m*/*z*: 320.2 [M+H]⁺; 342.2 [M+Na]⁺. HPLC *t_{R}* = 1.67 min. FT-IR(ATR) [cm⁻¹]: 3413, 3013, 2922, 2864, 2791, 2683, 2668, 1606, 1522, 1502, 1441, 1422, 1395, 1341, 1318, 1261, 1222, 1144, 1001, 984, 923, 885, 800, 749, 713.

### Example 25

### 2-(2-(furan-2-yl)ethyl)-N-(2-(methylsulfonyl)benzyl)-3H-imidazo[4,5-b]pyridin-7-amine

¹H NMR (400 MHz, DMSO) δ 12.46 (s, 1H), 7.95 (d, *J* = 7.5 Hz, 1H), 7.75 (d, *J* = 5.5 Hz, 1H), 7.63 (t, *J* = 7.2 Hz, 1H), 7.57 (d, *J* = 7.3 Hz, 1H), 7.54 - 7.48 (m, 2H), 7.21 (t, *J* = 6.4 Hz, 1H), 6.32 (dd, *J* = 2.8, 2.0 Hz, 1H), 6.23 (d, *J* = 5.6 Hz, 1H), 6.08 (d, *J* = 2.7 Hz, 1H), 5.05 (d, *J* = 5.8 Hz, 2H), 3.38 (s, 3H), 3.14 - 3.03 (m, 4H). TLC-MS (ESI) *m*/*z*: 397.0 [M+H]⁺; 419.0 [M+Na]⁺. HPLC *t_{R}* = 3.45 min. FT-IR(ATR) [cm⁻¹]: 2987, 2911, 2710, 1602, 1544, 1441, 1385, 1296, 1276, 1149, 1139, 1120, 1056, 970, 885, 798, 748, 727.

### Example 26

### 2-(2-(furan-2-yl)ethyl)-N-(3-(methylsulfonyl)benzyl)-3H-imidazo[4,5-b]pyridin-7-amine

¹H NMR (400 MHz, DMSO) δ 12.43 (s, 1H), 7.97 (s, 1H), 7.75 (dd, *J* = 27.5, 7.7 Hz, 3H), 7.59 (t, *J* = 7.7 Hz, 1H), 7.51 (s, 1H), 7.33 (s, 1H), 6.33 (s, 1H), 6.20 (d, *J* = 5.2 Hz, 1H), 6.11 (d, *J* = 2.6 Hz, 1H), 4.67 (d, *J* = 5.3 Hz, 2H), 3.18 (s, 3H), 3.11 (dd, *J* = 12.9, 5.2 Hz, 4H). TLC-MS (ESI) *m*/*z*: 397.4 [M+H]⁺; 419.5 [M+Na]⁺. HPLC *t_{R}* = 2.86 min. FT-IR(ATR) [cm⁻¹]: 2927, 2657, 2630, 2612, 1629, 1577, 1437, 1296, 1136, 890, 790, 750, 683.

### Example 27

### 2-(2-(furan-2-yl)ethyl)-N-(4-(methylsulfonyl)benzyl)-3H-imidazo[4,5-b]pyridin-7-amine

¹H NMR (400 MHz, DMSO) δ 12.41 (s, 1H), 7.86 (d, *J* = 8.2 Hz, 2H), 7.73 (d, *J* = 5.2 Hz, 1H), 7.62 (d, *J* = 8.3 Hz, 2H), 7.52 (d, *J* = 1.0 Hz, 1H), 7.34 (s, 1H), 6.33 (dd, *J* = 3.1, 1.9 Hz, 1H), 6.15 (d, *J* = 5.5 Hz, 1H), 6.11 (d, *J* = 2.8 Hz, 1H), 4.68 (d, *J* = 5.9 Hz, 2H), 3.17 (s, 3H), 3.16 - 3.04 (m, 4H). TLC-MS (ESI) *m*/*z*: 397.6 [M+H]⁺; 419.7 [M+Na]⁺. HPLC *t_{R}* = 2.67 min. FT-IR(ATR) [cm⁻¹]: 3213, 2641, 2616, 2577, 2562, 2513, 2464, 1630, 1585, 1550, 1437, 1336, 1299, 1139, 1088, 1069, 957, 892, 791, 750.

### Example 33

### 2-(2-(furan-2-yl)ethyl)-N-(4-(1-methyl-1H-1,2,3-triazol-4-yl)benzyl)-3H-imidazo[4,5-b]pyridin-7-amine

¹H NMR (400 MHz, DMSO) δ 12.39 (s, 1H), 8.44 (s, 1H), 7.75 (t, *J* = 8.7 Hz, 3H), 7.52 (d, *J* = 1.1 Hz, 1H), 7.43 (d, J= 8.1 Hz, 2H), 7.20 (s, 1H), 6.33 (dd, *J* = 3.1, 1.9 Hz, 1H), 6.20 (d, *J* = 5.0 Hz, 1H), 6.12 (d, *J* = 2.8 Hz, 1H), 4.58 (d, *J* = 5.4 Hz, 2H), 4.07 (s, 3H), 3.12 (pd, *J* = 9.7, 5.3 Hz, 4H). TLC-MS (ESI) *m*/*z*: 400.3 [M+H]⁺; 422.3 [M+Na]⁺. HPLC *t_{R}* = 3.69 min. FT-IR(ATR) [cm⁻¹]: 3229, 3110, 3082, 3025, 2919, 2846, 2786, 2716, 2650, 2538, 2522, 2495, 2444, 2373, 1629, 1584, 1522, 1437, 1334, 1279, 1222, 1145, 1073, 1013, 890, 792, 732, 699.

### Example 34

### 2-(2-(furan-2-yl)ethyl)-N-(4-(1-methyl-1H-1,2,4-triazol-3-yl)benzyl)-3H-imidazo[4,5-b]pyridin-7-amine

¹H NMR (400 MHz, DMSO) δ 12.42 (s, 1H), 8.48 (s, 1H), 7.93 (d, *J* = 8.0 Hz, 2H), 7.73 (d, *J* = 4.2 Hz, 1H), 7.52 (d, *J* = 0.9 Hz, 1H), 7.44 (d, *J* = 8.0 Hz, 2H), 7.22 (s, 1H), 6.33 (dd, *J* = 2.9, 1.9 Hz, 1H), 6.19 (d, *J* = 4.8 Hz, 1H), 6.12 (d, *J* = 2.9 Hz, 1H), 4.60 (d, *J* = 5.1 Hz, 2H), 3.90 (s, 3H), 3.15 (dd, *J* = 10.6, 5.1 Hz, 2H), 3.09 (dd, *J* = 10.8, 4.9 Hz, 2H). TLC-MS (ESI) *m*/*z*: 400.4 [M+H]⁺; 422.4 [M+Na]⁺. HPLC *t_{R}* = 3.94 min. FT-IR(ATR) [cm⁻¹]: 3319, 3078, 3006, 2922, 2863, 2792, 2743, 2711, 2685, 1610, 1504, 1441, 1395, 1331, 1280, 1221, 1146, 1005, 891, 831, 790, 734.

### Example 45

### 2-(2-(furan-2-yl)ethyl)-N-(4-(1-methyl-1H-pyrazol-4-yl)benzyl)-1H-imidazo[4,5-c]pyridin-4-amine

¹H NMR (400 MHz, DMSO) δ 12.32 (s, 1H), 8.05 (s, 1H), 7.79 (s, 1H), 7.63 (d, *J* = 4.8 Hz, 1H), 7.52 (s, 1H), 7.45 (d, *J* = 6.6 Hz, 2H), 7.33 (d, *J* = 7.3 Hz, 2H), 6.97 (s, 1H), 6.66 (s, 1H), 6.33 (s, 1H), 6.11 (d, *J* = 2.0 Hz, 1H), 4.64 (d, *J* = 5.7 Hz, 2H), 3.84 (s, 3H), 3.13 (s, 4H). TLC-MS (ESI) *m*/*z*: 399.5 [M+H]⁺; 421.6 [M+Na]⁺. HPLC *t_{R}* = 4.88 min. FT-IR(ATR) [cm⁻¹]: 3221, 3105, 3069, 3017, 2983, 2920, 2849, 2780, 2689, 1603, 1507, 1477, 1425, 1400, 1372, 1336, 1291, 1202, 1138, 1072, 1011, 985, 954, 923, 848, 790, 730, 653.

### Example 46

### 2-(2-(furan-2-yl)ethyl)-N-(4-(1-(methyl-d₃)-1H-pyrazol-4-yl)benzyl)-3H-imidazo[4,5-b]pyridin-7-amine

¹H NMR (400 MHz, DMSO) δ 12.40 (s, 1H), 8.06 (s, 1H), 7.79 (s, 1H), 7.72 (d, J= 5.3 Hz, 1H), 7.55 - 7.44 (m, *J* = 16.4, 4.4 Hz, 3H), 7.34 (d, *J* = 7.9 Hz, 2H), 7.14 (t, *J* = 5.3 Hz, 1H), 6.33 (dd, *J* = 2.9, 1.9 Hz, 1H), 6.19 (d, *J* = 5.4 Hz, 1H), 6.12 (d, *J* = 2.9 Hz, 1H), 4.53 (d, J= 5.0 Hz, 2H), 3.18 - 3.04 (m, 4H). TLC-MS (ESI) *m*/*z*: 402.1 [M+H]⁺; 424.1 [M+Na]⁺. HPLC *t_{R}* = 4.53 min. FT-IR(ATR) [cm⁻¹]: 3216, 3025, 2940, 2662, 2607, 1628, 1585, 1545, 1437, 1335, 1292, 1216, 1146, 988, 954, 892, 793, 733.

### Example 47

### 2-(2-(furan-3-yl)ethyl)-N-(4-(1-methyl-1H-pyrazol-4-yl)benzyl)-3H-imidazo[4,5-b]pyridin-7-amine

¹H NMR (400 MHz, DMSO) δ 12.37 (s, 1H), 8.06 (s, 1H), 7.80 (s, 1H), 7.73 (s, 1H), 7.54 (s, 1H), 7.48 (d, *J* = 9.1 Hz, 3H), 7.34 (d, *J*= 8.0 Hz, 2H), 7.12 (s, 1H), 6.37 (s, 1H), 6.20 (d, *J*= 3.4 Hz, 1H), 4.53 (d, *J*= 4.7 Hz, 2H), 3.84 (s, 3H), 3.01 (t, *J*= 7.3 Hz, 2H), 2.90 (t, 2H). TLC-MS (ESI) *m*/*z*: 399.3 [M+H]⁺; 421.3 [M+Na]⁺. HPLC *t_{R} =* 4.27 min. FT-IR(ATR) [cm⁻¹]: 3400, 3066, 2921, 2688, 1606, 1448, 1331, 1276, 1215, 984, 873, 792.

### Example 48

### N-(4-(1-methyl-1H-pyrazol-4-yl)benzyl)-2-(2-(thiophen-2-yl)ethyl)-3H-imidazo[4,5-b]pyridin-7-amine

¹H NMR (400 MHz, DMSO) δ 12.40 (s, 1H), 8.06 (s, 1H), 7.80 (s, 1H), 7.73 (d, *J* = 3.9 Hz, 1H), 7.48 (d, *J* = 8.0 Hz, 2H), 7.34 (d, *J* = 8.1 Hz, 2H), 7.30 (dd, *J* = 5.1, 1.2 Hz, 1H), 7.13 (s, 1H), 6.92 (dd, *J* = 5.0, 3.4 Hz, 1H), 6.88 (d, *J* = 2.5 Hz, 1H), 6.20 (d, *J* = 4.2 Hz, 1H), 4.53 (d, *J* = 5.8 Hz, 2H), 3.84 (s, 3H), 3.33 (t, *J* = 7.5 Hz, 4H), 3.11 (t, *J* = 7.6 Hz, 2H). TLC-MS (ESI) *m*/*z*: 414.9 [M+H]⁺; 436.9 [M+Na]⁺. HPLC *t_{R}* = 4.93 min. FT-IR(ATR) [cm⁻¹]: 3230, 3030, 2935, 2646, 1627, 1436, 1335, 1291, 1140, 987, 892, 795, 700.

### Example 56

### 2-isobutyl-N-(4-(1-methyl-1H-pyrazol-4-yl)benzyl)-3H-imidazo[4,5-b]pyridin-7-amine

¹H NMR (400 MHz, DMSO) δ 12.27 (s, 1H), 8.06 (s, 1H), 7.80 (s, 1H), 7.73 (s, 1H), 7.48 (d, *J* = 7.9 Hz, 2H), 7.35 (d, *J* = 8.0 Hz, 2H), 7.11 (s, 1H), 6.20 (d, *J* = 2.4 Hz, 1H), 4.52 (d, *J* = 5.6 Hz, 2H), 3.84 (s, 3H), 2.62 (d, *J* = 7.2 Hz, 2H), 2.15 (tt, *J* = 13.5, 6.5 Hz, 1H), 0.93 (d, *J* = 6.6 Hz, 6H). TLC-MS (ESI) *m*/*z*: 361.0 [M+H]⁺; 383.0 [M+Na]⁺. HPLC *t_{R}* = 4.61 min. FT-IR(ATR) [cm⁻¹]: 2954, 2870, 1609, 1522, 1332, 1281, 1216, 985, 954, 792.

### Example 57

### (4-(((2-(2-(furan-2-yl)ethyl)-3H-imidazo[4,5-b]pyridin-7-yl)amino)methyl)phenyl)dimethyl phosphine oxide

¹H NMR (400 MHz, DMSO) δ 12.43 (s, 1H), 7.75 - 7.66 (m, 3H), 7.53 - 7.47 (m, *J* = 8.5, 2.3 Hz, 3H), 7.25 (s, 1H), 6.33 (dd, *J* = 3.0, 1.9 Hz, 1H), 6.17 (d, *J* = 4.7 Hz, 1H), 6.11 (d, *J* = 2.9 Hz, 1H), 4.61 (d, *J* = 5.8 Hz, 2H), 3.11 (qt, *J* = 9.7, 4.9 Hz, 4H), 1.60 (d, *J* = 13.3 Hz, 6H). TLC-MS (ESI) *m*/*z*: 394.9 [M+H]⁺; 416.9 [M+Na]⁺. HPLC *t_{R} =* 2.47 min. FT-IR(ATR) [cm⁻¹]: 3239, 3021, 2973, 2911, 2659, 1629, 1583, 1436, 1338, 1303, 1170, 1110, 888, 792, 732.

### Example 70

### N-(4-chlorobenzyl)-2-(2-(furan-2-yl)ethyl)-3H-imidazo[4,5-b]pyridin-7-amine

¹H NMR (400 MHz, DMSO) δ 12.42 (s, 1H), 7.73 (d, *J* = 4.0 Hz, 1H), 7.52 (d, *J* = 0.8 Hz, 1H), 7.43 - 7.32 (m, 4H), 7.21 (s, 1H), 6.33 (dd, *J* = 2.9, 1.9 Hz, 1H), 6.16 (d, *J* = 3.9 Hz, 1H), 6.11 (d, *J* = 2.7 Hz, 1H), 4.54 (d, *J* = 6.0 Hz, 2H), 3.11 (td, *J* = 10.7, 5.7 Hz, 4H). TLC-MS (ESI) *m*/*z*: 353.1 [M+H]⁺; 375.1 [M+Na]⁺. HPLC *t_{R}* = 5.73 min. FT-IR(ATR) [cm⁻¹]: 3079, 2936, 2823, 2715, 1600, 1517, 1457, 1371, 1274, 1069, 978, 783.

### Example 71

### N-(2-bromobenzyl)-2-(2-(furan-2-yl)ethyl)-3H-imidazo[4,5-b]pyridin-7-amine

¹H NMR (400 MHz, DMSO) δ 12.45 (s, 1H), 7.75 (d, *J* = 4.5 Hz, 1H), 7.63 (d, *J* = 7.8 Hz, 1H), 7.52 (d, *J* = 1.0 Hz, 1H), 7.35 - 7.27 (m, 2H), 7.26 - 7.10 (m, 2H), 6.33 (dd, *J* = 3.1, 1.9 Hz, 1H), 6.11 (d, *J* = 2.8 Hz, 1H), 6.07 (d, *J* = 2.9 Hz, 1H), 4.59 (d, *J* = 5.7 Hz, 2H), 3.17 - 3.06 (m, *J* = 9.7, 4.9 Hz, 4H). TLC-MS (ESI) *m*/*z*: 398.0 [M+H]⁺; 420.0 [M+Na]⁺. HPLC *t_{R}* = 5.60 min. FT-IR(ATR) [cm⁻¹]: 3079, 2936, 2823, 2715, 1600, 1517, 1457, 1371, 1274, 1069, 978, 783.

### Example 80

### 2-(2-(furan-2-yl)ethyl)-N-(4-(pentafluoro-l6-sulfaneyl)benzyl)-3H-imidazo[4,5-b]pyridin-7-amine

¹H NMR (400 MHz, DMSO) δ 12.45 (s, 1H), 7.84 (d, *J* = 8.6 Hz, 2H), 7.74 (d, J= 4.7 Hz, 1H), 7.57 (d, *J* = 8.3 Hz, 2H), 7.52 (s, 1H), 7.33 (s, 1H), 6.36 - 6.29 (m, *J* = 2.0 Hz, 1H), 6.15 (d, *J* = 4.8 Hz, 1H), 6.10 (d, *J* = 2.7 Hz, 1H), 4.66 (d, *J* = 5.6 Hz, 2H), 3.11 (td, *J* = 11.2, 6.3 Hz, 4H). TLC-MS (ESI) *m*/*z*: 445.2 [M+H]⁺; 467.2 [M+Na]⁺. HPLC *t_{R}* = 6.63 min. FT-IR(ATR) [cm⁻¹]: 3079, 2936, 2823, 2715, 1600, 1517, 1457, 1371, 1274, 1069, 978, 783.

### Example 72

### N-(4-(1-methyl-1H-pyrazol-4-yl)benzyl)-2-(2-(tetrahydrofuran-3-yl)ethyl)-3H-imidazo[4,5-b]pyridin-7-amine

¹H NMR (600 MHz, DMSO) δ 12.32 (s, 1H), 8.06 (s, 1H), 7.80 (s, 1H), 7.73 (s, 1H), 7.48 (d, *J* = 7.9 Hz, 2H), 7.34 (d, *J* = 8.0 Hz, 2H), 7.10 (s, 1H), 6.20 (s, 1H), 3.84 (s, 3H), 3.80 - 3.76 (m, 1H), 3.72 (td, *J* = 8.2, 4.8 Hz, 1H), 3.61 (dd, *J* = 15.6, 7.6 Hz, 1H), 3.28 - 3.24 (m, 1H), 2.82 - 2.73 (m, 2H), 2.14 (dt, *J* = 14.7, 7.3 Hz, 1H), 1.99 (dtd, *J* = 12.3, 7.6, 4.9 Hz, 1H), 1.85 - 1.78 (m, 2H), 1.48 (dq, *J* = 12.1, 7.7 Hz, 1H). TLC-MS (ESI) *m*/*z*: 403.2 [M+H]⁺; 425.2 [M+Na]⁺. HPLC *t_{R}* = 3.87 min. FT-IR(ATR) [cm⁻¹]: 3079, 2936, 2823, 2715, 1600, 1517, 1457, 1371, 1274, 1069, 978, 783.

### Example 83

### 2-(2-(furan-2-yl)ethyl)-3-methyl-N-(4-(1-methyl-1H-pyrazol-4-yl)benzyl)-3H-imidazo[4,5-b]pyridin-7-amine

¹H NMR (400 MHz, DMSO) δ 8.06 (s, 1H), 7.79 (s, 1H), 7.77 (d, J= 5.6 Hz, 1H), 7.57 - 7.53 (m, 1H), 7.47 (d, *J* = 8.2 Hz, 2H), 7.33 (d, *J* = 8.2 Hz, 2H), 7.21 (t, *J* = 6.4 Hz, 1H), 6.36 (dd, *J* = 3.1, 1.9 Hz, 1H), 6.24 (d, *J* = 5.6 Hz, 1H), 6.18 (d, *J* = 3.0 Hz, 1H), 4.57 (d, *J* = 5.8 Hz, 2H), 3.84 (s, 3H), 3.61 (s, 3H), 3.16 (s, 4H). TLC-MS (ESI) *m*/*z*: 413.3 [M+H]⁺; 435.3 [M+Na]⁺. HPLC *t_{R}* = 5.49 min. FT-IR(ATR) [cm⁻¹]: 3079, 2936, 2823, 2715, 1600, 1517, 1457, 1371, 1274, 1069, 978, 783.

### Example 85

### N-(4-(1-methyl-1H-pyrazol-4-yl)benzyl)-2-(3-phenylpropyl)-3H-imidazo[4,5-b]pyridin-7-amine

¹H NMR (400 MHz, DMSO) δ 12.35 (s, 1H), 8.06 (s, 1H), 7.79 (s, 1H), 7.73 (d, J= 1.9 Hz, 1H), 7.48 (d, J= 7.9 Hz, 2H), 7.34 (d, *J* = 8.0 Hz, 2H), 7.31 - 7.26 (m, *J* = 7.4 Hz, 2H), 7.25 - 7.16 (m, 3H), 7.11 (s, 1H), 6.20 (d, J= 1.9 Hz, 1H), 4.53 (d, *J* = 5.6 Hz, 2H), 3.84 (s, 3H), 2.78 (t, *J* = 7.5 Hz, 2H), 2.66 (t, *J* = 7.6 Hz, 2H), 2.12 - 2.01 (m, 2H). TLC-MS (ESI) *m*/*z*: 423.4 [M+H]⁺; 445.4 [M+Na]⁺. HPLC *t_{R}* = 5.83 min. FT-IR(ATR) [cm⁻¹]: 3079, 2936, 2823, 2715, 1600, 1517, 1457, 1371, 1274, 1069, 978, 783.

### Example 88

### N-(4-(1-methyl-1H-pyrazol-4-yl)benzyl)-2-(phenoxymethyl)-3H-imidazo[4,5-b]pyridin-7-amine

¹H NMR (400 MHz, DMSO) δ 12.83 (s, 1H), 8.07 (s, 1H), 7.80 (s, 2H), 7.49 (d, *J* = 7.8 Hz, 2H), 7.40 - 7.26 (m, *J* = 16.1, 7.6 Hz, 5H), 7.08 (d, *J* = 7.9 Hz, 2H), 6.97 (t, *J* = 7.3 Hz, 1H), 6.25 (d, *J* = 3.0 Hz, 1H), 5.22 (s, 2H), 4.55 (s, 2H), 3.84 (s, 3H). TLC-MS (ESI) *m*/*z*: 411.0 [M+H]⁺; 433.0 [M+Na]⁺. HPLC *t_{R}* = 5.24 min. FT-IR(ATR) [cm⁻¹]: 3079, 2936, 2823, 2715, 1600, 1517, 1457, 1371, 1274, 1069, 978, 783.

### Example 96

### 2-(2-(furan-2-yl)ethyl)-N-(4-(trifluoromethyl)benzyl)-3H-imidazo[4,5-b]pyridin-7-amine

¹H NMR (400 MHz, DMSO) δ 12.43 (s, 1H), 7.73 (d, *J* = 5.1 Hz, 1H), 7.67 (d, *J* = 8.1 Hz, 2H), 7.57 (d, *J* = 8.1 Hz, 2H), 7.51 (d, *J* = 1.0 Hz, 1H), 7.31 (s, 1H), 6.33 (dd, *J* = 3.0, 1.9 Hz, 1H), 6.15 (d, *J* = 5.4 Hz, 1H), 6.11 (d, *J* = 2.9 Hz, 1H), 4.66 (d, *J* = 5.8 Hz, 2H), 3.19 - 3.04 (m, *J* = 9.9, 4.9 Hz, 4H). TLC-MS (ESI) *m*/*z*: 387.1 [M+H]⁺; 409.0 [M+Na]⁺. HPLC *t_{R}* = 6.12 min. FT-IR(ATR) [cm⁻¹]: 3226, 3030, 2932, 2642, 1630, 1437, 1326, 1117, 1067, 1014, 892, 792, 734.

### Shown as an example for Synthesis in Synthetic scheme C

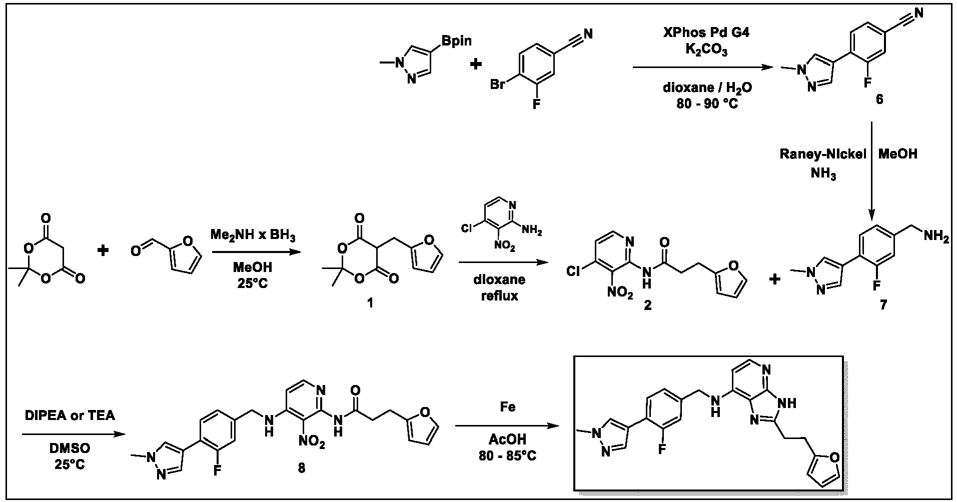

### 3-fluoro-4-(1-methyl-1H-pyrazol-4-yl)benzonitrile [6]

The mixture of 4-bromo-3-fluorobenzonitrile (1.00 eq.), 1-methyl-4-(4,4,5,5-tetramethyl-1,3,2-dioxaborolan-2-yl)-1*H*-pyrazole (1.10 eq.), K₂CO₃ (3.00 eq.) and XPhos Pd G4 (0.005 eq.) in 1,4-Dioxane / water (4+1; 2.85 ml/mmol of Dioxane) was stirred under nitrogen-atmosphere at 80 - 90°C overnight until complete consumption of the starting materials was observed. The mixture was cooled to room temperature, diluted dropwise with MeOH / H₂O (40/60) under constant stirring and cooled in an ice bath until complete precipitation of the product was observed. The precipitate was collected by filtration, washed with MeOH / H₂O (40/60) and dried. The product was obtained as a white solid and used without further purification.

¹H NMR (400 MHz, DMSO) δ 8.35 (d, *J* = 2.4 Hz, 1H), 8.09 (s, 1H), 7.98 (t, *J* = 8.0 Hz, 1H), 7.93 (dd, *J* = 11.3, 1.5 Hz, 1H), 7.74 (dd, *J* = 8.1, 1.6 Hz, 1H), 3.97 (s, 3H). TLC-MS (ESI) *m*/*z*: n.d.. HPLC *t_{R}* = 5.47 min.

### (3-fluoro-4-(1-methyl-1H-pyrazol-4-yl)phenyl)methylamine [7]

An aqueous suspension of Raney nickel was added to a solution of 6in 7N ammonia in methanol (10.00 ml/mmol). The reaction flask was then first flooded with nitrogen, followed by bubbling the solution with hydrogen for five minutes. The mixture was stirred under a hydrogen atmosphere for 16 h at room temperature until the reaction control showed complete conversion of the starting material. The hydrogen was then removed, the Raney nickel separated over diatomaceous earth and rinsed several times with methanol. The organic phase was then removed under reduced pressure and the resulting solid (partly also oily liquid) was used without further purification.

HPLC *t_{R}* = 1.91 min (96%)

### N-(4-((3-fluoro-4-(1-methyl-1H-pyrazol-4-yl)benzyl)amino)-3-nitropyridin-2-yl)-3-(furan-2-yl)propanamide [8]

To a mixture of 2 (1.00 eq.) and 7(1.05 eq.) in dimethyl sulfoxide (6.00 ml/mmol) was added DIPEA (2.00 eq.). The mixture was stirred at room temperature overnight until complete consumption of the starting materials. The mixture was diluted with methanol followed by addition of aqueous methanol (60% v/v) to induce precipitation of the product. The product was and dried at room temperature under vacuum to obtain the product as a yellow solid that was used without further purification. (*crude*)

### Example 30

### N-(3-fluoro-4-(1-methyl-1H-pyrazol-4-yl)benzyl)-2-(2-(furan-2-yl)ethyl)-3H-imidazo[4,5-b]pyridin-7-amine

To a solution of 5 (1.00 eq.) in a mixture of acetic acid and water (2.5 : 1; 7 ml/mmol acetic acid) was added powdered iron (3.00 eq.). The mixture was stirred at 85°C for 2 h until complete consumption of the starting material. After complete conversion of the reaction, the batch was cooled down to room temperature, diluted with water and NaOH-solution and filtered. The product was extracted from the clear mixture with ethyl acetate and washed with brine. The organic phase was dried over Na₂SO₄, filtered, and evaporated to dryness. After purification via column-chromatography (from 100% DCM to DCM/MeOH 9:1) the desired product was obtained as a white solid.

¹H NMR (400 MHz, DMSO) δ 12.42 (s, 1H), 8.07 (s, 1H), 7.83 (s, 1H), 7.73 (d, J= 5.1 Hz, 1H), 7.62 (t, *J* = 8.0 Hz, 1H), 7.52 (s, 1H), 7.28 - 7.14 (m, 3H), 6.33 (s, 1H), 6.19 (d, *J* = 5.2 Hz, 1H), 6.11 (s, 1H), 4.56 (d, *J* = 3.9 Hz, 2H), 3.87 (s, 3H), 3.17 - 3.06 (m, *J* = 13.4, 5.1 Hz, 4H). TLC-MS (ESI) *m*/*z*: 417.1 [M+H]⁺; 439.1 [M+Na]⁺. HPLC *t_{R}* = 5.68 min. FT-IR(ATR) [cm⁻¹]: 3076, 3013, 2936, 2857, 2797, 2745, 2727, 2696, 1608, 1570, 1522, 1442, 1394, 1362, 1330, 1277, 1224, 1123, 1098, 1014, 989, 964, 888, 789, 770, 722, 661.

### The following examples were prepared using the procedures above:

### Example 31

### 2-(2-(furan-2-yl)ethyl)-N-(2-methoxy-4-(1-methyl-1H-pyrazol-4-yl)benzyl)-3H-imidazo[4,5-b]pyridin-7-amine

¹H NMR (400 MHz, DMSO) δ 12.41 (s, 1H), 8.11 (s, 1H), 7.85 (s, 1H), 7.75 (s, 1H), 7.52 (d, *J* = 0.9 Hz, 1H), 7.17 (d, *J* = 8.1 Hz, 2H), 7.04 (d, *J* = 7.6 Hz, 1H), 6.84 (s, 1H), 6.33 (dd, *J* = 2.8, 2.0 Hz, 1H), 6.21 - 6.07 (m, *J* = 7.4 Hz, 2H), 4.47 (d, *J* = 4.7 Hz, 2H), 3.91 (s, 3H), 3.84 (s, 3H), 3.18 - 3.05 (m, *J* = 10.9, 6.1 Hz, 4H). TLC-MS (ESI) *m*/*z*: 429.2 [M+H]⁺; 451.2 [M+Na]⁺. HPLC *t_{R}* = 5.13 min. FT-IR(ATR) [cm⁻¹]: 3237, 2933, 2841, 2748, 2726, 2663, 2562, 2522, 1632, 1577, 1502, 1437, 1362, 1337, 1280, 1247, 1208, 1190, 1148, 1115, 1071, 1037, 1014, 983, 890, 855, 789, 733, 654.

### Example 32

### 2-(2-(furan-2-yl)ethyl)-N-(3-methoxy-4-(1-methyl-1H-pyrazol-4-yl)benzyl)-3H-imidazo[4,5-b]pyridin-7-amine

¹H NMR (400 MHz, DMSO) δ 12.41 (s, 1H), 8.04 (s, 1H), 7.82 (s, 1H), 7.73 (d, *J* = 3.7 Hz, 1H), 7.54 - 7.44 (m, 2H), 7.12 (d, *J* = 8.3 Hz, 2H), 6.95 (d, *J* = 7.1 Hz, 1H), 6.33 (s, 1H), 6.22 (d, *J* = 4.1 Hz, 1H), 6.11 (s, 1H), 4.54 (d, *J* = 1.9 Hz, 2H), 3.83 (d, *J* = 8.3 Hz, 6H), 3.17 - 3.06 (m, *J* = 8.1 Hz, 4H). TLC-MS (ESI) *m*/*z*: 429.8 [M+H]⁺; 451.8 [M+Na]⁺. HPLC *t_{R}* = 4.84 min. FT-IR(ATR) [cm⁻¹]: 2716, 2644, 2600, 2579, 2525, 1617, 1599, 1560, 1442, 1419, 1331, 1276, 1252, 1187, 1169, 1132, 1034, 986, 954, 890, 800, 730, 662.

### Example 49

### 2-(2-(furan-2-yl)ethyl)-N-((6-(1-methyl-1H-pyrazol-4-yl)pyridin-3-yl)methyl)-3H-imidazo[4,5-b]pyridin-7-amine

¹H NMR (400 MHz, DMSO) δ 12.42 (s, 1H), 8.52 (d, *J* = 1.3 Hz, 1H), 8.21 (s, 1H), 7.93 (s, 1H), 7.73 (dd, *J* = 11.5, 3.4 Hz, 2H), 7.56 (d, *J* = 8.1 Hz, 1H), 7.51 (d, *J* = 1.2 Hz, 1H), 7.21 (s, 1H), 6.33 (dd, *J* = 3.0, 1.9 Hz, 1H), 6.25 (d, *J* = 5.4 Hz, 1H), 6.11 (d, *J* = 3.0 Hz, 1H), 4.57 (d, *J* = 5.3 Hz, 2H), 3.86 (s, 3H), 3.16 - 3.05 (m, 4H). TLC-MS (ESI) *m*/*z*: 399.9 [M+H]⁺; 421.9 [M+Na]⁺. HPLC *t_{R}* = 2.73 min. FT-IR(ATR) [cm⁻¹]: 3390, 2735, 1620, 1595, 1517, 1390, 1334, 1229, 1140, 964, 886, 860, 798, 745.

### Example 50

### N-(2,6-difluoro-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.42 (s, 1H), 8.25 (s, 1H), 7.97 (s, 1H), 7.83 (s, 1H), 7.50 (s, 1H), 7.35 (d, *J* = 8.5 Hz, 2H), 6.72 (s, 1H), 6.42 - 6.27 (m, 2H), 6.11 (d, *J* = 2.4 Hz, 1H), 4.59 (s, 2H), 3.85 (s, 3H), 3.16 - 3.03 (m, *J* = 11.2, 4.5 Hz, 4H). TLC-MS (ESI) *m*/*z*: 435.1 [M+H]⁺; 457.1 [M+Na]⁺. HPLC *t_{R}* = 5.00 min. FT-IR(ATR) [cm⁻¹]: 3071, 2935, 2859, 2786, 1617, 1337, 1169, 1144, 1055, 984, 841, 793, 725.

### Example 51

### N-(2,5-difluoro-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.46 (s, 1H), 8.13 (s, 1H), 7.92 (s, 1H), 7.78 (d, *J* = 3.5 Hz, 1H), 7.60 (dd, *J* = 10.5, 6.2 Hz, 1H), 7.51 (s, 1H), 7.30 - 7.05 (m, 2H), 6.32 (s, 1H), 6.24 (d, *J* = 3.7 Hz, 1H), 6.10 (d, *J* = 2.0 Hz, 1H), 4.61 (d, *J* = 3.7 Hz, 2H), 3.87 (s, 3H), 3.11 (dd, *J* = 11.0, 4.6 Hz, 4H). TLC-MS (ESI) *m*/*z*: 435.1 [M+H]⁺; 457.0 [M+Na]⁺. HPLC *t_{R}* = 5.13 min. FT-IR(ATR) [cm⁻¹]: 3224, 2937, 2642, 1630, 1440, 1366, 1323, 1157, 985, 892, 788, 734.

### Example 52

### N-(2,3-difluoro-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.45 (s, 1H), 8.14 (d, *J* = 1.1 Hz, 1H), 7.86 (s, 1H), 7.78 (d, *J* = 4.9 Hz, 1H), 7.51 (d, *J* = 0.8 Hz, 1H), 7.42 (t, *J* = 7.3 Hz, 1H), 7.21 - 7.08 (m, 2H), 6.35 - 6.30 (m, 1H), 6.25 (d, *J* = 4.4 Hz, 1H), 6.10 (d, *J* = 2.9 Hz, 1H), 4.66 (d, *J* = 5.7 Hz, 2H), 3.88 (s, 3H), 3.17 - 3.06 (m, 4H). TLC-MS (ESI) *m*/*z*: 435.2 [M+H]⁺; 457.1 [M+Na]⁺. HPLC *t_{R}* = 5.32 min. FT-IR(ATR) [cm⁻¹]: 3311, 2922, 2866, 1623, 1601, 1465, 1391, 1339, 1118, 1042, 866, 795, 741.

### Example 53

### N-(3,5-difluoro-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.42 (s, 1H), 8.09 (s, 1H), 7.76 (d, *J* = 8.1 Hz, 2H), 7.51 (d, *J* = 0.9 Hz, 1H), 7.28 (s, 1H), 7.16 (d, *J* = 9.5 Hz, 2H), 6.32 (dd, *J* = 2.8, 1.9 Hz, 1H), 6.21 (d, *J* = 5.5 Hz, 1H), 6.11 (d, *J* = 2.8 Hz, 1H), 4.58 (d, *J* = 5.7 Hz, 2H), 3.90 (s, 3H), 3.17 - 3.06 (m, 4H). TLC-MS (ESI) *m*/*z*: 435.0 [M+H]⁺; 457.0 [M+Na]⁺. HPLC *t_{R}* = 5.33 min. FT-IR(ATR) [cm⁻¹]: 3267, 2933, 2658, 1609, 1436, 1336, 1181, 1023, 987, 867, 797, 728.

### Example 54

### 2-(2-(furan-2-yl)ethyl)-N-(2,3,5,6-tetrafluoro-4-(1-methyl-1H-pyrazol-4-yl)benzyl)-3H-imidazo[4,5-b]pyridin-7-amine

¹H NMR (400 MHz, DMSO) δ 12.44 (s, 1H), 8.25 (s, 1H), 7.87 (s, 1H), 7.83 (d, *J* = 5.5 Hz, 1H), 7.50 (d, *J* = 0.9 Hz, 1H), 6.98 (t, *J* = 5.6 Hz, 1H), 6.37 - 6.28 (m, 2H), 6.09 (d, *J* = 2.9 Hz, 1H), 4.75 (d, *J* = 5.0 Hz, 2H), 3.93 (s, 3H), 3.14 - 3.04 (m, 4H). TLC-MS (ESI) *m*/*z*: 471.1 [M+H]⁺; 493.1 [M+Na]⁺. HPLC *t_{R}* = 5.75 min. FT-IR(ATR) [cm⁻¹]: 3354, 3074, 2940, 2723, 1620, 1478, 1334, 1162, 1006, 924, 792, 732.

### Example 58

### 2-(2-(furan-2-yl)ethyl)-N-(4-(1-methyl-1H-pyrazol-4-yl)-2-(methylsulfonyl)benzyl)-3H-imidazo[4,5-b]pyridin-7-amine

¹H NMR (400 MHz, DMSO) δ 12.46 (s, 1H), 8.23 (s, 1H), 8.04 (d, *J* = 1.0 Hz, 1H), 7.89 (s, 1H), 7.78 (dd, J= 16.6, 6.3 Hz, 2H), 7.59 - 7.46 (m, *J* = 15.9, 4.4 Hz, 2H), 7.18 (s, 1H), 6.34 - 6.29 (m, 1H), 6.25 (d, *J* = 5.3 Hz, 1H), 6.08 (d, *J* = 2.3 Hz, 1H), 5.01 (d, *J* = 3.6 Hz, 2H), 3.86 (s, 3H), 3.41 (s, 3H), 3.15 - 3.02 (m, *J* = 11.8, 4.9 Hz, 4H). TLC-MS (ESI) *m*/*z*: 476.9 [M+H]⁺; 498.9 [M+Na]⁺. HPLC *t_{R}* = 3.85 min. FT-IR(ATR) [cm⁻¹]: 2929, 2725, 1603, 1522, 1342, 1287, 1225, 1129, 977, 776.

### Example 59

### 2-(2-(furan-2-yl)ethyl)-N-(2-methyl-4-(1-methyl-1H-pyrazol-4-yl)benzyl)-3H-imidazo[4,5-b]pyridin-7-amine

¹H NMR (400 MHz, DMSO) δ 12.42 (s, 1H), 8.05 (s, 1H), 7.82 - 7.71 (m, *J* = 17.2 Hz, 2H), 7.52 (s, 1H), 7.39 (s, 1H), 7.28 (d, *J* = 7.5 Hz, 1H), 7.19 (d, *J* = 7.6 Hz, 1H), 6.92 (s, 1H), 6.33 (dd, *J* = 2.8, 2.0 Hz, 1H), 6.23 - 6.09 (m, *J* = 10.3 Hz, 2H), 4.53 (d, 2H), 3.84 (s, 3H), 3.17 - 3.06 (m, 4H), 2.36 (s, 3H). TLC-MS (ESI) *m*/*z*: 413.3 [M+H]⁺; 435.3 [M+Na]⁺. HPLC *t_{R}* = 4.97 min. FT-IR(ATR) [cm⁻¹]: 3234, 2925, 2655, 1631, 1441, 1335, 1147, 1073, 980, 889, 790.

### Example 73

### N-(2-(difluoromethoxy)-4-(1-methyl-1H-pyrazol-4-yl)benzyl)-2-(2-(furan-2-yl)ethyl)-3H-imidazo[4,5-b]pyridin-7-amine

¹H NMR (600 MHz, DMSO) δ 12.37 (s, 1H), 8.13 (s, 1H), 7.85 (s, 1H), 7.75 (s, 1H), 7.51 (s, 1H), 7.39 - 7.35 (m, 2H), 7.47 - 7.21 (m, *J* = 74.2, 57.9, 49.8 Hz, 1H), 7.31 (d, *J* = 7.6 Hz, 1H), 7.06 (s, 1H), 6.36 - 6.30 (m, 1H), 6.14 (s, 1H), 6.11 (d, *J* = 2.7 Hz, 1H), 4.56 (s, 2H), 3.85 (s, 3H), 3.11 (td, *J* = 13.8, 6.5 Hz, 4H). TLC-MS (ESI) *m*/*z*: 465.3 [M+H]⁺; 487.3 [M+Na]⁺. HPLC *t_{R}* = 5.13 min. FT-IR(ATR) [cm⁻¹]: 3079, 2936, 2823, 2715, 1600, 1517, 1457, 1371, 1274, 1069, 978, 783.

### Example 77

### 2-(2-(furan-2-yl)ethyl)-N-(4-(1-methyl-1H-pyrazol-4-yl)-2-(trifluoromethyl)benzyl)-3H-imidazo[4,5-b]pyridin-7-amine

¹H NMR (400 MHz, DMSO) δ 12.54 (s, 1H), 8.25 (s, 1H), 7.93 (s, 1H), 7.88 (d, *J* = 2.1 Hz, 1H), 7.80 - 7.76 (m, 1H), 7.76 - 7.72 (m, 1H), 7.51 (dd, *J* = 1.9, 0.8 Hz, 1H), 7.49 (d, *J* = 8.1 Hz, 1H), 7.38 - 7.20 (m, 1H), 6.32 (dd, *J* = 3.1, 1.8 Hz, 1H), 6.11 (d, *J* = 3.2 Hz, 1H), 6.04 (d, *J* = 5.6 Hz, 1H), 4.76 - 4.70 (m, 2H), 3.85 (s, 3H), 3.19 - 3.13 (m, 2H), 3.13 - 3.05 (m, 2H). TLC-MS (ESI) *m*/*z*: 466.8 [M+H]⁺; 488.8 [M+Na]⁺. HPLC *t_{R}* = 5.81 min. FT-IR(ATR) [cm⁻¹]: 3079, 2936, 2823, 2715, 1600, 1517, 1457, 1371, 1274, 1069, 978, 783.

### Example 78

### 2-(2-(furan-2-yl)ethyl)-N-(4-(1-methyl-1H-pyrazol-4-yl)-3-(trifluoromethyl)benzyl)-3H-imidazo[4,5-b]pyridin-7-amine

¹H NMR (400 MHz, DMSO) δ 12.48 (s, 1H), 7.84 (s, 1H), 7.82 (d, *J* = 1.8 Hz, 1H), 7.75 (d, *J* = 5.6 Hz, 1H), 7.65 (dd, *J* = 7.9, 1.9 Hz, 1H), 7.52 (s, 1H), 7.51 (d, *J* = 1.8 Hz, 1H), 7.43 (d, *J* = 7.9 Hz, 1H), 7.35 (s, 1H), 6.32 (dd, *J* = 3.2, 1.9 Hz, 1H), 6.23 (d, *J* = 5.6 Hz, 1H), 6.11 (d, *J* = 3.2 Hz, 1H), 4.70 - 4.61 (m, *J* = 6.4 Hz, 2H), 3.89 - 3.85 (m, 3H), 3.17 - 3.12 (m, 2H), 3.12 - 3.07 (m, 2H). TLC-MS (ESI) *m*/*z*: 466.8 [M+H]⁺; 488.7 [M+Na]⁺. HPLC *t_{R} =* 5.54 min. FT-IR(ATR) [cm⁻¹]: 3079, 2936, 2823, 2715, 1600, 1517, 1457, 1371, 1274, 1069, 978, 783.

### Example 81

### 2-(2-(furan-2-yl)ethyl)-N-((4-(1-methyl-1H-pyrazol-4-yl)naphthalen-1-yl)methyl)-3H-imidazo[4,5-b]pyridin-7-amine

¹H NMR (400 MHz, DMSO) δ 12.47 (s, 1H), 8.29 (d, *J* = 8.1 Hz, 1H), 8.22 - 8.16 (m, 1H), 8.02 (s, 1H), 7.77 (s, 1H), 7.68 (s, 1H), 7.59 (dtd, *J* = 16.5, 6.8, 1.2 Hz, 2H), 7.54 - 7.47 (m, 2H), 7.40 (d, *J* = 7.3 Hz, 1H), 7.20 (s, 1H), 6.32 (dd, *J* = 3.1, 1.9 Hz, 1H), 6.27 (s, 1H), 6.10 (d, *J* = 2.9 Hz, 1H), 5.06 (d, *J* = 4.7 Hz, 2H), 3.94 (s, 3H), 3.16 - 3.06 (m, 4H). TLC-MS (ESI) *m*/*z*: 449.3 [M+H]⁺; 471.3 [M+Na]⁺. HPLC *t_{R}* = 5.85 min. FT-IR(ATR) [cm⁻¹]: 3079, 2936, 2823, 2715, 1600, 1517, 1457, 1371, 1274, 1069, 978, 783.

### Example 82

### 2-(2-(furan-2-yl)ethyl)-N-(4-(1-methyl-1H-pyrazol-4-yl)-2-(trifluoromethoxy)benzyl)-3H-imidazo[4,5-b]pyridin-7-amine

¹H NMR (400 MHz, DMSO) δ 12.45 (s, 1H), 8.03 (s, 1H), 7.81 - 7.71 (m, 2H), 7.66 (d, *J* = 7.9 Hz, 1H), 7.51 (d, *J* = 1.0 Hz, 1H), 7.44 - 7.37 (m, 2H), 7.35 - 7.24 (m, *J* = 5.7 Hz, 1H), 6.32 (dd, *J* = 2.9, 1.9 Hz, 1H), 6.22 (s, 1H), 6.11 (d, *J* = 2.9 Hz, 1H), 4.61 (d, *J* = 5.8 Hz, 2H), 3.88 (s, 3H), 3.18 - 3.05 (m, *J* = 11.3, 5.5 Hz, 4H). TLC-MS (ESI) *m*/*z*: 483.2 [M+H]⁺; 505.2 [M+Na]⁺. HPLC *t_{R}* = 5.89 min. FT-IR(ATR) [cm⁻¹]: 3079, 2936, 2823, 2715, 1600, 1517, 1457, 1371, 1274, 1069, 978, 783.

### Example 86

### N-(2-ethoxy-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.38 (s, 1H), 8.10 (s, 1H), 7.83 (s, 1H), 7.74 (d, *J* = 4.3 Hz, 1H), 7.52 (d, *J* = 1.1 Hz, 1H), 7.16 (d, *J* = 8.3 Hz, 2H), 7.03 (d, *J* = 7.7 Hz, 1H), 6.85 (s, 1H), 6.33 (dd, *J* = 3.1, 1.9 Hz, 1H), 6.17 (d, *J* = 4.4 Hz, 1H), 6.11 (d, *J* = 2.8 Hz, 1H), 4.47 (d, *J* = 4.6 Hz, 2H), 4.16 (q, *J* = 6.9 Hz, 2H), 3.84 (s, 3H), 3.17 - 3.05 (m, *J* = 9.5, 5.4 Hz, 4H), 1.40 (t, *J* = 6.9 Hz, 3H).TLC-MS (ESI) *m*/*z*: 443.4 [M+H]⁺; 465.4 [M+Na]⁺. HPLC *t_{R}* = 5.50 min. FT-IR(ATR) [cm⁻¹]: 3079, 2936, 2823, 2715, 1600, 1517, 1457, 1371, 1274, 1069, 978, 783.

### Example 89

### 2-(2-(furan-2-yl)ethyl)-N-(3-methyl-4-(1-methyl-1H-pyrazol-4-yl)benzyl)-3H-imidazo[4,5-b]pyridin-7-amine

¹H NMR (400 MHz, DMSO) δ 12.40 (s, 1H), 7.88 (s, 1H), 7.73 (d, *J* = 2.2 Hz, 1H), 7.61 (s, 1H), 7.52 (d, J= 1.1 Hz, 1H), 7.30 - 7.23 (m, 2H), 7.18 (d, *J* = 7.9 Hz, 1H), 7.13 (s, 1H), 6.33 (dd, *J* = 3.1, 1.9 Hz, 1H), 6.19 (d, *J* = 3.1 Hz, 1H), 6.12 (d, J= 2.8 Hz, 1H), 4.50 (d, J= 5.4 Hz, 2H), 3.86 (s, 3H), 3.17 - 3.06 (m, 4H), 2.32 (s, 3H). TLC-MS (ESI) *m*/*z*: 413.3 [M+H]⁺; 435.3 [M+Na]⁺. HPLC *t_{R}* = 5.08 min. FT-IR(ATR) [cm⁻¹]: 3233, 3032, 2931, 2640, 1628, 1439, 1321, 1144, 985, 890, 791.

### Example 90

### N-(3-(difluoromethoxy)-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.42 (s, 1H), 8.02 (s, 1H), 7.82 (s, 1H), 7.73 (d, *J* = 5.3 Hz, 1H), 7.61 (d, *J* = 7.9 Hz, 1H), 7.52 (s, 1H), 7.25 (dd, *J* = 16.0, 7.6 Hz, 3H), 7.18 - 6.97 (m, *J* = 74.0 Hz, 1H), 6.37 - 6.29 (m, 1H), 6.20 (d, *J* = 5.3 Hz, 1H), 6.11 (d, *J* = 2.8 Hz, 1H), 4.56 (d, *J* = 4.9 Hz, 2H), 3.87 (s, 3H), 3.18 - 3.03 (m, *J* = 12.9, 5.1 Hz, 4H). TLC-MS (ESI) *m*/*z*: 465.1 [M+H]⁺; 487.1 [M+Na]⁺. HPLC *t_{R}* = 5.18 min. FT-IR(ATR) [cm⁻¹]: 3435, 2871, 2713, 2642, 1604, 1330, 1126, 1098, 1049, 997, 795, 730.

### Example 98

### N-((3-fluoro-5-(1-methyl-1H-pyrazol-4-yl)pyridin-2-yl)methyl)-2-(2-(furan-2-yl)ethyl)-3H-imidazo[4,5-b]pyridin-7-amine

TLC-MS (ESI) *m*/*z*: 418.4 [M+H]⁺; 440.4 [M+Na]⁺. HPLC *t_{R}* = 4.20 min.

### Example 99

### N-(2,6-dimethoxy-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.37 (s, 1H), 8.21 (s, 1H), 7.93 (s, 1H), 7.81 (d, *J* = 4.2 Hz, 1H), 7.50 (d, *J* = 0.9 Hz, 1H), 6.87 (s, 2H), 6.47 (d, *J* = 5.6 Hz, 1H), 6.32 (dd, *J* = 3.0, 1.9 Hz, 1H), 6.10 (d, *J* = 3.0 Hz, 1H), 5.69 (s, 1H), 4.38 (d, *J* = 46.9 Hz, 2H), 3.88 (s, 6H), 3.86 (s, 3H), 3.13 - 3.02 (m, 4H). TLC-MS (ESI) *m*/*z*: 459.5 [M+H]⁺; 481.5 [M+Na]⁺. HPLC *t_{R}* = 5.63 min. FT-IR(ATR) [cm⁻¹]: 3431, 3078, 2936, 2841, 1608, 1579, 1452, 1360, 1343, 1126, 1008, 847, 796, 726.

### Example 100

### N-(2-fluoro-6-methoxy-4-(1-methyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.40 (s, 1H), 8.23 (s, 1H), 7.95 (s, 1H), 7.80 (d, *J* = 5.3 Hz, 1H), 7.50 (s, 1H), 7.13 - 7.01 (m, 2H), 6.41 (d, *J* = 5.4 Hz, 1H), 6.32 (dd, *J* = 2.9, 2.0 Hz, 1H), 6.18 (t, *J* = 5.2 Hz, 1H), 6.10 (d, *J* = 3.0 Hz, 1H), 4.51 (d, *J* = 4.2 Hz, 2H), 3.92 (s, 3H), 3.85 (s, 3H), 3.14 - 3.02 (m, *J* = 14.1, 5.3 Hz, 4H). TLC-MS (ESI) *m*/*z*: 447.3 [M+H]⁺; 469.3 [M+Na]⁺. HPLC *t_{R}* = 5.48 min. FT-IR(ATR) [cm⁻¹]: 3234, 3141, 3004, 2935, 2843, 1617, 1451, 1339, 1278, 1102, 1010, 832, 794, 731.

### Shown as an example for Synthesis in Synthetic scheme D

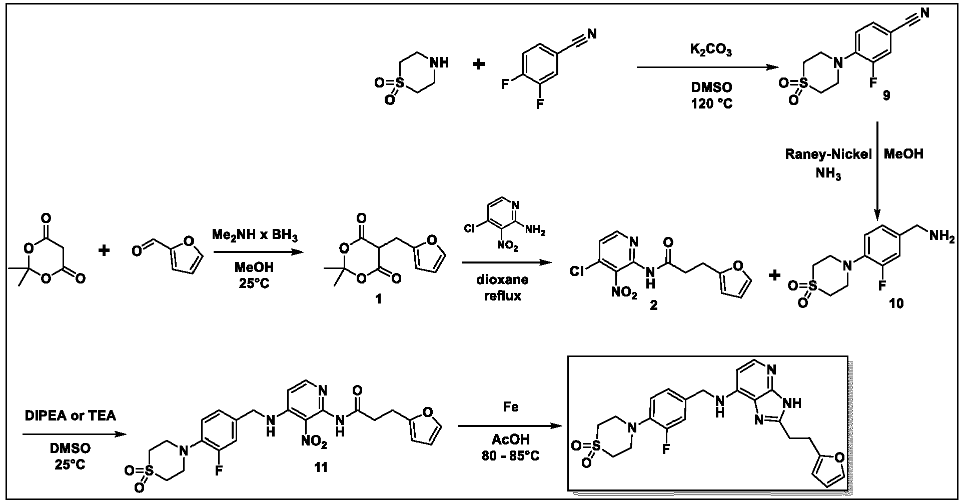

### 4-(1,1-dioxidothiomorpholino)-3-fluorobenzonitrile [9]

3,4-difluorobenzonitrile (1.00 eq.) was suspended in DMSO (2.00 ml/mmol) together with the thiomorpholine 1,1-dioxide (1.10 eq.) and potassium carbonate (2.00 eq.) and the mixture was heated at 120°C for two hours with intensive stirring until the reaction control showed complete conversion of the reactants. The mixture was then cooled to room temperature and added to ice water and the resulting solid was isolated by filtration. The product was obtained as a white solid and used without further purification.

¹H NMR (400 MHz, DMSO) δ 7.76 (dd, *J* = 13.3, 1.9 Hz, 1H), 7.59 (dd, *J* = 8.5, 1.7 Hz, 1H), 7.27 (t, *J* = 8.8 Hz, 1H), 3.69 (dd, *J* = 6.2, 4.0 Hz, 4H), 3.30 - 3.24 (m, 4H). TLC-MS (ESI) *m*/*z*: n.d.. HPLC *t_{R}* = 3.40 min.

### 4-(4-(aminomethyl)-2-fluorophenyl)thiomorpholine 1,1-dioxide [10]

An aqueous suspension of Raney nickel was added to a solution of *9* in 7N ammonia in methanol (10.00 ml/mmol). The reaction flask was then first flooded with nitrogen, followed by bubbling the solution with hydrogen for five minutes. The mixture was stirred under a hydrogen atmosphere for 16 h at room temperature until the reaction control showed complete conversion of the starting material. The hydrogen was then removed, the Raney nickel separated over diatomaceous earth and rinsed several times with methanol. The organic phase was then removed under reduced pressure and the resulting solid (partly also oily liquid) was used without further purification.

HPLC *t_{R}* = 1.19 min (100 %)

### N-(4-((4-(1,1-dioxidothiomorpholino)-3-fluorobenzyl)amino)-3-nitropyridin-2-yl)-3-(furan-2-yl)propanamide [11]

To a mixture of 2(1.00 eq.) and *10* (1.05 eq.) in dimethyl sulfoxide (6.00 ml/mmol) was added DIPEA (2.00 eq.). The mixture was stirred at room temperature overnight until complete consumption of the starting materials. The mixture was diluted with methanol followed by addition of aqueous methanol (60% v/v) to induce precipitation of the product. The product was and dried at room temperature under vacuum to obtain the product as a yellow solid that was used without further purification.

¹H NMR (400 MHz, DMSO) δ 10.68 (s, 1H), 7.95 (dd, *J* = 10.2, 6.1 Hz, 2H), 7.50 (d, *J* = 1.1 Hz, 1H), 7.18 (d, *J* = 13.5 Hz, 1H), 7.12 (t, *J* = 7.7 Hz, 2H), 6.62 (d, *J* = 6.1 Hz, 1H), 6.34 (dd, *J* = 3.0, 1.7 Hz, 1H), 6.08 (dd, *J* = 10.3, 2.8 Hz, 1H), 4.47 (d, *J* = 6.1 Hz, 2H), 3.46 (d, *J* = 3.3 Hz, 4H), 3.24 (d, *J* = 4.1 Hz, 4H), 2.86 - 2.82 (m, 2H), 2.64 (t, *J* = 7.5 Hz, 2H). TLC-MS (ESI) *m*/*z*: 539.6 [M+Na]⁺. HPLC *t_{R}* = 5.88 min.

### Example 11

### 4-(2-fluoro-4-(((2-(2-(furan-2-yl)ethyl)-3H-imidazo[4,5-b]pyridin-7-yl)amino)methyl)phenyl) thiomorpholine 1,1-dioxide

To a solution of 11 (1.00 eq.) in a mixture of acetic acid and water (2.5 : 1; 7 ml/mmol acetic acid) was added powdered iron (3.00 eq.). The mixture was stirred at 85°C for 2 h until complete consumption of the starting material. After complete conversion of the reaction, the batch was cooled down to room temperature, diluted with water and NaOH-solution and filtered. The product was extracted from the clear mixture with ethyl acetate and washed with brine. The organic phase was dried over Na₂SO₄, filtered, and evaporated to dryness. After purification via column-chromatography (from 100% DCM to DCM/MeOH 9:1) the desired product was obtained as a white solid.

¹H NMR (400 MHz, DMSO) δ 12.41 (s, 1H), 7.73 (d, *J* = 5.0 Hz, 1H), 7.52 (d, *J* = 0.8 Hz, 1H), 7.26 - 7.04 (m, 4H), 6.33 (dd, *J* = 2.8, 1.9 Hz, 1H), 6.18 (d, *J* = 5.1 Hz, 1H), 6.11 (d, *J* = 2.8 Hz, 1H), 4.49 (d, *J* = 5.0 Hz, 2H), 3.44 (s, 4H), 3.23 (s, 4H), 3.10 (dd, *J* = 12.6, 4.9 Hz, 4H). TLC-MS (ESI) *m*/*z*: 491.8 [M+Na]⁺. HPLC *t_{R}* = 3.63 min. FT-IR(ATR) [cm⁻¹]: 3404, 2979, 2919, 2839, 2799, 2731, 2686, 1620, 1513, 1432, 1391, 1337, 1305, 1274, 1230, 1182, 1142, 1114, 1044, 1017, 986, 890, 868, 821, 797, 725.

### The following examples were prepared using the procedures above:

### Example 12

### N-(4-(4,4-difluoropiperidin-1-yl)-3-fluorobenzyl)-2-(2-(furan-2-yl)ethyl)-3H-imidazo[4,5-b]pyridin-7-amine

¹H NMR (400 MHz, DMSO) δ 12.41 (s, 1H), 7.73 (d, *J* = 5.0 Hz, 1H), 7.56 - 7.47 (m, 1H), 7.13 (t, *J* = 10.8 Hz, 3H), 7.03 (t, *J* = 8.5 Hz, 1H), 6.33 (dd, *J* = 3.1, 1.9 Hz, 1H), 6.18 (d, *J* = 5.3 Hz, 1H), 6.11 (d, *J* = 2.7 Hz, 1H), 4.48 (d, *J* = 5.7 Hz, 2H), 3.16 - 3.05 (m, 8H), 2.08 (ddd, *J* = 19.7, 13.9, 5.6 Hz, 4H). TLC-MS (ESI) *m*/*z*: 478.3 [M+Na]⁺. HPLC *t_{R}* = 6.55 min. FT-IR(ATR) [cm⁻¹]: 3237, 3024, 2973, 2936, 2836, 2717, 2655, 2378, 1631, 1582, 1551, 1508, 1428, 1364, 1333, 1279, 1247, 1124, 1100, 1073, 1035, 1012, 984, 935, 890, 790, 731.

### Example 14

### N-(3-fluoro-4-morpholinobenzyl)-2-(2-(furan-2-yl)ethyl)-3H-imidazo[4,5-b]pyridin-7-amine

¹H NMR (400 MHz, DMSO) δ 12.40 (s, 1H), 7.73 (d, *J* = 3.9 Hz, 1H), 7.51 (s, 1H), 7.22 - 7.07 (m, 3H), 6.96 (t, *J* = 8.4 Hz, 1H), 6.33 (s, 1H), 6.18 (d, *J* = 4.5 Hz, 1H), 6.11 (s, 1H), 4.48 (d, *J* = 3.8 Hz, 2H), 3.71 (s, 4H), 3.11 (dd, *J* = 12.6, 4.8 Hz, 4H), 2.94 (s, 4H). TLC-MS (ESI) *m*/*z*: 422.4 [M+H]⁺; 444.4 [M+Na]⁺. HPLC *t_{R}* = 4.77 min. FT-IR(ATR) [cm⁻¹]: 3081, 3004, 2950, 2932, 2915, 2889, 2855, 2818, 2721, 2693, 1617, 1508, 1448, 1430, 1391, 1333, 1279, 1246, 1215, 1145, 1115, 1047, 996, 917, 891, 850, 827, 790, 772, 745.

### Example 21

### N-(4-(3,3-difluoropyrrolidin-1-yl)-3-fluorobenzyl)-2-(2-(furan-2-yl)ethyl)-3H-imidazo[4,5-b]pyridin-7-amine

¹H NMR (400 MHz, DMSO) δ 12.40 (s, 1H), 7.73 (s, 1H), 7.52 (s, 1H), 7.11 (dd, *J* = 17.2, 11.5 Hz, 3H), 6.76 (t, *J* = 8.7 Hz, 1H), 6.33 (s, 1H), 6.18 (d, *J* = 4.0 Hz, 1H), 6.11 (d, *J* = 2.1 Hz, 1H), 4.45 (d, *J* = 4.9 Hz, 2H), 3.66 (t, *J* = 13.4 Hz, 2H), 3.45 (t, *J* = 6.8 Hz, 2H), 3.11 (dd, *J* = 12.7, 4.9 Hz, 4H), 2.43 (td, *J* = 14.5, 7.3 Hz, 2H). TLC-MS (ESI) *m*/*z*: 442.3 [M+H]⁺; 464.4 [M+Na]⁺. HPLC *t_{R}* = 6.53 min. FT-IR(ATR) [cm⁻¹]: 3233, 3011, 2958, 2932, 2839, 2732, 2658, 2607, 2555, 1631, 1584, 1517, 1438, 1333, 1276, 1232, 1193, 1117, 1073, 1013, 995, 923, 891, 790, 729, 697.

### Example 55

### N-(4-(3,3-difluoroazetidin-1-yl)-3-fluorobenzyl)-2-(2-(furan-2-yl)ethyl)-3H-imidazo[4,5-b]pyridin-7-amine

¹H NMR (400 MHz, DMSO) δ 12.41 (s, 1H), 7.74 (d, *J* = 3.1 Hz, 1H), 7.51 (s, 1H), 7.17 - 7.07 (m, *J* = 14.5, 10.9 Hz, 3H), 6.63 (t, *J* = 8.8 Hz, 1H), 6.33 (s, 1H), 6.20 (d, *J* = 3.4 Hz, 1H), 6.11 (d, *J* = 2.7 Hz, 1H), 4.46 (d, *J* = 5.9 Hz, 2H), 4.29 (t, *J* = 12.1 Hz, 4H), 3.16 - 3.06 (m, *J* = 16.1, 5.5 Hz, 4H). TLC-MS (ESI) *m*/*z*: 428.0 [M+H]⁺; 450.1 [M+Na]⁺. HPLC *t_{R}* = 5.91 min. FT-IR(ATR) [cm⁻¹]: 3242, 2940, 2851, 2650, 1630, 1585, 1517, 1440, 1364, 1228, 1196, 889, 791, 731.

### Example 69

### N-(3-fluoro-4-(2-oxa-6-azaspiro[3.3]heptan-6-yl)benzyl)-2-(2-(furan-2-yl)ethyl)-3H-imidazo[4,5-b]pyridin-7-amine

¹H NMR (600 MHz, DMSO) δ 12.38 (s, 1H), 7.72 (s, 1H), 7.51 (s, 1H), 7.12 - 6.98 (m, *J* = 10.1 Hz, 3H), 6.47 (t, *J* = 8.7 Hz, 1H), 6.33 (s, 1H), 6.18 (s, 1H), 6.11 (d, J= 2.4 Hz, 1H), 4.68 (s, 4H), 4.41 (d, *J* = 3.1 Hz, 2H), 4.00 (s, 4H), 3.14 - 3.05 (m, 4H). TLC-MS (ESI) *m*/*z*: 434.3 [M+H]⁺; 456.3 [M+Na]⁺. HPLC *t_{R}* = 4.53 min. FT-IR(ATR) [cm⁻¹]: 3079, 2936, 2823, 2715, 1600, 1517, 1457, 1371, 1274, 1069, 978, 783.

### Example 84

### 2-(2-(furan-2-yl)ethyl)-N-(4-morpholinobenzyl)-3H-imidazo[4,5-b]pyridin-7-amine

¹H NMR (400 MHz, DMSO) δ 12.44 (s, 1H), 7.74 (d, *J* = 2.7 Hz, 1H), 7.51 (d, *J* = 1.0 Hz, 1H), 7.24 (d, J= 8.5 Hz, 2H), 7.04 (s, 1H), 6.88 (d, *J* = 8.5 Hz, 2H), 6.33 (dd, *J* = 3.0, 1.9 Hz, 1H), 6.22 (d, *J* = 1.2 Hz, 1H), 6.11 (d, *J* = 3.0 Hz, 1H), 4.44 (d, *J* = 5.8 Hz, 2H), 3.74 - 3.68 (m, 4H), 3.16 - 3.07 (m, *J* = 12.4, 6.0 Hz, 4H), 3.06 - 3.02 (m, 4H). TLC-MS (ESI) *m*/*z*: 404.3 [M+H]⁺; 426.3 [M+Na]⁺. HPLC *t_{R}* = 4.24 min. FT-IR(ATR) [cm⁻¹]: 3079, 2936, 2823, 2715, 1600, 1517, 1457, 1371, 1274, 1069, 978, 783.

### Example 84

### N-(3-fluoro-4-(3-methoxypyrrolidin-1-yl)benzyl)-2-(2-(furan-2-yl)ethyl)-3H-imidazo[4,5-b]pyridin-7-amine

¹H NMR (400 MHz, DMSO) δ 12.40 (s, 1H), 7.73 (s, 1H), 7.52 (s, 1H), 7.05 (d, *J* = 14.9 Hz, 3H), 6.65 (s, 1H), 6.33 (s, 1H), 6.15 (d, *J* = 31.6 Hz, 2H), 4.41 (s, 2H), 3.28 (d, *J* = 25.4 Hz, 8H), 3.10 (d, *J* = 7.0 Hz, 4H), 1.97 (s, 1H), 1.61 (s, 1H). TLC-MS (ESI) *m*/*z*: 404.3 [M+H]⁺; 426.3 [M+Na]⁺. HPLC *t_{R}* = 6.20 min. FT-IR(ATR) [cm⁻¹]: 3079, 2936, 2823, 2715, 1600, 1517, 1457, 1371, 1274, 1069, 978, 783.

### Example 92

### (R)-N-(3-fluoro-4-(2-methylmorpholino)benzyl)-2-(2-(furan-2-yl)ethyl)-3H-imidazo[4,5-b]pyridin-7-amine

¹H NMR (400 MHz, DMSO) δ 12.40 (s, 1H), 7.74 (d, *J* = 4.4 Hz, 1H), 7.52 (t, *J* = 3.0 Hz, 1H), 7.22 - 7.09 (m, 3H), 6.95 (t, *J* = 8.7 Hz, 1H), 6.33 (dd, *J* = 2.9, 1.9 Hz, 1H), 6.19 (d, *J* = 5.2 Hz, 1H), 6.11 (d, *J* = 2.8 Hz, 1H), 4.48 (d, *J* = 5.8 Hz, 2H), 3.83 (d, *J* = 10.1 Hz, 1H), 3.69 - 3.60 (m, 2H), 3.20 - 3.06 (m, 6H), 2.68 (td, *J* = 11.5, 3.0 Hz, 1H), 2.42 - 2.34 (m, 1H), 1.09 (d, *J* = 6.2 Hz, 3H). TLC-MS (ESI) *m*/*z*: 436.3 [M+H]⁺; 458.3 [M+Na]⁺. HPLC *t_{R} =* 5.36 min. FT-IR(ATR) [cm⁻¹]: 3230, 2972, 2933, 2853, 2650, 1628, 1507, 1437, 1332, 1245, 1146, 1107, 1074, 890, 792, 730.

### Example 93

### N-(4-((1S,4S)-2-oxa-5-azabicyclo[2.2.1]heptan-5-yl)-3-fluorobenzyl)-2-(2-(furan-2-yl)ethyl)-3H-imidazo[4,5-b]pyridin-7-amine

¹H NMR (400 MHz, DMSO) δ 12.38 (s, 1H), 7.73 (d, *J* = 5.1 Hz, 1H), 7.58 - 7.45 (m, 1H), 7.05 (dd, *J* = 21.3, 11.0 Hz, 3H), 6.72 (t, *J* = 8.9 Hz, 1H), 6.33 (dd, *J* = 3.1, 1.9 Hz, 1H), 6.21 (d, *J* = 5.3 Hz, 1H), 6.11 (d, *J* = 3.1 Hz, 1H), 4.52 (s, 1H), 4.49 - 4.36 (m, *J* = 6.7 Hz, 3H), 3.76 (d, *J* = 7.6 Hz, 1H), 3.71 (d, *J* = 7.5 Hz, 1H), 3.57 (dd, *J* = 9.6, 2.7 Hz, 1H), 3.10 (pd, *J* = 9.4, 5.5 Hz, 4H), 3.00 (dd, *J* = 9.7, 3.3 Hz, 1H), 1.87 (dd, *J* = 9.6, 1.6 Hz, 1H), 1.78 (d, *J* = 9.6 Hz, 1H). TLC-MS (ESI) *m*/*z*: 434.3 [M+H]⁺; 456.2 [M+Na]⁺. HPLC *t_{R}* = 4.97 min. FT-IR(ATR) [cm⁻¹]: 3237, 2934, 2871, 2749, 2655, 1627, 1512, 1427, 1326, 1226, 1145, 884, 849, 793, 738.

### Example 94

### N-(4-(8-oxa-3-azabicyclo[3.2.1]octan-3-yl)-3-fluorobenzyl)-2-(2-(furan-2-yl)ethyl)-3H-imidazo[4,5-b]pyridin-7-amine

¹H NMR (400 MHz, DMSO) δ 12.40 (s, 1H), 7.72 (d, *J* = 5.4 Hz, 1H), 7.51 (d, *J* = 1.0 Hz, 1H), 7.18 - 7.06 (m, J= 16.0, 6.7 Hz, 3H), 6.87 (t, *J* = 8.7 Hz, 1H), 6.33 (dd, *J* = 3.0, 1.9 Hz, 1H), 6.17 (d, *J* = 5.4 Hz, 1H), 6.11 (d, *J* = 2.9 Hz, 1H), 4.46 (d, *J* = 5.2 Hz, 2H), 4.32 (s, 2H), 3.16 - 3.06 (m, 4H), 3.02 (d, *J* = 10.9 Hz, 2H), 2.86 (d, *J* = 10.4 Hz, 2H), 1.96 - 1.88 (m, 2H), 1.84 - 1.75 (m, 2H). TLC-MS (ESI) *m*/*z*: 448.3 [M+H]⁺; 470.3 [M+Na]⁺. HPLC *t_{R}* = 6.01 min. FT-IR(ATR) [cm⁻¹]: 3235, 2948, 2824, 2659, 1617, 1507, 1334, 1244, 1153, 1067, 994, 884, 792, 725.

### Example 95

### N-(4-(azetidin-1-yl)-3-fluorobenzyl)-2-(2-(furan-2-yl)ethyl)-3H-imidazo[4,5-b]pyridin-7-amine

¹H NMR (400 MHz, DMSO) δ 12.34 (s, 1H), 7.74 (s, 1H), 7.51 (s, 1H), 7.15 - 6.96 (m, 3H), 6.45 (t, *J* = 8.7 Hz, 1H), 6.33 (s, 1H), 6.19 (d, *J* = 4.5 Hz, 1H), 6.11 (d, *J* = 2.5 Hz, 1H), 4.41 (d, *J* = 5.0 Hz, 2H), 3.82 (t, *J* = 6.4 Hz, 4H), 3.17 - 3.03 (m, *J* = 12.8, 5.1 Hz, 4H), 2.31 - 2.16 (m, 2H). TLC-MS (ESI) *m*/*z*: 392.1 [M+H]⁺; 414.1 [M+Na]⁺. HPLC *t_{R}* = 5.49 min. FT-IR(ATR) [cm⁻¹]: 3238, 2963, 2925, 2848, 2657, 1628, 1507, 1440, 1332, 1224, 888, 791, 729.

### Example 97

### N-(3-fluoro-4-(tetrahydro-1H-furo[3,4-c]pyrrol-5(3H)-yl)benzyl)-2-(2-(furan-2-yl)ethyl)-3H-imidazo[4,5-b]pyridin-7-amine

¹H NMR (400 MHz, DMSO) δ 12.39 (s, 1H), 7.72 (d, *J* = 5.3 Hz, 1H), 7.51 (s, 1H), 7.13 - 7.01 (m, *J* = 15.6, 7.7 Hz, 3H), 6.76 (t, *J* = 8.6 Hz, 1H), 6.35 - 6.31 (m, 1H), 6.17 (d, *J* = 5.4 Hz, 1H), 6.11 (d, *J* = 2.9 Hz, 1H), 4.44 (d, *J* = 5.1 Hz, 2H), 3.85 - 3.79 (m, 2H), 3.46 (dd, *J* = 8.6, 3.3 Hz, 2H), 3.26 - 3.21 (m, 2H), 3.14 - 3.04 (m, *J* = 14.7, 6.3 Hz, 6H), 2.87 (d, *J* = 2.4 Hz, 2H). TLC-MS (ESI) *m*/*z*: 448.2 [M+H]⁺; 470.2 [M+Na]⁺. HPLC *t_{R}* = 5.22 min. FT-IR(ATR) [cm⁻¹]: 3233, 2939, 2843, 2654, 1628, 1513, 1427, 1321, 1225, 884, 790, 732.

### Example 6: Synthesis of example compounds according to the invention with the Suzuki reaction

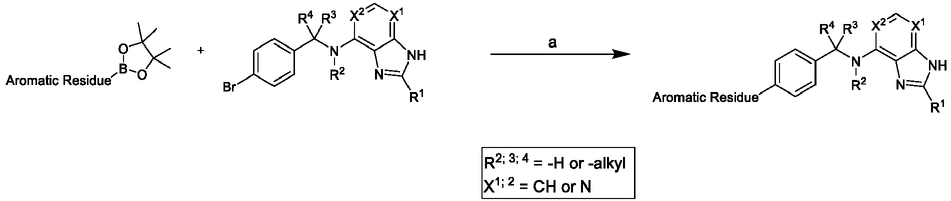

^{a}Reagents and conditions: (a) XPhos Pd G₄, K₂CO₃, Dioxan, H₂O, 80 - 90°C.

### Suzuki-Reaction (a):

The mixture of the substituted aryl halide (75 - 100 mg), the respective boronic acid pinacol ester (1.10 eq.), K₂CO₃ (3.00 eq.) and XPhos Pd G4 (0.01 eq.) in 2 ml of *1,4*-Dioxan and 0.5 ml of water was stirred at 80 - 90°C overnight until complete consumption of the starting materials. Afterwards the mixture was cooled to room temperature, diluted with water and the product was extracted with ethyl acetate and washed with brine. The organic phase was dried over Na₂SO₄, filtered, and evaporated to dryness. After purification via column-chromatography (from 100% DCM to DCM/MeOH 9:1) the desired product was obtained as a white solid.

### Shown as an example for Synthesis in Synthetic scheme E

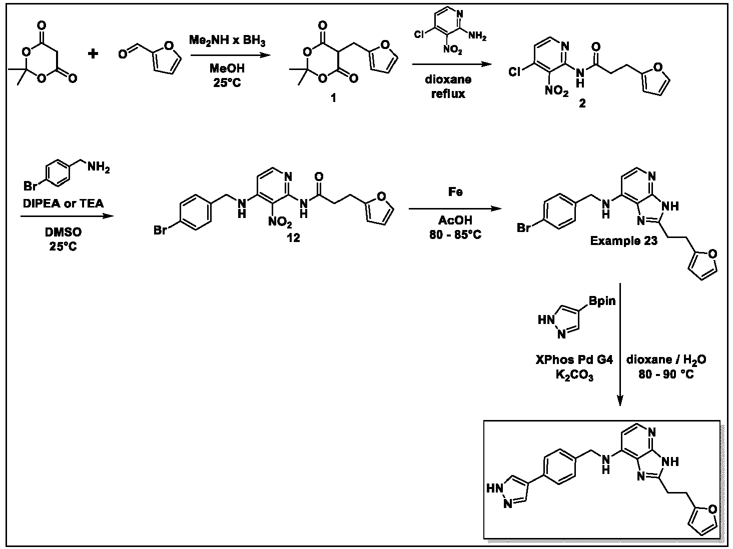

### N-(4-((4-Bromobenzyl)amino)-3-nitropyridin-2-yl)-3-(furan-2-yl)propanamid [12]

To a mixture of 2(1.00 eq.) and 4-bromobenzylamine (1.05 eq.) in dimethyl sulfoxide (6.00 ml/mmol) was added DIPEA (2.00 eq.). The mixture was stirred at room temperature overnight until complete consumption of the starting materials. The mixture was diluted with methanol followed by addition of aqueous methanol (60% v/v) to induce precipitation of the product. The product was and dried at room temperature under vacuum to obtain the product as a yellow solid that was used without further purification.

¹H NMR (400 MHz, DMSO) δ 10.67 (s, 1H), 8.01 - 7.93 (m, 2H), 7.51 (dd, *J* = 13.9, 4.7 Hz, 3H), 7.30 (d, *J* = 8.4 Hz, 2H), 6.55 (d, *J* = 6.1 Hz, 1H), 6.34 (dd, *J* = 3.0, 1.9 Hz, 1H), 6.10 (d, *J* = 2.5 Hz, 1H), 4.51 (d, *J* = 6.1 Hz, 2H), 2.85 (t, *J* = 7.5 Hz, 2H), 2.65 (t, *J* = 7.5 Hz, 2H). TLC-MS (ESI) *m*/*z*: 467.4 [M+Na]⁺. HPLC *t_{R}* = 7.79 min

### Example 23

### N-(4-bromobenzyl)-2-(2-(furan-2-yl)ethyl)-3H-imidazo[4,5-b]pyridin-7-amine

To a solution of 12 (1.00 eq.) in a mixture of acetic acid and water (2.5 : 1; 7 ml/mmol acetic acid) was added powdered iron (3.00 eq.). The mixture was stirred at 85°C for 2 h until complete consumption of the starting material. After complete conversion of the reaction, the batch was cooled down to room temperature, diluted with water and NaOH-solution and filtered. The product was extracted from the clear mixture with ethyl acetate and washed with brine. The organic phase was dried over Na₂SO₄, filtered, and evaporated to dryness. After purification via column-chromatography (from 100% DCM to DCM/MeOH 9:1) the desired product was obtained as a white solid.

¹H NMR (400 MHz, DMSO) δ 12.41 (s, 1H), 7.72 (s, 1H), 7.57 - 7.43 (m, 3H), 7.32 (d, *J* = 7.7 Hz, 2H), 7.22 (s, 1H), 6.33 (s, 1H), 6.20 - 6.05 (m, 2H), 4.52 (d, *J* = 3.4 Hz, 2H), 3.18 - 3.03 (m, *J* = 7.3 Hz, 4H). TLC-MS (ESI) *m*/*z*: 398.0 [M+H]⁺; 420.0 [M+Na]⁺. HPLC *t_{R}* = 6.23 min. FT-IR(ATR) [cm⁻¹]: 3228, 3024, 2974, 2925, 2640, 1718, 1629, 1582, 1550, 1522, 1485, 1436, 1420, 1405, 1377, 1332, 1278, 1259, 1249, 1144, 1131, 1069, 1010, 891, 791, 730.

### Example 28

### N-(4-(1H-pyrazol-4-yl)benzyl)-2-(2-(furan-2-yl)ethyl)-3H-imidazo[4,5-b]pyridin-7-amine

The mixture of *Example 23* (1.00 eq.), 4-(4,4,5,5-tetramethyl-1,3,2-dioxaborolan-2-yl)-1*H*-pyrazole (1.10 eq.), K₂CO₃ (3.00 eq.) and XPhos Pd G4 (0.005 eq.) in *1,4*-Dioxane / water (4+1; 2.85 ml/mmol of Dioxane) was stirred under nitrogen-atmosphere at 80 - 90°C overnight until complete consumption of the starting materials was observed. The mixture was cooled to room temperature, diluted dropwise with MeOH / H₂O (40/60) under constant stirring and cooled in an ice bath until complete precipitation of the product was observed. The precipitate was collected by filtration, washed with MeOH / H₂O (40/60) and dried. After purification via column-chromatography (from 100% DCM to DCM/MeOH 9:1) the desired product was obtained as a white solid.

¹H NMR (400 MHz, DMSO) δ 12.66 (d, *J* = 153.2 Hz, 2H), 8.00 (s, 2H), 7.72 (d, *J* = 4.9 Hz, 1H), 7.53 (d, *J* = 7.8 Hz, 3H), 7.34 (d, *J* = 7.8 Hz, 2H), 7.15 (s, 1H), 6.33 (dd, *J* = 2.9, 1.9 Hz, 1H), 6.19 (d, *J* = 5.0 Hz, 1H), 6.12 (d, *J* = 2.8 Hz, 1H), 4.53 (d, *J* = 4.0 Hz, 2H), 3.17 - 3.06 (m, *J* = 13.1, 5.2 Hz, 4H). TLC-MS (ESI) *m*/*z*: 385.6 [M+H]⁺; 407.7 [M+Na]⁺. HPLC *t_{R}* = 4.15 min. FT-IR(ATR) [cm⁻¹]: 2923, 2857, 2782, 2714, 1607, 1522, 1457, 1334, 1280, 1146, 995, 932, 889, 856, 792, 727.

### The following examples were prepared using the procedures above:

### Example 29

### N-(4-(1,3-dimethyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.41 (s, 1H), 7.81 (s, 1H), 7.73 (d, *J* = 3.8 Hz, 1H), 7.52 (s, 1H), 7.35 (q, *J* = 8.0 Hz, 4H), 7.16 (s, 1H), 6.33 (dd, *J* = 2.7, 2.0 Hz, 1H), 6.21 (d, *J* = 3.9 Hz, 1H), 6.12 (d, *J* = 2.8 Hz, 1H), 4.55 (d, *J* = 3.7 Hz, 2H), 3.76 (s, 3H), 3.16 - 3.06 (m, *J* = 12.5, 5.0 Hz, 4H), 2.26 (s, 3H). TLC-MS (ESI) *m*/*z*: 413.8 [M+H]⁺; 435.8 [M+Na]⁺. HPLC *t_{R}* = 5.17 min. FT-IR(ATR) [cm⁻¹]: 3233, 3181, 3100, 3080, 3023, 2923, 2856, 2820, 2712, 2660, 2551, 1624, 1584, 1551, 1522, 1507, 1438, 1415, 1336, 1296, 1278, 1145, 1004, 887, 793, 731, 651.

### Example 35

### 2-(2-(furan-2-yl)ethyl)-N-(4-(pyrimidin-5-yl)benzyl)-3H-imidazo[4,5-b]pyridin-7-amine

¹H NMR (400 MHz, DMSO) δ 12.42 (s, 1H), 9.16 (s, 1H), 9.11 (s, 2H), 7.76 (s, 1H), 7.74 (s, 2H), 7.54 (s, 1H), 7.52 (s, 2H), 7.28 (s, 1H), 6.33 (dd, *J* = 3.0, 1.9 Hz, 1H), 6.20 (d, *J* = 5.1 Hz, 1H), 6.12 (d, *J* = 2.9 Hz, 1H), 4.63 (d, J = 5.8 Hz, 2H), 3.15 (dd, *J* = 10.9, 5.1 Hz, 2H), 3.12 - 3.07 (m, 2H). TLC-MS (ESI) *m*/*z*: 397.3 [M+H]⁺; 419.3 [M+Na]⁺. HPLC *t_{R}* = 4.10 min. FT-IR(ATR) [cm⁻¹]: 3280, 3146, 3074, 3010, 2919, 2850, 2790, 2742, 2723, 2705, 2564, 2548, 1604, 1554, 1523, 1416, 1394, 1331, 1303, 1278, 1217, 1148, 1003, 984, 885, 788, 768, 723.

### Example 36

### 2-(2-(furan-2-yl)ethyl)-N-(4-(1-methyl-1H-pyrazol-3-yl)benzyl)-3H-imidazo[4,5-b]pyridin-7-amine

¹H NMR (400 MHz, DMSO) δ 12.40 (s, 1H), 7.70 (d, J= 7.9 Hz, 4H), 7.52 (s, 1H), 7.37 (d, J= 7.8 Hz, 2H), 7.17 (s, 1H), 6.62 (s, 1H), 6.33 (d, *J* = 1.7 Hz, 1H), 6.19 (d, *J* = 5.0 Hz, 1H), 6.12 (d, *J* = 2.8 Hz, 1H), 4.56 (d, *J* = 4.2 Hz, 2H), 3.86 (s, 3H), 3.11 (dd, *J* = 13.6, 5.5 Hz, 4H). TLC-MS (ESI) *m*/*z*: 399.7 [M+H]⁺; 421.8 [M+Na]⁺. HPLC *t_{R}* = 4.70 min. FT-IR(ATR) [cm⁻¹]: 3397, 3069, 2933, 2920, 2853, 2700, 1602, 1506, 1450, 1430, 1394, 1333, 1278, 1231, 1138, 997, 883, 799, 754, 732.

### Example 37

### 2-(2-(furan-2-yl)ethyl)-N-(4-(1-methyl-1H-pyrazol-5-yl)benzyl)-3H-imidazo[4,5-b]pyridin-7-amine

¹H NMR (400 MHz, DMSO) δ 12.42 (s, 1H), 7.74 (d, *J* = 5.5 Hz, 1H), 7.53 - 7.51 (m, 1H), 7.50 - 7.43 (m, 5H), 7.25 (t, *J* = 6.2 Hz, 1H), 6.35 (d, *J* = 1.7 Hz, 1H), 6.33 (dd, *J* = 3.1, 1.9 Hz, 1H), 6.21 (d, *J* = 5.6 Hz, 1H), 6.12 (d, *J* = 2.9 Hz, 1H), 4.62 (d, *J* = 5.8 Hz, 2H), 3.82 (s, 3H), 3.17 - 3.06 (m, 4H). TLC-MS (ESI) *m*/*z*: 399.8 [M+H]⁺; 421.8 [M+Na]⁺. HPLC *t_{R}* = 4.78 min. FT-IR(ATR) [cm⁻¹]: 3229, 3017, 2928, 2848, 2643, 1628, 1584, 1545, 1436, 1379, 1336, 1271, 1144, 1070, 978, 923, 891, 790, 774, 732.

### Example 38

### 2-(2-(furan-2-yl)ethyl)-N-(4-(2-methylthiazol-5-yl)benzyl)-3H-imidazo[4,5-b]pyridin-7-amine

¹H NMR (400 MHz, DMSO) δ 12.41 (s, 1H), 7.96 (s, 1H), 7.72 (d, *J* = 5.3 Hz, 1H), 7.57 - 7.50 (m, 3H), 7.41 (d, *J* = 8.0 Hz, 2H), 7.22 (t, *J* = 5.9 Hz, 1H), 6.36 - 6.31 (m, 1H), 6.17 (d, *J* = 5.4 Hz, 1H), 6.11 (d, *J* = 2.9 Hz, 1H), 4.57 (d, *J* = 5.3 Hz, 2H), 3.11 (dd, *J* = 13.7, 5.7 Hz, 4H), 2.65 (s, 3H). TLC-MS (ESI) *m*/*z*: 416.0 [M+H]⁺; 438.0 [M+Na]⁺. HPLC *t_{R}* = 5.56 min. FT-IR(ATR) [cm⁻¹]: 3226, 3022, 2918, 2848, 2643, 2115, 1627, 1584, 1436, 1336, 1295, 1278, 1166, 1144, 1071, 1011, 997, 890, 792, 731.

### Example 39

### N-(4-(1-ethyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.41 (s, 1H), 8.11 (s, 1H), 7.81 (s, 1H), 7.73 (s, 1H), 7.55 - 7.44 (m, 3H), 7.34 (d, *J* = 6.6 Hz, 2H), 7.14 (s, 1H), 6.33 (s, 1H), 6.20 (s, 1H), 6.12 (s, 1H), 4.53 (s, 2H), 4.12 (d, *J* = 6.7 Hz, 2H), 3.18 - 3.04 (m, *J* = 7.5 Hz, 4H), 1.38 (t, *J* = 6.1 Hz, 3H). TLC-MS (ESI) *m*/*z*: 413.1 [M+H]⁺; 435.1 [M+Na]⁺. HPLC *t_{R} =* 4.92 min. FT-IR(ATR) [cm⁻¹]: 3232, 3024, 2983, 2939, 2647, 1628, 1585, 1570, 1437, 1336, 1293, 1146, 1073, 996, 953, 891, 793, 734.

### Example 40

### 2-(2-(furan-2-yl)ethyl)-N-(4-(1-isopropyl-1H-pyrazol-4-yl)benzyl)-3H-imidazo[4,5-b]pyridin-7-amine

¹H NMR (400 MHz, DMSO) δ 12.40 (s, 1H), 8.15 (s, 1H), 7.80 (s, 1H), 7.73 (s, 1H), 7.58 - 7.43 (m, *J* = 7.7 Hz, 3H), 7.34 (d, *J* = 6.9 Hz, 2H), 7.13 (s, 1H), 6.33 (s, 1H), 6.20 (s, 1H), 6.12 (s, 1H), 4.61 - 4.39 (m, *J* = 15.7, 8.7 Hz, 3H), 3.11 (d, *J* = 7.3 Hz, 4H), 1.43 (d, *J* = 5.9 Hz, 6H). TLC-MS (ESI) *m*/*z*: 427.1 [M+H]⁺; 449.1 [M+Na]⁺. HPLC *t_{R}* = 5.30 min. FT-IR(ATR) [cm⁻¹]: 3231, 3022, 2974, 2933, 2647, 1628, 1436, 1364, 1334, 1293, 1262, 1218, 1191, 1181, 1146, 984, 954, 890, 792, 730.

### Example 41

### N-(4-(1-cyclopropyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.40 (s, 1H), 8.16 (s, 1H), 7.80 (s, 1H), 7.73 (s, 1H), 7.57 - 7.44 (m, *J* = 9.1 Hz, 3H), 7.34 (d, *J* = 6.8 Hz, 2H), 7.13 (s, 1H), 6.33 (s, 1H), 6.20 (s, 1H), 6.12 (s, 1H), 4.52 (d, *J* = 1.7 Hz, 2H), 3.71 (s, 1H), 3.11 (d, *J* = 7.1 Hz, 4H), 1.05 (s, 2H), 0.96 (d, *J* = 4.6 Hz, 2H). TLC-MS (ESI) *m*/*z*: 425.1 [M+H]⁺; 447.1 [M+Na]⁺. HPLC *t_{R}* = 5.10 min. FT-IR(ATR) [cm⁻¹]: 3229, 3023, 2942, 2642, 1628, 1584, 1436, 1334, 1293, 1146, 985, 891, 792, 732.

### Example 42

### 2-(2-(furan-2-yl)ethyl)-N-(4-(1-(oxetan-3-yl)-1H-pyrazol-4-yl)benzyl)-3H-imidazo[4,5-b]pyridin-7-amine

¹H NMR (400 MHz, DMSO) δ 12.41 (s, 1H), 8.30 (s, 1H), 7.98 (s, 1H), 7.73 (d, J= 4.0 Hz, 1H), 7.56 - 7.50 (m, *J* = 4.5 Hz, 3H), 7.36 (d, *J* = 8.1 Hz, 2H), 7.15 (s, 1H), 6.33 (dd, *J* = 3.1, 1.9 Hz, 1H), 6.20 (d, *J* = 4.9 Hz, 1H), 6.12 (dd, *J* = 3.1, 0.5 Hz, 1H), 5.60 - 5.53 (m, 1H), 4.92 (dd, *J* = 7.0, 2.4 Hz, 4H), 4.54 (d, *J* = 5.5 Hz, 2H), 3.17 - 3.12 (m, 2H), 3.11 - 3.06 (m, 2H). TLC-MS (ESI) *m*/*z*: 441.4 [M+H]⁺; 463.4 [M+Na]⁺. HPLC *t_{R}* = 4.36 min. FT-IR(ATR) [cm⁻¹]: 3236, 3219, 3019, 2947, 2878, 2718, 2645, 2553, 1629, 1584, 1438, 1379, 1333, 1294, 1273, 1145, 976, 953, 892, 823, 792, 731, 699.

### Example 43

### 2-(2-(furan-2-yl)ethyl)-N-methyl-N-(4-(1-methyl-1H-pyrazol-4-yl)benzyl)-3H-imidazo[4,5-b]pyridin-7-amine

¹H NMR (400 MHz, DMSO) δ 12.51 (s, 1H), 8.05 (s, 1H), 7.82 (d, *J* = 5.7 Hz, 1H), 7.79 (s, 1H), 7.49 (d, *J* = 1.0 Hz, 1H), 7.46 (d, *J* = 8.1 Hz, 2H), 7.20 (d, *J* = 8.1 Hz, 2H), 6.29 (dd, *J* = 3.0, 1.9 Hz, 1H), 6.26 (d, *J* = 5.8 Hz, 1H), 6.06 (d, *J* = 2.9 Hz, 1H), 5.35 (s, 2H), 3.84 (s, 3H), 3.13 - 3.05 (m, 7H). TLC-MS (ESI) *m*/*z*: 413.4 [M+H]⁺; 435.4 [M+Na]⁺. HPLC *t_{R}* = 5.49 min. FT-IR(ATR) [cm⁻¹]: 2978, 2926, 2902, 2877, 2846, 2810, 2740, 2705, 1592, 1521, 1449, 1370, 1283, 1071, 986, 953, 923, 876, 829, 797, 744.

### Example 44

### 2-(2-(furan-2-yl)ethyl)-7-(2-(4-(1-methyl-1H-pyrazol-4-yl)phenyl)pyrrolidin-1-yl)-3H-imidazo[4,5-b]pyridine

¹H NMR (400 MHz, DMSO) δ 12.35 (s, 1H), 8.04 (s, 1H), 7.78 (s, 1H), 7.67 (d, *J* = 3.5 Hz, 1H), 7.51 (s, 1H), 7.45 (d, *J* = 7.4 Hz, 2H), 7.19 (d, *J* = 7.2 Hz, 2H), 6.30 (s, 1H), 6.05 (s, 1H), 5.97 (s, 1H), 5.41 (s, 1H), 4.30 (s, 1H), 4.01 (s, 1H), 3.84 (s, 3H), 3.06 (d, *J* = 13.8 Hz, 4H), 2.44 - 2.33 (m, 1H), 1.97 (s, 2H), 1.85 (d, *J* = 5.8 Hz, 1H). TLC-MS (ESI) *m*/*z*: 439.6 [M+H]⁺; 461.7 [M+Na]⁺. HPLC *t_{R}* = 5.28 min. FT-IR(ATR) [cm⁻¹]: 2940, 2904, 2853, 1597, 1507, 1453, 1370, 1272, 954, 834, 790, 732.

### Example 60

### N-(4-(1,5-dimethyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.40 (s, 1H), 7.73 (d, *J* = 5.5 Hz, 1H), 7.51 (s, 2H), 7.38 (d, *J* = 8.0 Hz, 2H), 7.31 (d, *J* = 8.1 Hz, 2H), 7.17 (t, *J* = 6.3 Hz, 1H), 6.33 (dd, *J* = 2.8, 2.0 Hz, 1H), 6.21 (d, *J* = 5.6 Hz, 1H), 6.12 (d, *J* = 2.9 Hz, 1H), 4.56 (d, *J* = 5.7 Hz, 2H), 3.76 (s, 3H), 3.16 - 3.06 (m, 4H), 2.33 (s, 3H). TLC-MS (ESI) *m*/*z*: 412.9 [M+H]⁺; 434.8 [M+Na]⁺. HPLC *t_{R}* = 5.02 min. FT-IR(ATR) [cm⁻¹]: 3235, 2937, 2648, 1630, 1585, 1550, 1440, 1336, 1146, 1013, 892, 790, 748.

### Example 61

### 2-(2-(furan-2-yl)ethyl)-N-(4-(3-methyl-1H-pyrazol-4-yl)benzyl)-3H-imidazo[4,5-b]pyridin-7-amine

¹H NMR (400 MHz, DMSO) δ 12.49 (d, *J* = 48.3 Hz, 2H), 7.82 - 7.60 (m, *J* = 5.5 Hz, 2H), 7.52 (d, *J* = 1.1 Hz, 1H), 7.37 (s, 4H), 7.16 (t, *J* = 6.3 Hz, 1H), 6.33 (dd, *J* = 2.8, 2.0 Hz, 1H), 6.20 (d, *J* = 5.6 Hz, 1H), 6.12 (d, *J* = 3.0 Hz, 1H), 4.55 (d, *J* = 5.9 Hz, 2H), 3.17 - 3.05 (m, 4H), 2.33 (s, 3H). TLC-MS (ESI) *m*/*z*: 398.8 [M+H]⁺; 420.8 [M+Na]⁺. HPLC *t_{R}* = 4.39 min. FT-IR(ATR) [cm⁻¹]: 3205, 2855, 1606, 1521, 1394, 1335, 1276, 1149, 929, 883, 861, 820, 793, 728.

### Example 62

### 2-(2-(furan-2-yl)ethyl)-N-(4-(1-methyl-3-(trifluoromethyl)-1H-pyrazol-4-yl)benzyl)-3H-imidazo[4,5-b]pyridin-7-amine

¹H NMR (400 MHz, DMSO) δ 12.41 (s, 1H), 8.10 (s, 1H), 7.74 (d, *J* = 3.7 Hz, 1H), 7.51 (d, *J* = 1.0 Hz, 1H), 7.42 (d, *J* = 8.0 Hz, 2H), 7.33 (d, *J* = 7.8 Hz, 2H), 7.20 (s, 1H), 6.33 (dd, *J* = 3.0, 1.9 Hz, 1H), 6.21 (d, *J* = 4.2 Hz, 1H), 6.12 (d, *J* = 2.9 Hz, 1H), 4.59 (d, *J* = 4.9 Hz, 2H), 3.93 (s, 3H), 3.17 - 3.06 (m, 4H). TLC-MS (ESI) *m*/*z*: 467.0 [M+H]⁺; 489.0 [M+Na]⁺. HPLC *t_{R}* = 5.73 min. FT-IR(ATR) [cm⁻¹]: 2996, 2666, 2592, 1628, 1437, 1285, 1174, 1118, 984, 889, 793, 734.

### Example 63

### N-(4-(1-propyl-1H-pyrazol-4-yl)benzyl)-2-(2-(furan-2-yl)ethyl)-3H-imidazo[4,5-b]pyridin-7-amine

¹H NMR (400 MHz, DMSO) δ 12.40 (s, 1H), 8.10 (s, 1H), 7.81 (s, 1H), 7.72 (d, *J* = 4.5 Hz, 1H), 7.55 - 7.46 (m, 3H), 7.34 (d, *J* = 8.1 Hz, 2H), 7.14 (s, 1H), 6.33 (dd, *J* = 3.1, 1.9 Hz, 1H), 6.18 (d, *J* = 4.7 Hz, 1H), 6.12 (dd, *J* = 3.1, 0.5 Hz, 1H), 4.53 (d, *J* = 5.3 Hz, 2H), 4.05 (t, *J* = 7.0 Hz, 2H), 3.16 - 3.06 (m, 4H), 1.84 - 1.74 (m, 2H), 0.83 (t, *J* = 7.4 Hz, 3H). TLC-MS (ESI) *m*/*z*: 427.0 [M+H]⁺; 449.0 [M+Na]⁺. HPLC *t_{R}* = 4.87 min. FT-IR(ATR) [cm⁻¹]: 3225, 3024, 2965, 2935, 2648, 1628, 1585, 1437, 1336, 1293, 1146, 987, 954, 890, 792, 730.

### Example 64

### 2-(4-(4-(((2-(2-(furan-2-yl)ethyl)-3H-imidazo[4,5-b]pyridin-7-yl)amino)methyl)phenyl)-1H-pyrazol-1-yl)ethan-1-ol

¹H NMR (400 MHz, DMSO) δ 12.40 (s, 1H), 8.08 (s, 1H), 7.82 (s, 1H), 7.72 (d, *J* = 3.3 Hz, 1H), 7.51 (dd, *J* = 9.8, 4.5 Hz, 3H), 7.34 (d, *J* = 8.1 Hz, 2H), 7.14 (s, 1H), 6.33 (dd, *J* = 3.1, 1.9 Hz, 1H), 6.19 (d, *J* = 4.3 Hz, 1H), 6.12 (d, *J* = 2.8 Hz, 1H), 4.91 (t, *J* = 5.2 Hz, 1H), 4.53 (d, J= 5.3 Hz, 2H), 4.13 (t, J= 5.6 Hz, 2H), 3.75 (q, *J* = 5.4 Hz, 2H), 3.17 - 3.06 (m, 4H). TLC-MS (ESI) *m*/*z*: 429.1 [M+H]⁺; 451.2 [M+Na]⁺. HPLC *t_{R}* = 3.75 min. FT-IR(ATR) [cm⁻¹]: 3238, 3093, 3019, 2945, 2719, 1616, 1436, 1366, 1334, 1276, 1224, 1186, 1145, 1060, 998, 891, 793, 732.

### Example 65

### 2-(2-(furan-2-yl)ethyl)-N-(4-(1-(2-methoxyethyl)-1H-pyrazol-4-yl)benzyl)-3H-imidazo[4,5-b]pyridin-7-amine

¹H NMR (400 MHz, DMSO) δ 12.40 (s, 1H), 8.08 (s, 1H), 7.83 (s, 1H), 7.73 (s, 1H), 7.54 - 7.45 (m, 3H), 7.34 (d, *J* = 7.9 Hz, 2H), 7.14 (s, 1H), 6.36 - 6.31 (m, 1H), 6.19 (s, 1H), 6.12 (d, *J* = 2.9 Hz, 1H), 4.53 (d, *J* = 5.3 Hz, 2H), 4.25 (t, *J* = 5.2 Hz, 2H), 3.69 (t, *J* = 5.2 Hz, 2H), 3.22 (s, 3H), 3.17 - 3.05 (m, 4H). TLC-MS (ESI) *m*/*z*: 442.9 [M+H]⁺; 464.9 [M+Na]⁺. HPLC *t_{R}* = 4.67 min. FT-IR(ATR) [cm⁻¹]: 3215, 3026, 2923, 2849, 2638, 1628, 1437, 1336, 1293, 1145, 1118, 1072, 1011, 891, 792, 732.

### Example 66

### 2-(2-(furan-2-yl)ethyl)-N-(4-(1-(2-morpholinoethyl)-1H-pyrazol-4-yl)benzyl)-3H-imidazo[4,5-b]pyridin-7-amine

¹H NMR (400 MHz, DMSO) δ 12.40 (s, 1H), 8.12 (s, 1H), 7.81 (s, 1H), 7.72 (d, *J* = 5.1 Hz, 1H), 7.53 - 7.45 (m, 3H), 7.34 (d, *J* = 8.0 Hz, 2H), 7.14 (s, 1H), 6.33 (dd, *J* = 3.1, 1.9 Hz, 1H), 6.18 (d, *J* = 5.2 Hz, 1H), 6.12 (d, *J* = 3.1 Hz, 1H), 4.53 (d, *J* = 5.1 Hz, 2H), 4.21 (t, *J* = 6.6 Hz, 2H), 3.55 - 3.51 (m, 4H), 3.17 - 3.06 (m, 4H), 2.71 (t, *J* = 6.6 Hz, 2H), 2.43 - 2.36 (m, 4H). TLC-MS (ESI) *m*/*z*: 498.1 [M+H]⁺; 520.0 [M+Na]⁺. HPLC *t_{R}* = 2.25 min. FT-IR(ATR) [cm⁻¹]: 3230, 2938, 2853, 2807, 1623, 1437, 1336, 1293, 1114, 888, 790, 730.

### Example 67

### 2-(2-(furan-2-yl)ethyl)-N-(4-(1-methyl-1H-pyrazol-4-yl)phenethyl)-3H-imidazo[4,5-b]pyridin-7-amine

¹H NMR (400 MHz, DMSO) δ 12.38 (s, 1H), 8.08 (s, 1H), 7.86 - 7.77 (m, 2H), 7.53 - 7.50 (m, 1H), 7.48 (d, *J* = 8.1 Hz, 2H), 7.27 (d, *J* = 8.1 Hz, 2H), 6.48 (s, 1H), 6.32 (dd, *J* = 3.1, 1.9 Hz, 2H), 6.11 (d, *J* = 2.7 Hz, 1H), 3.85 (s, 3H), 3.62 - 3.52 (m, 2H), 3.15 - 3.04 (m, 4H), 2.88 (t, *J* = 7.4 Hz, 2H). TLC-MS (ESI) *m*/*z*: 412.9 [M+H]⁺; 434.9 [M+Na]⁺. HPLC *t_{R}* = 4.86 min. FT-IR(ATR) [cm⁻¹]: 3237, 2929, 2643, 1630, 1576, 1440, 1336, 1282, 1144, 987, 892, 789, 732.

### Example 68

### 2-(2-(furan-2-yl)ethyl)-7-(5-(1-methyl-1H-pyrazol-4-yl)isoindolin-2-yl)-3H-imidazo[4,5-b]pyridine

¹H NMR (400 MHz, DMSO) δ 12.50 (s, 1H), 8.14 (s, 1H), 7.89 (s, 1H), 7.65 (s, 1H), 7.52 (d, *J* = 6.2 Hz, 1H), 7.42 (d, J= 7.7 Hz, 1H), 6.34 (s, 1H), 6.26 (d, *J* = 4.9 Hz, 1H), 6.16 (s, 1H), 5.07 (d, *J* = 17.1 Hz, 1H), 3.87 (s, 1H), 3.22 - 3.07 (m, *J* = 5.7 Hz, 4H). TLC-MS (ESI) *m*/*z*: 411.0 [M+H]⁺; 433.0 [M+Na]⁺. HPLC *t_{R}* = 5.44 min. FT-IR(ATR) [cm⁻¹]: 3079, 2936, 2823, 2715, 1600, 1517, 1457, 1371, 1274, 1069, 978, 783.

### Example 74

### 2-(2-(furan-2-yl)ethyl)-N-((5-(1-methyl-1H-pyrazol-4-yl)thiophen-2-yl)methyl)-3H-imidazo[4,5-b]pyridin-7-amine

¹H NMR (600 MHz, DMSO) δ 12.42 (s, 1H), 7.92 (s, 1H), 7.78 (s, 1H), 7.61 (s, 1H), 7.51 (d, *J* = 1.0 Hz, 1H), 7.13 (s, 1H), 6.96 (s, 2H), 6.33 (dd, *J* = 3.1, 1.9 Hz, 2H), 6.11 (d, *J* = 3.0 Hz, 1H), 4.72 (d, *J* = 3.9 Hz, 2H), 3.80 (s, 3H), 3.15 - 3.12 (m, 2H), 3.11 - 3.06 (m, 2H). TLC-MS (ESI) *m*/*z*: 405.1 [M+H]⁺; 427.1 [M+Na]⁺. HPLC *t_{R}* = 4.61 min. FT-IR(ATR) [cm⁻¹]: 3079, 2936, 2823, 2715, 1600, 1517, 1457, 1371, 1274, 1069, 978, 783.

### Example 75

### (S)-2-(2-(furan-2-yl)ethyl)-N-(1-(4-(1-methyl-1H-pyrazol-4-yl)phenyl)ethyl)-3H-imidazo[4,5-b]pyridin-7-amine

¹H NMR (600 MHz, DMSO) δ 12.39 (s, 1H), 8.04 (s, 1H), 7.78 (s, 1H), 7.68 (d, *J* = 5.4 Hz, 1H), 7.52 (s, 1H), 7.46 (d, *J* = 7.9 Hz, 2H), 7.40 (d, *J* = 8.0 Hz, 2H), 6.78 (d, *J* = 7.7 Hz, 1H), 6.33 (s, 1H), 6.17 - 6.09 (m, 2H), 4.84 (d, *J* = 143.5 Hz, 1H), 3.83 (s, 3H), 3.12 (dt, *J* = 13.9, 6.4 Hz, 4H), 1.53 (d, *J* = 6.8 Hz, 3H). TLC-MS (ESI) *m*/*z*: 412.8 [M+H]⁺; 434.8 [M+Na]⁺. HPLC *t_{R}* = 4.95 min. FT FT-IR(ATR) [cm⁻¹]: 3079, 2936, 2823, 2715, 1600, 1517, 1457, 1371, 1274, 1069, 978, 783.

### Example 76

### (R)-2-(2-(furan-2-yl)ethyl)-N-(1-(4-(1-methyl-1H-pyrazol-4-yl)phenyl)ethyl)-3H-imidazo[4,5-b]pyridin-7-amine

¹H NMR (600 MHz, DMSO) δ 12.39 (s, 1H), 8.04 (s, 1H), 7.78 (s, 1H), 7.68 (d, *J* = 4.6 Hz, 1H), 7.52 (d, *J* = 0.8 Hz, 1H), 7.46 (d, *J* = 7.9 Hz, 2H), 7.40 (d, *J* = 7.9 Hz, 2H), 6.78 (d, *J* = 6.9 Hz, 1H), 6.34 (dd, *J* = 2.7, 2.0 Hz, 1H), 6.13 (dd, *J* = 10.5, 4.0 Hz, 2H), 4.96 (s, 1H), 3.83 (s, 3H), 3.16 - 3.13 (m, 2H), 3.12 - 3.06 (m, *J* = 7.2 Hz, 2H), 1.53 (d, *J* = 6.8 Hz, 3H). TLC-MS (ESI) *m*/*z*: 413.3 [M+H]⁺; 435.3 [M+Na]⁺. HPLC *t_{R}* = 4.98 min. FT-IR(ATR) [cm⁻¹]: 3079, 2936, 2823, 2715, 1600, 1517, 1457, 1371, 1274, 1069, 978, 783.

### Example 79

### N-(4-(3,5-dimethylisoxazol-4-yl)benzyl)-2-(2-(furan-2-yl)ethyl)-3H-imidazo[4,5-b]pyridin-7-amine

¹H NMR (400 MHz, DMSO) δ = 12.41 (s, 1H), 7.78 - 7.72 (m, 1H), 7.54 - 7.50 (m, 1H), 7.49 - 7.43 (m, 2H), 7.35 - 7.29 (m, 2H), 7.19 (s, 1H), 6.36 - 6.30 (m, 1H), 6.25 (d, *J*=5.6, 1H), 6.11 (d, *J*=3.2, 1H), 4.61 (d, *J*=6.4, 2H), 3.18 - 3.12 (m, 2H), 3.12 - 3.03 (m, 2H), 2.37 (s, 3H), 2.19 (s, 3H).TLC-MS (ESI) *m*/*z*: 414.6 [M+H]⁺; 436.6 [M+Na]⁺. HPLC *t_{R}* = 5.12 min. FT-IR(ATR) [cm⁻¹]: 3079, 2936, 2823, 2715, 1600, 1517, 1457, 1371, 1274, 1069, 978, 783.

### Example 91

### 2-(2-(furan-2-yl)ethyl)-N-(4-(1-methyl-1H-pyrrol-3-yl)benzyl)-3H-imidazo[4,5-b]pyridin-7-amine

¹H NMR (400 MHz, DMSO) δ 12.39 (s, 1H), 7.72 (d, *J* = 4.5 Hz, 1H), 7.52 (d, *J* = 1.1 Hz, 1H), 7.41 (d, *J* = 7.8 Hz, 2H), 7.29 (d, *J* = 7.9 Hz, 2H), 7.10 (s, 2H), 6.70 (t, *J* = 2.3 Hz, 1H), 6.33 (dd, *J* = 3.1, 1.9 Hz, 2H), 6.19 (d, *J* = 4.5 Hz, 1H), 6.12 (d, *J* = 2.8 Hz, 1H), 4.50 (d, J= 4.3 Hz, 2H), 3.61 (s, 3H), 3.16 - 3.06 (m, 4H). TLC-MS (ESI) *m*/*z*: 398.2 [M+H]⁺; 420.2 [M+Na]⁺. HPLC *t_{R}* = 5.75 min. FT-IR(ATR) [cm⁻¹]: 3233, 3021, 2918, 2657, 1628, 1436, 1333, 1144, 890, 792, 770, 731.

## Claims

1. **A compound** of formula (I) 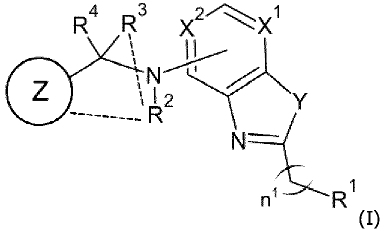 or solvates, salts, N-oxides, racemic mixtures, diastereomers, enantiomers, tautomers, isotopically labeled forms, and combinations thereof; wherein:
X¹ and X² are each independently selected from C or N;
Y is selected from the group consisting of NH, N-alkyl, O, S;
n¹ is any natural number selected from 0, 1, 2, 3, and preferably is 2;
R¹ is selected from the group consisting of substituted or unsubstituted homocyclic ring, substituted or
unsubstituted heterocyclic ring, alkyl, and alkoxy;
R² is selected from H, alkyl and alkylene group that is also covalently bonded to any of Z, R³ or R⁴;
R³ is selected from H, alkyl;
R⁴ is selected from H, alkyl, and
Z constitutes one or more unsubstituted or substituted aromatic ring:

2. The compound of claim 1, wherein Z is selected from 4-(methyl-1*H*-pyrazol-4-yl)naphthalen-1-yl,5-(methyl-1*H*-pyrazol-4-yl)furan-2-yl, 5-(methyl-1*H*-pyrazol-4-yl) thiophene-2-yl.

3. The compound of claim 1, wherein Z has the formula (ZI), 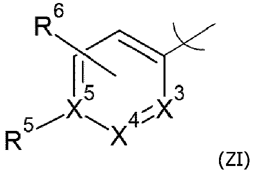
wherein X³, X⁴ and X⁵ are each independently selected from C or N; and wherein if X⁵ is N, R⁵ is dispensed with, and if X⁵ is C, R⁵ is selected from H, halogen, particularly Br; Cl and F ; C₁₋₃-alkyl, in particular isoprop-2-yl; C₁₋₃-alkoxy, in particular methoxy-1-yl; diC₁₋₃-alkylamine group, in particular N,N-dimethylamin-1-yl; N,N-(Dimethy(*d*₃))amin-1-yl; diC₁₋₃-alkyl phosphine oxide, in particular dimethylphosphine oxide, halogen substituted sulfane, in particular pentafluoro-λ6-sulfane;
substituted and/or unsubstituted five-membered hetero and/or homocycles, in particular substituted and/or unsubstituted pyrrole, particularly, 1-methyl-1*H*-pyrrole-3-yl; hexahydro-1*H*-furo[3,4-*c*]pyrrole-1-yl,substituted and/or unsubstituted Oxazole, in particular 3,5-dimethylisoxazol-4-yl; substituted and/or unsubstituted imidazole, in particular 1-methyl-1*H*-imidazol-4-yl; 4-methyl-1*H*-imidazol-1-yl; substituted and/or unsubstituted pyrazole, in particular 1-cyclopropyl-1*H*-pyrazol-4-yl; 1,3-dimethy-1*H*-pyrazol-4-yl; 1,5-dimethyl-1*H*-pyrazole-4-yl; 1,3,5-trimethyl-1*H*-pyrazol-4-yl; 1-difluormethyl-1*H*-pyrazol-4-yl; 1-ethyl-1*H-*pyrazol-4-yl; 1-(2-(morpholin-4-yl)-ethyl)-1*H*-pyrazol-4-yl; 1*H*-pyrazol-4-yl; 1-isopropyl-1*H*-pyrazol-4-yl; 1-(methyl-(*d*₃))-1*H*-pyrazol-4-yl; 1-methyl-1*H*-pyrazol-3-yl; 1-methyl-1*H*-pyrazol-4-yl; 1-methyl-1*H*-pyrazol-5-yl; 1-(methylslfonyl)-1*H*-pyrazol-4-yl; 1-(oxetan-3-yl)-1*H*-pyrazol-4-yl; 1-(tetrahydro-2*H*-pyran-4-yl)-1*H*-pyrazol-4-yl; 1-(trifluoromethyl)-1*H*-pyrazol-4-yl; 3,5-dimethyl-1*H*-pyrazol-4-yl;3-methyl-1*H*-pyrazol-4-yl; 1-methyl-3-(trifluoromethyl)-1*H*-pyrazole-4-yl; 1-propyl-1*H*-pyrazole-4-yl; 2-(ethan-1-ol)-1*H*-pyrazol-4-yl; 1-(2-methoxyethyl)-1*H*-pyrazole-4-yl; 4-methyl-1*H*-pyrazol-1-yl; substituted and/or unsubstituted Triazole, in particular 1-methyl-1,2,4-1*H*-triazol-3-yl; 1-methyl-1,2,3-1*H*-triazol-4-yl; substituted and/or unsubstituted Thiazole, in particular 2-Methylthiazol-5-yl; substituted and/or unsubstituted Pyrrolidine, in particular, 3,3-difluoropyrrolidin-1-yl; 3-methoxypyrrolidin-1-yl;
substituted and/or unsubstituted six-membered hetero and/or homocycles, particularly substituted and/or unsubstituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or unsubstituted pyrimidine, in particular, pyrimidin-5-yl; substituted and/or unsubstituted morpholine, in particular, thiomorpholin-1,1-dioxid-4-yl; (2*R*,6*S*)-dimethylmorpholin-4-yl; (2*S*,6*S*)-dimethylmorpholin-4-yl; (3*R*,5*S*)-dimethylmorpholin-4-yl; (3*S*,5*S*)-dimethylmorpholin-4-yl; (*R*)-2-methylmorpholin-4yl; (*R*)-3-methylmorpholin-4-yl; (*S*)-2-methylmorpholin-4-yl; (*S*)-3-methylmorpholin-4-yl; 2,2-dimethylmorpholin-4-yl; 3,3-dimethylmorpholin-4-yl; morpholin-4-yl; substituted and/or unsubstituted piperidine, in particular 4,4-difluoropiperidin-1-yl;
substituted and/or unsubstituted four-membered hetero and/or homocycles, in particular substituted and/or unsubstituted azetidine, particularly, 3,3-difluoroazetidin-1-yl; 3-dimethylaminoazetidin-1-yl; 3-fluoroazetidin-1-yl; azetidin-1-yl; and
substituted and/or unsubstituted hetero and/or homo bicycles, in particular (1*S*,4*S*)-2-oxa-5-azabicyclo[2.2.1]heptan-5-yl; (1*R*, 4*R*)-2-oxa-5-azabicyclo[2.2.1]heptan-5-yl;3-oxa-8-azabicyclo[3.2.1]octan-8-yl; 8-oxa-3-azabicyclo[3.2.1]octan-3-yl; 2-oxa-6-azaspiro[3.3]heptan-6-yl; 1*H*-pyrrolo[2,3-*b*]pyridin-4-yl; 2-methyl-2*H*-indazol-5-yl; pyrazolo[1,5-*a*]pyridin-3-yl; quinolin-4-yl;
R⁶ is selected from H, halogen, in particular Br, Cl, F; unsubstituted or substituted C₁₋₃-alkoxy, in particular methoxy, ethoxy, OCF₃, OCHF₂; methylsulfonyl, dimethylphosphine oxide; and unsubstituted or substituted C₁₋₃-alkyl, in particular methyl or CF₃.

4. The compound of claim 1, wherein Z has the formula (ZII) 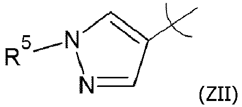 wherein R⁵ is selected from H and C₁₋₃-alkyl, in particular methyl.

5. The compound of any one of claims 1 to 4, wherein (i): R², R³ and/or R⁴ are H; and/or (ii) Y is NH, X¹ is N and X² is C.

6. The compound of any one of claims 1 to 5, wherein R¹ is selected from benzofuran-5-yl, benzofuran-6-yl, cyclopropyl, cyclobutyl, furan-2-yl, furan-3-yl, 5-methylfuran-2-yl, 5-phenylfuran-2-yl, phenoxy, phenyl, tetrahydrofuran-3-yl, thiophen-2-yl, thiophen-3-yl, isoprop-2-yl and methoxy.

7. The compound of any one of claims 1 to 6, wherein the compound has the formula (II) 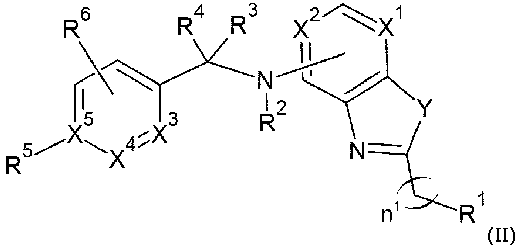 Wherein
X¹, X², X³, X⁴ and X⁵ are each individually selected from C or N atom;
Y is selected from the group consisting of NH, N-alkyl, unsubstituted or substituted C₁₋₆-alkyl or cycloalkyl, O, S;
n¹ is any number selected from 0, 1, 2, 3;
R¹ is selected from the group consisting of benzofuran-5-yl, benzofuran-6-yl, cyclopropyl, cyclobutyl, furan-2-yl, furan-3-yl, 5-methylfuran-2-yl, 5-phenylfuran-2-yl, phenoxy, phenyl, tetrahydrofuran-3-yl, thiophen-2-yl, thiophen-3-yl, isoprop-2-yl and methoxy;
R² is selected from H, alkyl, in particular methyl;
R³ is selected from H, alkyl;
R⁴ is selected from H, alkyl;
if X⁵ = N, R⁵ is dispensed with;
and if X⁵ is C, R⁵ is selected from H, halogen, particularly Br; Cl and F; C₁₋₃-alkyl, in particular isoprop-2-yl; C₁₋₃-alkoxy, in particular methoxy-1-yl; diC₁₋₃-alkylamine group, in particular N,N-dimethylamin-1-yl; N,N-(Dimethy(*d*₃))amin-1-yl; diC₁₋₃-alkyl phosphine oxide, in particular dimethylphosphine oxide, halogen substituted sulfane, in particular pentafluoro-λ6-sulfane;
substituted and/or unsubstituted five-membered hetero and/or homocycles, in particular substituted and/or unsubstituted pyrrole, particularly, 1-methyl-1*H*-pyrrole-3-yl; hexahydro-1*H*-furo[3,4-*c*]pyrrole-1-yl,substituted and/or unsubstituted oxazole, in particular 3,5-dimethylisoxazol-4-yl; substituted and/or unsubstituted imidazole, in particular 1-methyl-1*H*-imidazol-4-yl; 4-methyl-1*H*-imidazol-1-yl; substituted and/or unsubstituted pyrazole, in particular 1-cyclopropyl-1*H*-pyrazol-4-yl; 1,3-dimethy-1*H*-pyrazol-4-yl; 1,5-dimethyl-1*H*-yrazole-4-yl; 1,3,5-trimethyl-1*H*-pyrazol-4-yl; 1-difluormethyl-1*H*-pyrazol-4-yl; 1-ethyl-1*H-*pyrazol-4-yl; 1-(2-(morpholin-4-yl)-ethyl)-1*H*-pyrazol-4-yl; 1*H*-pyrazol-4-yl; 1-isopropyl-1*H*-pyrazol-4-yl; 1-(methyl-(*d*₃))-1*H*-pyrazol-4-yl; 1-methyl-1*H*-pyrazol- 3-yl; 1-methyl-1*H*-pyrazol-4-yl; 1-methyl-1*H*-pyrazol-5-yl; 1-(methylsulfonyl)-1*H*-pyrazol-4-yl; 1-(oxetan-3-yl)-1*H*-pyrazol-4-yl; 1-(tetrahydro-2*H*-pyran-4-yl)-1*H*-pyrazol-4-yl; 1-(trifluoromethyl)-1*H*-pyrazol-4-yl; 3,5-dimethyl-1*H*-pyrazol-4-yl;3-methyl-1*H*-pyrazol-4-yl; 1-methyl-3-(trifluoromethyl)-1*H*-pyrazole-4-yl; 1-propyl-1*H*-pyrazole-4-yl; 2-(ethan-1-ol)-1*H*-pyrazol-4-yl; 1-(2-methoxyethyl)-1*H*-pyrazole-4-yl; 4-methyl-1*H*-pyrazol-1-yl; substituted and/or unsubstituted Triazole, in particular 1-methyl-1,2,4-1*H*-triazol-3-yl; 1-methyl-1,2,3-1*H*-triazol-4-yl; substituted and/or unsubstituted Thiazole, in particular 2-Methylthiazol-5-yl; substituted and/or unsubstituted Pyrrolidine, in particular, 3,3-difluoropyrrolidin-1-yl; 3-methoxypyrrolidin-1-yl;
substituted and/or unsubstituted six-membered hetero and/or homocycles, particularly substituted and/or unsubstituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or unsubstituted pyrimidine, in particular, pyrimidin-5-yl; substituted and/or unsubstituted morpholine, in particular, thiomorpholin-1,1-dioxid-4-yl; (2*R*,6*S*)-dimethylmorpholin-4-yl; (2*S*,6*S*)-dimethylmorpholin-4-yl; (3*R*,5*S*)-dimethylmorpholin-4-yl; (3*S*,5*S*)-dimethylmorpholin-4-yl; (*R*)-2-methylmorpholin-4yl; (*R*)-3-methylmorpholin-4-yl; (*S*)-2-methylmorpholin-4-yl; (*S*)-3-methylmorpholin-4-yl; 2,2-dimethylmorpholin-4-yl; 3,3-dimethylmorpholin-4-yl; morpholin-4-yl; substituted and/or unsubstituted piperidine, in particular 4,4-difluoropiperidin-1-yl;
substituted and/or unsubstituted four-membered hetero and/or homocycles, in particular substituted and/or unsubstituted azetidine, particularly, 3,3-difluoroazetidin-1-yl; 3-dimethylaminoazetidin-1-yl; 3-fluoroazetidin-1-yl; azetidin-1-yl; and
substituted and/or unsubstituted hetero and/or homo bicycles, in particular (1*S*,4*S*)-2-oxa-5-azabicyclo[2.2.1]heptan-5-yl; (1*R*, 4*R*)-2-oxa-5-azabicyclo[2.2.1]heptan-5-y1;3-oxa-8-azabicyclo[3.2.1]octan-8-yl; 8-oxa-3-azabicyclo[3.2.1]octan-3-yl; 2-oxa-6-azaspiro[3.3]heptan-6-yl; 1*H*-pyrrolo[2,3-*b*]pyridin-4-yl;2-methyl-2*H*-indazol-5-yl; pyrazolo[1,5-*a*]pyridin-3-yl; quinolin-4-yl;
R⁶ is selected from H, halogen, in particular Br, Cl, F; unsubstituted or substituted C₁₋₃-alkoxy, in particular methoxy, ethoxy, OCF₃, OCHF₂; methylsulfonyl, dimethylphosphine oxide; and unsubstituted or substituted C₁₋₃-alkyl, in particular methyl or CF₃.

8. The compound of any one of the claims 1 to 7, wherein the compound has formula III: 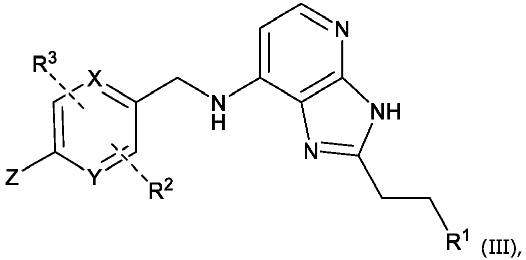 wherein X and Y are each individually selected from C or N atom;
R¹ is selected from the group consisting of cycloalkane, in particular cyclopropyl, cyclobutyl or heteroaryl,
in particular furan, particularly furan-2-yl, furan-3-yl, methylfuran, in particular 5-methylfuran-2-yl, thiophen,
in particular thiophen-2-yl, thiophen-3-yl and phenyl;
R² and R³ are each individually selected from H, F, alkyl, in particular methyl, alkoxy, in particular methoxy and ethoxy and -O-halogen alkyl, in particular OCF₃, OCHF₂;
and wherein Z is selected from unsubstituted or substituted aromatic or aliphatic heterocycle, in particular of the formula (ZIII), (ZIV), (ZV), and (ZVI)
wherein the formula (ZIII) is, 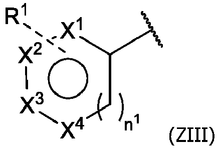
Wherein X¹, X², X³ and X⁴ are each independently selected from C or N,
n¹ is any number selected from 0, 1, 2, 3;
R¹ is selected from H, F, alkyl, in particular methyl
and wherein formula (ZIV) is, 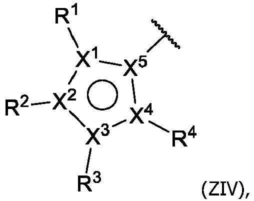
wherein X¹, X², X³ and X⁴ are each independently selected from C, N, O or S and X⁵ is selected from C or N;
and wherein if X¹ is S or O, R¹ is dispensed with, and if X¹ is C or N, R¹ is selected from H, F, alkyl, in particular methyl, methyl-*d*₃, ethyl and cyclopropyl, cyclic ether, in particular oxetane, halogen alkyl, in particular CHF₂ and CF₃
and wherein if X² is S or O, R² is dispensed with, and if X² is C or N, R² is selected from H, F, alkyl, in particular methyl, methyl-*d*₃, ethyl and cyclopropyl, cyclic ether, in particular oxetane, halogen alkyl, in particular CHF₂ and CF₃
and wherein if X³ is S or O, R³ is dispensed with, and if X³ is C or N, R³ is selected from H, F, alkyl, in particular methyl, methyl-*d*₃, ethyl and cyclopropyl, cyclic ether, in particular oxetane, halogen alkyl, in particular CHF₂ and CF₃
and wherein if X⁴ is S or O, R⁴ is dispensed with, and if X⁴ is C or N, R⁴ is selected from H, F, alkyl, in particular methyl, methyl-*d*₃, ethyl and cyclopropyl, cyclic ether, in particular oxetane, halogen alkyl, in particular CHF₂ and CF₃,
and wherein the formula (ZV) is, 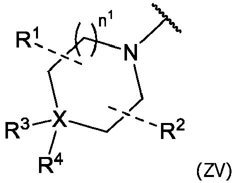
wherein X is selected from C, O, S or SOz;
n¹ is any number selected from 0, 1, 2;
R¹ and R²are each independently selected from H, F, alkyl, in particular methyl;
and wherein if X is O, S or SO₂, R³ and R⁴ are dispensed with, and if X is C, R³and R⁴ are selected from H, F;
and wherein the formula (ZVI) is, 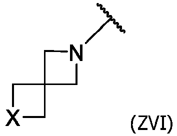
wherein X is selected from O, S or SOz.

9. The compound of any one of claims 1 to 8, wherein the compound is selected from table 1 in the description, and preferably is Example 3, 4, 7-14, 16, 17, 18, 20, 28-32, 36, 37,38, 39, 46-48, 50-54, 60, 69, 73, 82, 84, 86, or 88.

10. The compound of any one of claims 1 to 9, wherein the compound is for use in medicine, for example for the treatment of a proliferative disorder.

11. **A pharmaceutical composition,** comprising the compound according to any one of claims 1 to 10, preferably together with a pharmaceutically acceptable carrier and/or excipient.

12. **A method** of preparing a compound according to any one of claims 1 to 10, wherein the method is an organic synthesis method.

13. The method according to claim 12, wherein said compound is of the formula (A), and 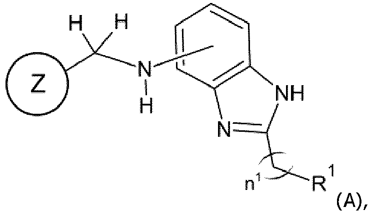
wherein Z is as defined in claim 3, and wherein X³, X⁴ and X⁵ are C;
wherein said method comprise the steps of reacting an aldehyde and an aromatic amine through reductive amination, preferably, wherein said method further comprises a step of deprotection of a tosyl group.

14. The method according to claim 12, wherein said compound is of the formula (B), 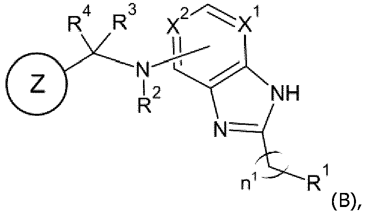
wherein Z is as defined in any one of claims 1 to 4,
said method comprising the steps of
bi) reacting a substituted aromatic nitro-substituted amid-substituted chloroderivative and a substituted aliphatic amine in a nucleophilic aromatic substitution forming an amide-substituted and nitro-substituted aromatic intermediate, and
bii) performing a cyclisation reaction of said amide-substituted and nitro-substituted aromatic intermediate from the previous step.

15. The method according to claim 12, wherein said compound is of the formula (C), 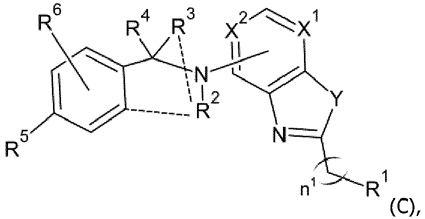
wherein R¹, R², R³, R⁴, R⁶, X¹, X² and Y are as defined in any one of claims 1 to 2, and wherein R⁵ an aromatic residue is selected from substituted and/or unsubstituted five-membered hetero and/or homocycles, in particular substituted and/or unsubstituted pyrrole, particularly, 1-methyl-1*H*-pyrrole-3-yl;substituted and/or unsubstituted Oxazole, in particular 3,5-dimethylisoxazol-4-yl; substituted and/or unsubstituted imidazole, in particular 1-methyl-1*H*-imidazol-4-yl; substituted and/or unsubstituted pyrazole, in particular 1-cyclopropyl-1*H*-pyrazol-4-yl; 1,3-dimethy-1*H*-pyrazol-4-yl; 1,5-dimethyl-1*H*-pyrazole-4-yl; 1,3,5-trimethyl-1*H*-pyrazol-4-yl; 1-difluormethyl-1*H*-pyrazol-4-yl; 1-ethyl-1*H*-pyrazol-4-yl; 1-(2-(morpholin-4-yl)-ethyl)-1*H*-pyrazol-4-yl; 1*H-*pyrazol-4-yl; 1-isopropyl-1*H*-pyrazol-4-yl; 1-(methyl-(*d*₃))-1*H*-pyrazol-4-yl; 1-methyl-1*H*-pyrazol- 3-yl; 1-methyl-1*H*-pyrazol-4-yl; 1-methyl-1*H*-pyrazol-5-yl; 1-(methylsulfonyl)-1*H*-pyrazol-4-yl; 1-(oxetan-3-yl)-1*H-*pyrazol-4-yl; 1-(tetrahydro-2*H*-pyran-4-yl)-1*H*-pyrazol-4-yl; 1-(trifluoromethyl)-1*H*-pyrazol-4-yl; 3,5-dimethyl-1*H*-pyrazol-4-yl; 3-methyl-1*H*-pyrazol-4-yl; 1-methyl-3-(trifluoromethyl)-1*H*-pyrazole-4-yl; 1-propyl-1*H-*pyrazole-4-yl; 2-(ethan-1-ol)-1*H*-pyrazol-4-yl; 1-(2-methoxyethyl)-1*H*-pyrazole-4-yl; substituted and/or unsubstituted Triazole, in particular 1-methyl-1,2,4-1*H*-triazol-3-yl; 1-methyl-1,2,3-1*H*-triazol-4-yl; substituted and/or unsubstituted Thiazole, in particular 2-Methylthiazol-5-yl;
substituted and/or unsubstituted six-membered hetero and/or homocycles, particularly substituted and/or unsubstituted pyridine, in particular, pyridin-3-yl; pyridin-4-yl; 2-fluorpyridin-4-yl; 2-methylpyridin-4-yl; 3-fluorpyridin-4-yl; substituted and/or unsubstituted pyridazin, in particular, pyridazin-4-yl; substituted and/or unsubstituted pyrimidine, in particular, pyrimidin-5-yl;
substituted and/or unsubstituted hetero and/or homo bicycles, in particular 1*H*-pyrrolo[2,3-*b*]pyridin-4-yl; 2-methyl-2*H*-indazol-5-yl; pyrazolo[1,5-*a*]pyridin-3-yl; quinolin-4-yl;
said method comprising the step of reacting a substituted aryl halide and a boronic acid pinacol ester substituted aromatic in a Suzuki reaction.
